(19) Europäisches Patentamt
European Patent Office
Office européen des brevets

(11) EP 2 062 596 A1

(12) EUROPEAN PATENT APPLICATION
published in accordance with Art. 153(4) EPC

(43) Date of publication:
**27.05.2009 Bulletin 2009/22**

(21) Application number: **07806162.9**

(22) Date of filing: **28.08.2007**

(51) Int Cl.:
***A61K 39/395*** *(2006.01)* ***A61P 35/00*** *(2006.01)*

(86) International application number:
**PCT/JP2007/066685**

(87) International publication number:
**WO 2008/026603 (06.03.2008 Gazette 2008/10)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **28.08.2006 JP 2006230300**

(83) **Declaration under Rule 32(1) EPC (expert solution)**

(71) Applicant: **Kyowa Hakko Kirin Co., Ltd.**
**Tokyo 100-8185 (JP)**

(72) Inventors:
- **SATO, Takashi**
  **Shizuoka 411-8731 (JP)**
- **ISHIDA, Hiroyuki**
  **Shizuoka 411-8731 (JP)**
- **ASADA, Masao**
  **Shizuoka 411-8731 (JP)**
- **UOCHI, Takaaki**
  **Tokyo 194-8533 (JP)**
- **OHTA, So**
  **Sunto-gun**
  **Shizuoka 411-8731 (JP)**

(74) Representative: **Vossius & Partner**
**Siebertstrasse 4**
**81675 München (DE)**

# (54) ANTI-TUMOR AGENT

(57) Therapeutic agents for removal of CD10-expressing cells from the bodies of patients are expected to be useful as therapeutic agents for various cancers such as lymphoma and leukemia in which CD 10 is expressed and for inflammatory diseases mediated by the cells in which CD10 is expressed (B cells and neutrophils). However, there is a problem that patients continuously administered with an anti-tumor agent comprising an anti-CD20 antibody as an active ingredient become resistant to the anti-tumor agent comprising the anti-CD20 antibody as an active ingredient whereby the therapeutic effect lowers. The present invention provides an agent which is effective to a patient who is administered by an agent comprising anti-CD20 antibody as an active ingredient.



Printed by Jouve, 75001 PARIS (FR)

SEQUENCE LISTING

<110> KYOWA HAKKO KOGYO CO., LTD.

<120> anti-tumor agent

<130> R1335 EP S3

<140> 07806162.9
<141> 2007-08-28

<150> JP2006/230300
<151> 2006-08-28

<160> 43

<170> PatentIn Ver. 2.1

<210> 1
<211> 750
<212> PRT
<213> Homo sapiens

<400> 1

```
Met Gly Lys Ser Glu Ser Gln Met Asp Ile Thr Asp Ile Asn Thr Pro
 1               5                  10                  15

Lys Pro Lys Lys Lys Gln Arg Trp Thr Pro Leu Glu Ile Ser Leu Ser
             20                  25                  30

Val Leu Val Leu Leu Leu Thr Ile Ile Ala Val Thr Met Ile Ala Leu
         35                  40                  45

Tyr Ala Thr Tyr Asp Asp Gly Ile Cys Lys Ser Ser Asp Cys Ile Lys
    50                  55                  60

Ser Ala Ala Arg Leu Ile Gln Asn Met Asp Ala Thr Thr Glu Pro Cys
65                  70                  75                  80

Thr Asp Phe Phe Lys Tyr Ala Cys Gly Gly Trp Leu Lys Arg Asn Val
                85                  90                  95

Ile Pro Glu Thr Ser Ser Arg Tyr Gly Asn Phe Asp Ile Leu Arg Asp
            100                 105                 110

Glu Leu Glu Val Val Leu Lys Asp Val Leu Gln Glu Pro Lys Thr Glu
        115                 120                 125

Asp Ile Val Ala Val Gln Lys Ala Lys Ala Leu Tyr Arg Ser Cys Ile
    130                 135                 140

Asn Glu Ser Ala Ile Asp Ser Arg Gly Gly Glu Pro Leu Leu Lys Leu
145                 150                 155                 160

Leu Pro Asp Ile Tyr Gly Trp Pro Val Ala Thr Glu Asn Trp Glu Gln
                165                 170                 175

Lys Tyr Gly Ala Ser Trp Thr Ala Glu Lys Ala Ile Ala Gln Leu Asn
            180                 185                 190
```

```
Ser Lys Tyr Gly Lys Lys Val Leu Ile Asn Leu Phe Val Gly Thr Asp
        195                 200                 205

Asp Lys Asn Ser Val Asn His Val Ile His Ile Asp Gln Pro Arg Leu
    210                 215                 220

Gly Leu Pro Ser Arg Asp Tyr Tyr Glu Cys Thr Gly Ile Tyr Lys Glu
225                 230                 235                 240

Ala Cys Thr Ala Tyr Val Asp Phe Met Ile Ser Val Ala Arg Leu Ile
                245                 250                 255

Arg Gln Glu Glu Arg Leu Pro Ile Asp Glu Asn Gln Leu Ala Leu Glu
            260                 265                 270

Met Asn Lys Val Met Glu Leu Glu Lys Glu Ile Ala Asn Ala Thr Ala
        275                 280                 285

Lys Pro Glu Asp Arg Asn Asp Pro Met Leu Leu Tyr Asn Lys Met Thr
    290                 295                 300

Leu Ala Gln Ile Gln Asn Asn Phe Ser Leu Glu Ile Asn Gly Lys Pro
305                 310                 315                 320

Phe Ser Trp Leu Asn Phe Thr Asn Glu Ile Met Ser Thr Val Asn Ile
                325                 330                 335

Ser Ile Thr Asn Glu Glu Asp Val Val Val Tyr Ala Pro Glu Tyr Leu
            340                 345                 350

Thr Lys Leu Lys Pro Ile Leu Thr Lys Tyr Ser Ala Arg Asp Leu Gln
        355                 360                 365

Asn Leu Met Ser Trp Arg Phe Ile Met Asp Leu Val Ser Ser Leu Ser
    370                 375                 380

Arg Thr Tyr Lys Glu Ser Arg Asn Ala Phe Arg Lys Ala Leu Tyr Gly
385                 390                 395                 400

Thr Thr Ser Glu Thr Ala Thr Trp Arg Arg Cys Ala Asn Tyr Val Asn
                405                 410                 415

Gly Asn Met Glu Asn Ala Val Gly Arg Leu Tyr Val Glu Ala Ala Phe
            420                 425                 430

Ala Gly Glu Ser Lys His Val Val Glu Asp Leu Ile Ala Gln Ile Arg
        435                 440                 445

Glu Val Phe Ile Gln Thr Leu Asp Asp Leu Thr Trp Met Asp Ala Glu
    450                 455                 460

Thr Lys Lys Arg Ala Glu Glu Lys Ala Leu Ala Ile Lys Glu Arg Ile
465                 470                 475                 480

Gly Tyr Pro Asp Asp Ile Val Ser Asn Asp Asn Lys Leu Asn Asn Glu
                485                 490                 495

Tyr Leu Glu Leu Asn Tyr Lys Glu Asp Glu Tyr Phe Glu Asn Ile Ile
            500                 505                 510
```

```
Gln Asn Leu Lys Phe Ser Gln Ser Lys Gln Leu Lys Lys Leu Arg Glu
        515                 520                 525

Lys Val Asp Lys Asp Glu Trp Ile Ser Gly Ala Ala Val Val Asn Ala
    530                 535                 540

Phe Tyr Ser Ser Gly Arg Asn Gln Ile Val Phe Pro Ala Gly Ile Leu
545                 550                 555                 560

Gln Pro Pro Phe Phe Ser Ala Gln Gln Ser Asn Ser Leu Asn Tyr Gly
                565                 570                 575

Gly Ile Gly Met Val Ile Gly His Glu Ile Thr His Gly Phe Asp Asp
            580                 585                 590

Asn Gly Arg Asn Phe Asn Lys Asp Gly Asp Leu Val Asp Trp Trp Thr
        595                 600                 605

Gln Gln Ser Ala Ser Asn Phe Lys Glu Gln Ser Gln Cys Met Val Tyr
    610                 615                 620

Gln Tyr Gly Asn Phe Ser Trp Asp Leu Ala Gly Gly Gln His Leu Asn
625                 630                 635                 640

Gly Ile Asn Thr Leu Gly Glu Asn Ile Ala Asp Asn Gly Gly Leu Gly
                645                 650                 655

Gln Ala Tyr Arg Ala Tyr Gln Asn Tyr Ile Lys Lys Asn Gly Glu Glu
            660                 665                 670

Lys Leu Leu Pro Gly Leu Asp Leu Asn His Lys Gln Leu Phe Phe Leu
        675                 680                 685

Asn Phe Ala Gln Val Trp Cys Gly Thr Tyr Arg Pro Glu Tyr Ala Val
    690                 695                 700

Asn Ser Ile Lys Thr Asp Val His Ser Pro Gly Asn Phe Arg Ile Ile
705                 710                 715                 720

Gly Thr Leu Gln Asn Ser Ala Glu Phe Ser Glu Ala Phe His Cys Arg
                725                 730                 735

Lys Asn Ser Tyr Met Asn Pro Glu Lys Lys Cys Arg Val Trp
            740                 745                 750


<210> 2
<211> 5
<212> PRT
<213> Mus musculus

<400> 2
Ser Phe Gly Met His
  1               5


<210> 3
<211> 17
<212> PRT
<213> Mus musculus
```

```
<400> 3
Tyr Ile Ser Gly Gly Ser Tyr Thr Ile Tyr Tyr Ala Asp Thr Val Lys
  1               5                  10                  15

Gly


<210> 4
<211> 10
<212> PRT
<213> Mus musculus

<400> 4
Ser Tyr Gly Asn Phe Trp Tyr Phe Asp Val
  1               5                  10


<210> 5
<211> 12
<212> PRT
<213> Mus musculus

<400> 5
Ser Val Ser Ser Ser Ile Ser Ser Ser Asn Leu His
  1               5                  10


<210> 6
<211> 7
<212> PRT
<213> Mus musculus

<400> 6
Gly Thr Ser Asn Leu Ala Ser
  1               5


<210> 7
<211> 9
<212> PRT
<213> Mus musculus

<400> 7
Gln Gln Trp Ser Ser Tyr Pro Leu Thr
  1               5


<210> 8
<211> 119
<212> PRT
<213> Artificial Sequence

<220>
<221> source
<223> /note= "Description of artificial sequence: Amino Acid Sequence of
Antibody Heavy Chain Vaiable Region"

<400> 8
 Glu Val Gln Leu Val Glu Ser Gly Gly Gly Val Val Gln Pro Gly Arg
```

```
 1               5                   10                  15

Ser Leu Arg Leu Ser Cys Ala Ala Ser Gly Phe Thr Phe Ser Ser Phe
            20                  25                  30

Gly Met His Trp Val Arg Gln Ala Pro Gly Lys Gly Leu Glu Trp Val
        35                  40                  45

Ala Tyr Ile Ser Gly Gly Ser Tyr Thr Ile Tyr Tyr Ala Asp Thr Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ser Tyr Gly Asn Phe Trp Tyr Phe Asp Val Trp Gly Gln Gly
            100                 105                 110

Thr Thr Val Thr Val Ser Ser
        115


<210> 9
<211> 357
<212> DNA
<213> Artificial Sequence

<220>
<221> source
<223> /note= "Description of artificial sequence:Synthetic DNA"

<220>
<221> CDS
<222> (1)..(357)

<400> 9
gag gtg caa ttg gtg gag tct ggg gga ggc gtg gtc cag cct ggg agg    48
Glu Val Gln Leu Val Glu Ser Gly Gly Gly Val Val Gln Pro Gly Arg
 1               5                   10                  15

tcc ctg aga ctc tcc tgt gca gcc tct gga ttc acc ttc tca agc ttt    96
Ser Leu Arg Leu Ser Cys Ala Ala Ser Gly Phe Thr Phe Ser Ser Phe
            20                  25                  30

gga atg cac tgg gtc cgc cag gct cca ggc aag ggg ctc gag tgg gtg    144
Gly Met His Trp Val Arg Gln Ala Pro Gly Lys Gly Leu Glu Trp Val
        35                  40                  45

gca tat att agt ggt gga tcc tat acc atc tac tat gca gac aca gtg    192
Ala Tyr Ile Ser Gly Gly Ser Tyr Thr Ile Tyr Tyr Ala Asp Thr Val
    50                  55                  60

aag ggc cga ttc acc atc tcc aga gac aat tcc aag aac acg ctg tat    240
Lys Gly Arg Phe Thr Ile Ser Arg Asp Asn Ser Lys Asn Thr Leu Tyr
65                  70                  75                  80

ctg cag atg aac agc ctg aga gct gag gac acg gct gtg tat tac tgt    288
Leu Gln Met Asn Ser Leu Arg Ala Glu Asp Thr Ala Val Tyr Tyr Cys
```

```
                85                  90                  95

gcg aga tct tat ggt aac ttc tgg tac ttc gat gtc tgg ggc caa ggg    336
Ala Arg Ser Tyr Gly Asn Phe Trp Tyr Phe Asp Val Trp Gly Gln Gly
            100                 105                 110

acc acg gtc acc gtc tcc tca                                        357
Thr Thr Val Thr Val Ser Ser
        115


<210> 10
<211> 109
<212> PRT
<213> Artificial Sequence

<220>
<221> source
<223> /note= "Description of artificial sequence: Amino Acid Sequence of
Antibody Light Chain Vaiable Region"

<400> 10
Asp Ile Val Met Thr Gln Ser Pro Asp Ser Leu Ala Val Ser Leu Gly
  1               5                  10                  15

Glu Arg Ala Thr Ile Asn Cys Ser Val Ser Ser Ser Ile Ser Ser Ser
            20                  25                  30

Asn Leu His Trp Tyr Gln Gln Lys Pro Gly Gln Pro Pro Lys Leu Leu
        35                  40                  45

Ile Tyr Gly Thr Ser Asn Leu Ala Ser Gly Val Pro Asp Arg Phe Ser
    50                  55                  60

Gly Ser Gly Ser Gly Thr Asp Phe Thr Leu Thr Ile Ser Ser Leu Gln
 65                  70                  75                  80

Ala Glu Asp Val Ala Val Tyr Tyr Cys Gln Gln Trp Ser Ser Tyr Pro
                85                  90                  95

Leu Thr Phe Gly Gln Gly Thr Lys Val Glu Ile Lys Arg
            100                 105


<210> 11
<211> 327
<212> DNA
<213> Artificial Sequence

<220>
<221> source
<223> /note= "Description of artificial sequence: Synthetic DNA"

<220>
<221> CDS
<222> (1)..(327)

<400> 11
gac atc gtg atg acc cag tct cca gac tcc ctg gct gtg tct ctg ggc    48
Asp Ile Val Met Thr Gln Ser Pro Asp Ser Leu Ala Val Ser Leu Gly
```

```
 1               5                   10                  15

gag agg gcc acc atc aat tgc agt gtc agc tca agt ata agt tcc agc     96
Glu Arg Ala Thr Ile Asn Cys Ser Val Ser Ser Ser Ile Ser Ser Ser
            20                  25                  30

aac ttg cac tgg tac cag cag aaa cca gga cag cct cct aag ctg ctc     144
Asn Leu His Trp Tyr Gln Gln Lys Pro Gly Gln Pro Pro Lys Leu Leu
        35                  40                  45

att tac ggc aca tcc aac ctg gct agc ggg gtc cct gac cga ttc agt     192
Ile Tyr Gly Thr Ser Asn Leu Ala Ser Gly Val Pro Asp Arg Phe Ser
    50                  55                  60

ggc agc ggg tct ggg aca gat ttc act ctc acc atc agc agc ctg cag     240
Gly Ser Gly Ser Gly Thr Asp Phe Thr Leu Thr Ile Ser Ser Leu Gln
65                  70                  75                  80

gct gaa gat gtg gca gtt tat tac tgt caa cag tgg agt agt tac cca     288
Ala Glu Asp Val Ala Val Tyr Tyr Cys Gln Gln Trp Ser Ser Tyr Pro
                85                  90                  95

ctc acg ttc ggc caa ggg acc aag gtg gaa atc aaa cgt                 327
Leu Thr Phe Gly Gln Gly Thr Lys Val Glu Ile Lys Arg
            100                 105
```

<210> 12
<211> 109
<212> PRT
<213> Artificial Sequence

<220>
<221> source
<223> /note= "Description of artificial sequence: Amino Acid Sequence of Antibody Light Chain Vaiable Region"

<400> 12

```
Asp Ile Val Met Thr Gln Ser Pro Asp Ser Leu Ala Val Ser Leu Gly
 1               5                   10                  15

Glu Arg Ala Thr Ile Ala Cys Ser Val Ser Ser Ser Ile Ser Ser Ser
            20                  25                  30

Asn Leu His Trp Tyr Gln Gln Lys Pro Gly Gln Ser Pro Lys Pro Trp
        35                  40                  45

Ile Tyr Gly Thr Ser Asn Leu Ala Ser Gly Val Pro Val Arg Phe Ser
    50                  55                  60

Gly Ser Gly Ser Gly Thr Ser Tyr Phe Leu Thr Ile Ser Ser Leu Gln
65                  70                  75                  80

Ala Glu Asp Val Ala Thr Tyr Tyr Cys Gln Gln Trp Ser Ser Tyr Pro
                85                  90                  95

Leu Thr Phe Gly Gln Gly Thr Lys Val Glu Ile Lys Arg
            100                 105
```

```
<210> 13
<211> 327
<212> DNA
<213> Artificial Sequence

<220>
<221> source
<223> /note= "Description of artificial sequence: Synthetic DNA

<220>
<221> CDS
<222> (1)..(327)

<400> 13
gac atc gtg atg acc cag tct cca gac tcc ctg gct gtg tct ctg ggc   48
Asp Ile Val Met Thr Gln Ser Pro Asp Ser Leu Ala Val Ser Leu Gly
  1               5                  10                  15

gag agg gcc acc atc gcc tgc agt gtc agc tca agt ata agt tcc agc   96
Glu Arg Ala Thr Ile Ala Cys Ser Val Ser Ser Ser Ile Ser Ser Ser
             20                  25                  30

aac ttg cac tgg tac cag cag aaa cca gga cag tcc cct aag cca tgg   144
Asn Leu His Trp Tyr Gln Gln Lys Pro Gly Gln Ser Pro Lys Pro Trp
         35                  40                  45

att tac ggc aca tcc aac ctg gct agc ggg gtc cct gtg cga ttc agt   192
Ile Tyr Gly Thr Ser Asn Leu Ala Ser Gly Val Pro Val Arg Phe Ser
     50                  55                  60

ggc agc ggg tct ggg aca tcc tac ttc ctc acc atc agc agc ctg cag   240
Gly Ser Gly Ser Gly Thr Ser Tyr Phe Leu Thr Ile Ser Ser Leu Gln
 65                  70                  75                  80

gct gaa gat gtg gca acc tat tac tgt caa cag tgg agt agt tac cca   288
Ala Glu Asp Val Ala Thr Tyr Tyr Cys Gln Gln Trp Ser Ser Tyr Pro
                 85                  90                  95

ctc acg ttc ggc caa ggg acc aag gtg gaa atc aaa cgt               327
Leu Thr Phe Gly Gln Gly Thr Lys Val Glu Ile Lys Arg
            100                 105


<210> 14
<211> 126
<212> DNA
<213> Artificial Sequence

<220>
<221> source
<223> /note= "Description of artificial sequence: Synthetic DNA"

<400> 14
tgcccagttc ctcacattca gtcagcactg aacacggacc cctcaccatg aacctcgggc 60

tcagtttgat tttccttgcc ctcattttaa aaggtgtcca gtgtgaggtg caattggtgg 120

agtctg                                                            126
```

<210> 15
<211> 127
<212> DNA
<213> Artificial Sequence

<220>
<221> source
<223> /note= "Description of artificial sequence: Synthetic DNA"

<400> 15
cttgcctgga gcctggcgga cccagtgcat tccaaagctt gagaaggtga atccagaggc 60

tgcacaggag agtctcaggg acctcccagg ctggaccacg cctcccccag actccaccaa 120

ttgcacc 127

<210> 16
<211> 150
<212> DNA
<213> Artificial Sequence

<220>
<221> source
<223> /note= "Description of artificial sequence: Synthetic DNA"

<400> 16
gccaggctcc aggcaagggg ctcgagtggg tggcatatat tagtggtgga tcctatacca 60

tctactatgc agacacagtg aagggccgat tcaccatctc cagagacaat tccaagaaca 120

cgctgtatct gcagatgaac agcctgagag 150

<210> 17
<211> 126
<212> DNA
<213> Artificial Sequence

<220>
<221> source
<223> /note= "Description of artificial sequence: Synthetic DNA"

<400> 17
ttggtggagg ctgaggagac ggtgaccgtg gtcccttggc cccagacatc gaagtaccag 60

aagttaccat aagatctcgc acagtaatac acagccgtgt cctcagctct caggctgttc 120

atctgc 126

<210> 18
<211> 33
<212> DNA
<213> Artificial Sequence

<220>
<221> source
<223> /note= "Description of artificial sequence: Synthetic DNA"

<400> 18
gggcggccgc tgcccagttc ctcacattca gtc 33

<210> 19
<211> 31
<212> DNA
<213> Artificial Sequence

<220>
<221> source
<223> /note= "Description of artificial sequence: Synthetic DNA"

<400> 19
gggggccctt ggtggaggct gaggagacgg t 31

<210> 20
<211> 124
<212> DNA
<213> Artificial Sequence

<220>
<221> source
<223> /note= "Description of artificial sequence: Synthetic DNA"

<400> 20
tcctcaggct gtctcctcag gttgcctcct caaaatgaag ttgcctgtta ggctgttggt 60

gctgatgttc tggattcctg cttccagcag tgacatcgtg atgacccagt ctccagactc 120

cctg 124

<210> 21
<211> 130
<212> DNA
<213> Artificial Sequence

<220>
<221> source
<223> /note= "Description of artificial sequence: Synthetic DNA"

<400> 21
agcagcttag gaggctgtcc tggtttctgc tggtaccagt gcaagttgct ggaacttata 60

cttgagctga cactgcaatt gatggtggcc ctctcgccca gagacacagc cagggagtct 120

ggagactggg 130

<210> 22
<211> 120
<212> DNA
<213> Artificial Sequence

<220>
<221> source
<223> /note= "Description of artificial sequence: Synthetic DNA"

<400> 22
ggacagcctc ctaagctgct catttacggc acatccaacc tggctagcgg ggtccctgac 60

cgattcagtg gcagcgggtc tgggacagat ttcactctca ccatcagcag cctgcaggct 120

<210> 23
<211> 101
<212> DNA
<213> Artificial Sequence

<220>
<221> source
<223> /note= "Description of artificial sequence: Synthetic DNA"

<400> 23
tttgatttcc accttggtcc cttggccgaa cgtgagtggg taactactcc actgttgaca 60

gtaataaact gccacatctt cagcctgcag gctgctgatg g 101

<210> 24
<211> 32
<212> DNA
<213> Artificial Sequence

<220>
<221> source
<223> /note= "Description of artificial sequence: Synthetic DNA"

<400> 24
gggaattctc ctcaggctgt ctcctcaggt tg 32

<210> 25
<211> 32
<212> DNA
<213> Artificial Sequence

<220>
<221> source
<223> /note= "Description of artificial sequence: Synthetic DNA"

<400> 25
ggcgtacgtt tgatttccac cttggtccct tg 32

<210> 26
<211> 130
<212> DNA
<213> Artificial Sequence

<220>
<221> source
<223> /note= "Description of artificial sequence: Synthetic DNA"

<400> 26
catggcttag gggactgtcc tggtttctgc tggtaccagt gcaagttgct ggaacttata 60

cttgagctga cactgcaggc gatggtggcc ctctcgccca gagacacagc cagggagtct 120

```
ggagactggg                                                          130


<210> 27
<211> 120
<212> DNA
<213> Artificial Sequence

<220>
<221> source
<223> /note= "Description of artificial sequence: Synthetic DNA"

<400> 27
ggacagtccc ctaagccatg gatttacggc acatccaacc tggctagcgg ggtccctgtg 60

cgattcagtg gcagcgggtc tgggacatcc tacttcctca ccatcagcag cctgcaggct 120


<210> 28
<211> 101
<212> DNA
<213> Artificial Sequence

<220>
<221> source
<223> /note= "Description of artificial sequence: Synthetic DNA"

<400> 28
tttgatttcc accttggtcc cttggccgaa cgtgagtggg taactactcc actgttgaca 60

gtaataggtt gccacatctt cagcctgcag gctgctgatg g                     101


<210> 29
<211> 414
<212> DNA
<213> Mus musculus

<220>
<221> CDS
<222> (1)..(414)

<400> 29
atg gac tcc agg ctc aat tta gtt ttc ctt gtc ctt att tta aaa ggt 48
Met Asp Ser Arg Leu Asn Leu Val Phe Leu Var Leu Ile Leu Lys Gly
 1               5                  10                  15

gtc cag tgt gat gtg cag ctg gtg gag tct ggg gga ggc tta gtg cag 96
Val Gln Cys Asp Val Gln Leu Val Glu Ser Gly Gly Gly Leu Var Gln
            20                  25                  30

cct gga ggg tcc cgg aaa ctc tcc tgt gca gcc tct gga ttc act ttc 144
Pro Gly Gly Ser Arg Lys Leu Ser Cys Ala Ala Ser Gly Phe Thr Phe
        35                  40                  45

agt agc ttt gga atg cac tgg gtt cgt cag gct cca gag aag ggg ctg 192
Ser Ser Phe Gly Met His Trp Val Arg Gln Ala Pro Glu Lys Gly Leu
    50                  55                  60
```

```
gag tgg gtc gca tat att agt ggt ggc agt tat acc atc tac tat gca 240
Glu Trp Val Ala Tyr Ile Ser Gly Gly Ser Tyr Thr Ile Tyr Tyr Ala
 65                  70                  75                  80

gac aca gtg aag ggc cga ttc acc atc tcc aga gac aat ccc aag aac 288
Asp Thr Val Lys Gly Arg Phe Thr Ile Ser Arg Asp Asn Pro Lys Asn
                 85                  90                  95

acc ctg ttc cta caa atg acc agt cta agg tct gag gac acg gcc atg 336
Thr Leu Phe Leu Gln Met Thr Ser Leu Arg Ser Glu Asp Thr Ala Met
            100                 105                 110

tat tac tgt gca agt tcc tat ggt aac ttc tgg tac ttc gat gtc tgg 384
Tyr Tyr Cys Ala Ser Ser Tyr Gly Asn Phe Trp Tyr Phe Asp Val Trp
        115                 120                 125

ggc gca ggg acc acg gtc acc gtc tcc tca                         414
Gly Ala Gly Thr Thr Val Thr Val Ser Ser
    130                 135


<210> 30
<211> 119
<212> PRT
<213> Mus musculus

<400> 30
Asp Val Gln Leu Val Glu Ser Gly Gly Gly Leu Var Gln Pro Gly Gly
  1               5                  10                  15

Ser Arg Lys Leu Ser Cys Ala Ala Ser Gly Phe Thr Phe Ser Ser Phe
            20                  25                  30

Gly Met His Trp Val Arg Gln Ala Pro Glu Lys Gly Leu Glu Trp Val
        35                  40                  45

Ala Tyr Ile Ser Gly Gly Ser Tyr Thr Ile Tyr Tyr Ala Asp Thr Val
     50                  55                  60

Lys Gly Arg Phe Thr Ile Ser Arg Asp Asn Pro Lys Asn Thr Leu Phe
 65                  70                  75                  80

Leu Gln Met Thr Ser Leu Arg Ser Glu Asp Thr Ala Met Tyr Tyr Cys
                 85                  90                  95

Ala Ser Ser Tyr Gly Asn Phe Trp Tyr Phe Asp Val Trp Gly Ala Gly
            100                 105                 110

Thr Thr Val Thr Val Ser Ser
        115


<210> 31
<211> 390
<212> DNA
<213> Mus musculus

<220>
<221> CDS
<222> (1)..(390)
```

<400> 31

```
atg gat ttt cat gtg cag att ttc agc ttc atg cta atc agt gtc aca 48
Met Asp Phe His Val Gln Ile Phe Ser Phe Met Leu Ile Ser Val Thr
  1               5                  10                  15

gtc ata ttg tcc agt gga gaa att gtg ctc acc cag tct cca gca ctc 96
Val Ile Leu Ser Ser Gly Glu Ile Val Leu Thr Gln Ser Pro Ala Leu
             20                  25                  30

atg gct gct tct cca ggg gag aag gtc acc atc gcc tgc agt gtc agc 144
Met Ala Ala Ser Pro Gly Glu Lys Val Thr Ile Ala Cys Ser Val Ser
         35                  40                  45

tca agt ata agt tcc agc aac ttg cac tgg tac cag cag aag tca gaa 192
Ser Ser Ile Ser Ser Ser Asn Leu His Trp Tyr Gln Gln Lys Ser Glu
     50                  55                  60

acc tcc ccc aaa ccc tgg att tat ggc aca tcc aac ctg gct tct gga 240
Thr Ser Pro Lys Pro Trp Ile Tyr Gly Thr Ser Asn Leu Ala Ser Gly
 65                  70                  75                  80

gtc cct gtt cgt ttc agt ggc agt gga tct ggg acc tct tat ttt ctc 288
Val Pro Val Arg Phe Ser Gly Ser Gly Ser Gly Thr Ser Tyr Phe Leu
                 85                  90                  95

aca atc agc agc atg gag gct gaa gat gct gcc act tat tac tgt caa 336
Thr Ile Ser Ser Met Glu Ala Glu Asp Ala Ala Thr Tyr Tyr Cys Gln
             100                 105                 110

cag tgg agt agt tac cca ctc acg ttc gga ggg ggg acc aag ctg gaa 384
Gln Trp Ser Ser Tyr Pro Leu Thr Phe Gly Gly Gly Thr Lys Leu Glu
         115                 120                 125

ata aaa                                                         390
Ile Lys
    130
```

<210> 32
<211> 108
<212> PRT
<213> Mus musculus

<400> 32

```
Glu Ile Val Leu Thr Gln Ser Pro Ala Leu Met Ala Ala Ser Pro Gly
  1               5                  10                  15

Glu Lys Val Thr Ile Ala Cys Ser Val Ser Ser Ser Ile Ser Ser Ser
             20                  25                  30

Asn Leu His Trp Tyr Gln Gln Lys Ser Glu Thr Ser Pro Lys Pro Trp
         35                  40                  45

Ile Tyr Gly Thr Ser Asn Leu Ala Ser Gly Val Pro Val Arg Phe Ser
     50                  55                  60

Gly Ser Gly Ser Gly Thr Ser Tyr Phe Leu Thr Ile Ser Ser Met Glu
 65                  70                  75                  80
```

```
Ala Glu Asp Ala Ala Thr Tyr Tyr Cys Gln Gln Trp Ser Ser Tyr Pro
                85                  90                  95

Leu Thr Phe Gly Gly Gly Thr Lys Leu Glu Ile Lys
            100                 105


<210> 33
<211> 109
<212> PRT
<213> Artificial Sequence

<220>
<221> source
<223> /note= "Description of artificial sequence: Amino Acid Sequence of
Antibody Light Chain Vaiable Region"

<400> 33
Asp Ile Val Met Thr Gln Ser Pro Ala Leu Met Ala Val Ser Leu Gly
 1               5                  10                  15

 Glu Arg Ala Thr Ile Ala Cys Ser Val Ser Ser Ser Ile Ser Ser Ser
             20                  25                  30

 Asn Leu His Trp Tyr Gln Gln Lys Pro Glu Thr Pro Pro Lys Pro Trp
         35                  40                  45

 Ile Tyr Gly Thr Ser Asn Leu Ala Ser Gly Val Pro Asp Arg Phe Ser
     50                  55                  60

 Gly Ser Gly Ser Gly Thr Ser Tyr Phe Leu Thr Ile Ser Ser Met Glu
 65                  70                  75                  80

 Ala Glu Asp Val Ala Val Tyr Tyr Cys Gln Gln Trp Ser Ser Tyr Pro
                 85                  90                  95

 Leu Thr Phe Gly Gln Gly Thr Lys Val Glu Ile Lys Arg
             100                 105


<210> 34
<211> 327
<212> DNA
<213> Artificial Sequence

<220>
<221> source
<223> /note= "Description of artificial sequence: Synthetic DNA"

<220>
<221> CDS
<222> (1)..(327)

<400> 34
gac atc gtg atg acc cag tct cca gct ctg atg gct gtg tct ctg ggc  48
Asp Ile Val Met Thr Gln Ser Pro Ala Leu Met Ala Val Ser Leu Gly
 1               5                  10                  15

gag agg gcc acc atc gcc tgc agt gtc agc tca agt ata agt tcc agc  96
Glu Arg Ala Thr Ile Ala Cys Ser Val Ser Ser Ser Ile Ser Ser Ser
```

```
            20                  25                  30

aac ttg cac tgg tac cag cag aaa cca gag acc cct cct aag cca tgg 144
Asn Leu His Trp Tyr Gln Gln Lys Pro Glu Thr Pro Pro Lys Pro Trp
        35                  40                  45

att tac ggc aca tcc aac ctg gct agc ggg gtc cct gac cga ttc agt 192
Ile Tyr Gly Thr Ser Asn Leu Ala Ser Gly Val Pro Asp Arg Phe Ser
    50                  55                  60

ggc agc ggg tct ggg aca tcc tac ttc ctc acc atc agc agc atg gag 240
Gly Ser Gly Ser Gly Thr Ser Tyr Phe Leu Thr Ile Ser Ser Met Glu
65                  70                  75                  80

gct gaa gat gtg gca gtc tat tac tgt caa cag tgg agt agt tac cca 288
Ala Glu Asp Val Ala Val Tyr Tyr Cys Gln Gln Trp Ser Ser Tyr Pro
                85                  90                  95

ctc acg ttc ggc caa ggg acc aag gtg gaa atc aaa cgt             327
Leu Thr Phe Gly Gln Gly Thr Lys Val Glu Ile Lys Arg
            100                 105


<210> 35
<211> 119
<212> PRT
<213> Artificial Sequence

<220>
<221> source
<223> /note= "Description of artificial sequence: Amino Acid Sequence of
Antibody Heavy Chain Vaiable Region"

<400> 35
Asp Val Gln Leu Val Glu Ser Gly Gly Gly Val Val Gln Pro Gly Gly
1               5                   10                  15

Ser Arg Arg Leu Ser Cys Ala Ala Ser Gly Phe Thr Phe Ser Ser Phe
            20                  25                  30

Gly Met His Trp Val Arg Gln Ala Pro Gly Lys Gly Leu Glu Trp Val
        35                  40                  45

Ala Tyr Ile Ser Gly Gly Ser Tyr Thr Ile Tyr Tyr Ala Asp Thr Val
    50                  55                  60

Lys Gly Arg Phe Thr Ile Ser Arg Asp Asn Ser Lys Asn Thr Leu Phe
65                  70                  75                  80

Leu Gln Met Thr Ser Leu Arg Ala Glu Asp Thr Ala Val Tyr Tyr Cys
                85                  90                  95

Ala Ser Ser Tyr Gly Asn Phe Trp Tyr Phe Asp Val Trp Gly Gln Gly
            100                 105                 110

Thr Thr Val Thr Val Ser Ser
        115


<210> 36
```

<211> 357
<212> DNA
<213> Artificial Sequence

<220>
<221> source
<223> /note= "Description of artificial sequence: Synthetic DNA"

<220>
<221> CDS
<222> (1)..(357)

<400> 36
```
gac gtg caa ttg gtg gag tct ggg gga ggc gtg gtc cag cct ggg ggg  48
Asp Val Gln Leu Val Glu Ser Gly Gly Gly Val Val Gln Pro Gly Gly
 1               5                  10                  15

tcc aga aga ctc tcc tgt gca gcc tct gga ttc acc ttc tca agc ttt  96
Ser Arg Arg Leu Ser Cys Ala Ala Ser Gly Phe Thr Phe Ser Ser Phe
            20                  25                  30

gga atg cac tgg gtc cgc cag gct cca ggg aag ggg ctc gag tgg gtg 144
Gly Met His Trp Val Arg Gln Ala Pro Gly Lys Gly Leu Glu Trp Val
        35                  40                  45

gca tat att agt ggt gga tcc tat acc atc tac tat gca gac aca gtg 192
Ala Tyr Ile Ser Gly Gly Ser Tyr Thr Ile Tyr Tyr Ala Asp Thr Val
    50                  55                  60

aag ggc cga ttc acc atc tcc aga gac aat tcc aag aac acg ctg ttc 240
Lys Gly Arg Phe Thr Ile Ser Arg Asp Asn Ser Lys Asn Thr Leu Phe
65                  70                  75                  80

ctg cag atg aca agc ctg aga gct gag gac acg gct gtg tat tac tgt 288
Leu Gln Met Thr Ser Leu Arg Ala Glu Asp Thr Ala Val Tyr Tyr Cys
                85                  90                  95

gcg tcc tct tat ggt aac ttc tgg tac ttc gat gtc tgg ggc caa ggg 336
Ala Ser Ser Tyr Gly Asn Phe Trp Tyr Phe Asp Val Trp Gly Gln Gly
            100                 105                 110

acc acg gtc acc gtc tcc tca                                     357
Thr Thr Val Thr Val Ser Ser
        115
```

<210> 37
<211> 119
<212> PRT
<213> Artificial Sequence

<220>
<221> source
<223> /note= "Description of artificial sequence: Amino Acid Sequence of Antibody Heavy Chain Vaiable Region"

<400> 37
```
Glu Val Gln Leu Val Glu Ser Gly Gly Gly Val Val Gln Pro Gly Arg
 1               5                  10                  15
```

```
Ser Leu Arg Leu Ser Cys Ala Ala Ser Gly Phe Thr Phe Ser Ser Phe
            20                  25                  30

Gly Met His Trp Val Arg Gln Ala Pro Glu Lys Gly Leu Glu Trp Val
        35                  40                  45

Ala Tyr Ile Ser Gly Gly Ser Tyr Thr Ile Tyr Tyr Ala Asp Thr Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ser Ser Tyr Gly Asn Phe Trp Tyr Phe Asp Val Trp Gly Gln Gly
            100                 105                 110

Thr Thr Val Thr Val Ser Ser
        115


<210> 38
<211> 357
<212> DNA
<213> Artificial Sequence

<220>
<221> source
<223> /note= "Description of artificial sequence: Synthetic DNA"


<220>
<221> CDS
<222> (1)..(357)

<400> 38
gag gtg caa ttg gtg gag tct ggg gga ggc gtg gtc cag cct ggg agg    48
Glu Val Gln Leu Val Glu Ser Gly Gly Gly Val Val Gln Pro Gly Arg
1               5                   10                  15

tcc ctg aga ctc tcc tgt gca gcc tct gga ttc acc ttc tca agc ttt    96
Ser Leu Arg Leu Ser Cys Ala Ala Ser Gly Phe Thr Phe Ser Ser Phe
            20                  25                  30

gga atg cac tgg gtc cgc cag gct cca gag aag ggg ctc gag tgg gtg    144
Gly Met His Trp Val Arg Gln Ala Pro Glu Lys Gly Leu Glu Trp Val
        35                  40                  45

gca tat att agt ggt gga tcc tat acc atc tac tat gca gac aca gtg    192
Ala Tyr Ile Ser Gly Gly Ser Tyr Thr Ile Tyr Tyr Ala Asp Thr Val
    50                  55                  60

aag ggc cga ttc acc atc tcc aga gac aat tcc aag aac acg ctg tat    240
Lys Gly Arg Phe Thr Ile Ser Arg Asp Asn Ser Lys Asn Thr Leu Tyr
65                  70                  75                  80

ctg cag atg aac agc ctg aga gct gag gac acg gct gtg tat tac tgt    288
Leu Gln Met Asn Ser Leu Arg Ala Glu Asp Thr Ala Val Tyr Tyr Cys
                85                  90                  95
```

```
gcg tcc tct tat ggt aac ttc tgg tac ttc gat gtc tgg ggc caa ggg   336
Ala Ser Ser Tyr Gly Asn Phe Trp Tyr Phe Asp Val Trp Gly Gln Gly
            100                 105                 110

acc acg gtc acc gtc tcc tca                                       357
Thr Thr Val Thr Val Ser Ser
        115
```

<210> 39
<211> 127
<212> DNA
<213> Artificial Sequence

<220>
<221> source
<223> /note= "Description of artificial sequence: Synthetic DNA"

<400> 39

```
cttctctgga gcctggcgga cccagtgcat tccaaagctt gagaaggtga atccagaggc 60

tgcacaggag agtctcaggg acctcccagg ctggaccacg cctcccccag actccaccaa 120

ttgcacc                                                           127
```

<210> 40
<211> 150
<212> DNA
<213> Artificial Sequence

<220>
<221> source
<223> /note= "Description of artificial sequence: Synthetic DNA"

<400> 40

```
gccaggctcc agagaagggg ctcgagtggg tggcatatat tagtggtgga tcctatacca 60

tctactatgc agacacagtg aagggccgat tcaccatctc cagagacaat tccaagaaca 120

cgctgtatct gcagatgaac agcctgagag                                  150
```

<210> 41
<211> 126
<212> DNA
<213> Artificial Sequence

<220>
<221> source
<223> /note= "Description of artificial sequence: Synthetic DNA"

<400> 41

```
ttggtggagg ctgaggagac ggtgaccgtg gtcccttggc cccagacatc gaagtaccag 60

aagttaccat aagaggacgc acagtaatac acagccgtgt cctcagctct caggctgttc 120

atctgc                                                            126
```

<210> 42
<211> 97
<212> DNA
<213> Artificial Sequence

<220>
<221> source
<223> /note= "Description of artificial sequence: Synthetic DNA"

<400> 42
gaattctcct caggctgtct cctcaggttg cctcctcaaa atg aag ttg cct gtt 55
Met Lys Leu Pro Val
1 5

agg ctg ttg gtg ctg atg ttc tgg att cct gct tcc agc agt 97
Arg Leu Leu Val Leu Met Phe Trp Ile Pro Ala Ser Ser Ser
10 15

<210> 43
<211> 109
<212> DNA
<213> Artificial Sequence

<220>
<221> source
<223> /note= "Description of artificial sequence: Synthetic DNA"

<400> 43
gcggccgctg cccagttcct cacattcagt cagcactgaa cacggacccc tcacc atg 58
Met
1

aac ctc ggg ctc agt ttg att ttc ctt gcc ctc att tta aaa ggt gtc 106
Asn Leu Gly Leu Ser Leu Ile Phe Leu Ala Leu Ile Leu Lys Gly Val
5 10 15

cag 109
Gln

## Description

Technical Field

The present invention relates to an agent for treating tumor resistant to an anti-CD20 antibody, comprising an antibody which specifically binds to CD10 and has cytotoxic activity or the antibody fragment thereof as an active ingredient.

Background Art

In the field of hematology, studies for differentiation antigens related to blood cells have been carried out from an early period based on analysis using anti-serum or monoclonal antibody and pigeonholing thereof has progressed as a CD (cluster of differentiation) classification. At present, many antigens expressing in various cells such as leukocytes are subjected to a CD classification (Non-Patent Document 1).
In addition, biochemical analysis, cloning of gene and functional analysis of antigen specifically or selectively expressing in tumor called a tumor-related antigen, have been also carried out based on analysis using monoclonal antibody. At present, analysis of expression of tumor-related antigen is widely used for serum diagnosis and monitoring of progress in the stage even in hematopoietic malignancies such as leukemia and lymphoma.
CD 10 is a molecule which has been found in the above-mentioned process and expression thereof is recognized on the cell surface of acute lymphoblastic leukemia (ALL). It is one of the molecules of tumor-related antigen which was called common acute lymphoblastic leukemia antigen (CALLA) (Non-Patent Document 2). As a result of the studies thereafter, it has been also found to be expressed in tumors of hematopoietic organs such as non-Hodgkin's lymphoma including chronic myelocyte leukemia (CML), Burkitt lymphoma and the like in addition to ALL. It has known that expression of CD10 found in neutrophil and part of B cell in the case of leukocytes (Non-Patent Document 3).
As a result of biochemical analysis thus far, CD10 has been known to be a type 2 transmembrane glycoprotein having molecular weight of about 100 kDa molecular weight and, as a result of further analysis using a molecular biology technique, it has been found to be identical with a molecule which was called neutral endopeptidase (NEP, EC3.4.24.11) and enkephalinase. Moreover, it has been reported that CD10 has a decomposing activity for various peptide hormones such as enkephalin, atrial natriuretic factor or atrial natriuretic peptide (ANF or ANP), substance P, and the like, regulated their activities (Non-Patent Document 3, 4) and is participated in growth, differentiation and the like of B cells (Non-Patent Document 4).
Recently, it has been also reported to be expressed not only in hematopoietic malignancies but also in solid cancers such as hepatoma, renal cancer, transitional cell cancer, prostatic cancer and endometrial cancer as well as interstitial tissue thereof (Non-Patent Document 5, 6, 7).
With regard to an antibody against CD10, there have been reported J5 antibody (Non-Patent Document 8), NL-1 antibody (Non-Patent Document 9), and the like but all of those antibodies are mouse antibodies. There has been also reported an anti-CD10 chimeric antibody of a human IgG1 type showing a cytotoxic activity to human CD10-expressing cells in which mouse myeloma cell X63Ag8.653 is used as a host-producing cell (Non-Patent Document 10; Patent Document 1 to 3). However, no CDR-grafted antibody which specifically shows reactivity to CD10 has been known so far.
It has been known that, when an antibody of non-human animals other than human is administered to human, it is usually recognized as a foreign substance resulting in adverse effects (Non-Patent Document 11 to 14) and that disappearance of the antibody from the body is accelerated (Non-Patent Document 12, 14, 15) whereby a therapeutic effect of the antibody decreases (Non-Patent Document 16, 17).
In order to solve these problems attempts have been made to concert non-human animals into a humanized antibody such as human CDR-grafted antibody (Non-Patent Document 18). In the humanized antibody, adverse effects are reduced (Non-Patent Document 19) and the therapeutic effect is extended (Non-Patent Document 20, 21) as compared with the antibody of non-human animals .
Rituximab (registered trade mark thereof is Rituxan) is an anti-tumor agent comprising active a mouse/human chimeric monoclonal antibody specifically binding to CD20 is an active ingredient (Patent Document 4). It has been reported that, when rituximab is bound to CD20 on cancer cells, CD20-expressing cells are injured via a complement-dependent cytotoxicity (CDC) (Non-Patent Document 22) or antibody-dependent cell-mediated cytotoxicity (ADCC) (Non-Patent Document 23).
Rituximab has been known as a standard therapeutic agent for a non-Hodgkin's lymphoma of a B cell type (Non-Patent Document 24). CD20 is expressed at a high frequency on tumor cells of patients suffering from a non-Hodgkin's lymphoma of a B cell type. In clinical trails of rituximab for patients suffering from recurrent or intractable low malignant or follicular non-Hodgkin's lymphoma of a B cell type, response rate was 48% (partial remission: 42%; complete remission: 6%) among 166 cases of lowly malignant or follicular non-Hodgkin's lymphoma of a B cell type which was recurrent or intractable to chemotherapy and, in the remaining patients, no anti-tumor effect of rituximab was recognized. Although efficacy of rituximab has been confirmed as above, it has been known that there are patients for whom no therapeutic effect of rituximab was recognized, showing resistance thereto (Non-Patent Document 25).

It has been pointed out that endowment with resistance to rituximab is due to repetition of the therapy by rituximab (Non-Patent Document 26). It has been said that resistance to rituximab is due to lowering or disappearance of expression level of CD20 on the tumor in the living body (Non-Patent Document 27).

It has been said that, when the level of the antigen is enhanced expression on target cells increases, an anti-tumor activity of an therapeutic antibody rises. When rituximab was allowed to react with a plurality of sub-clones established from a mouse thymoma strain EL-4 in which gene of human CD20 as antigen is introduced and having various expression level of human CD20, the clone with larger expression of CD20 showed higher antibody-dependent cell-mediated cytotoxicity and complement-dependent cytotoxicity (Non-Patent Document 28). However, influence on the tumor after the treatment with an anti-CD20 antibody has not been known.

Non-Patent Document 1: Expert Opin. Biol. Ther., 1, 375 (2001)
Non-Patent Document 2: J. Exp. Med., 168, 1247 (1988)
Non-Patent Document 3: Blood, 73, 625 (1989)
Non-Patent Document 4: Blood, 82, 1052 (1993)
Non-Patent Document 5: Anticancer Res., 17, 3233 (1997)
Non-Patent Document 6: Am. J. Pathol., 159, 1415 (2001)
Non-Patent Document 7: Am. J. Clin. Pathol., 113, 374 (2000)
Non-Patent Document 8: Blood, 58, 648 (1981)
Non-Patent Document 9: Proc. Natl. Acad. Sci. U. S. A., 79, 4386 (1982)
Non-Patent Document 10: Cancer Res., 47, 999 (1987)
Non-Patent Document 11: J. Clin. Oncol., 2, 881 (1984)
Non-Patent Document 12: Blood, 65, 1349 (1985)
Non-Patent Document 13: J. Natl. Cancer Inst., 80, 932 (1988);
Non-Patent Document 14: Proc. Natl. Acad. Sci. U.S.A., 82, 1242 (1985)
Non-Patent Document 15: J. Nucl. Med., 26, 1011 (1985)
Non-Patent Document 16: J. Immunol., 135, 1530 (1985)
Non-Patent Document 17: Cancer Res., 46, 6489 (1986)
Non-Patent Document 18: Nature, 321, 522 (1986)
Non-Patent Document 19: Proc. Natl. Acad. Sci. U.S.A., 86, 4220 (1989)
Non-Patent Document 20: Cancer Res., 56, 1118 (1996)
Non-Patent Document 21: Immunol., 85, 668 (1995)
Non-Patent Document 22: Blood, 83, 435 (1994)
Non-Patent Document 23: Cell Immunol., 204, 55 (2000)
Non-Patent Document 24: Int J Clin Oncol., 10: supplement (2005)
Non-Patent Document 25: J Clin Oncol., 16, 2825 (1998)
Non-Patent Document 26: Am Society of Hematology, Annual Meeting, 2005, Educ Program on Dec, 10. (2005)
Non-Patent Document 27: Br J Haematol., 119, 412 (2002)
Non-Patent Document 28: Clin Cancer Res., 11, 2327 (2005)
Patent Document 1: JP 1966042
Patent Document 2: EP184187
Patent Document 3: US4935496
Patent Document 4: US5736137

## DISCLOSURE OF THE INVENTION

### Problems that the Invention is to Solve

Therapeutic agents for removal of CD10-expressing cells from the bodies of patients are expected to be useful as therapeutic agents for various cancers such as lymphoma and leukemia in which CD10 is expressed and for inflammatory diseases mediated by the cells in which CD10 is expressed (B cells and neutrophils). However, there is a problem that patients continuously administered with an anti-tumor agent comprising an anti-CD20 antibody as an active ingredient become resistant to the anti-tumor agent comprising the anti-CD20 antibody as an active ingredient whereby the therapeutic effect lowers.

The present invention provides an agent for treating tumor resistant to an anti-CD20 antibody, comprising an antibody which specifically binds to CD10 and has cytotoxic activity or the antibody fragment thereof as an active ingredient.

Means for Solving the Problems

Specifically, the present invention relates to the following (1) to (9):

(1) A therapeutic agent for treating a tumor resistant to an anti-CD20 antibody, comprising administering an antibody which specifically binds to CD10 and has cytotoxic activity or the antibody fragment thereof;
(2) The therapeutic agent according to the above-described (1), wherein the antibody is a gene recombinant antibody;
(3) The therapeutic agent according to the above-described (1), wherein the cytotoxic activity is at least one activity of antibody-dependent cell-mediated cytotoxicity and complement-dependent cytotoxicity;
(4) The therapeutic agent according to the above-described (2), wherein the gene recombinant antibody is selected from the group consisting of a human chimeric antibody, a humanized antibody and a human antibody;
(5) The therapeutic agent according to the above-described (1), wherein the antibody fragment is an antibody selected from Fab, Fab', $F(ab')_2$, a single chain antibody (scFv), a dimerized variable region (diabody), a disulfide stabilized variable region (dsFv) and a CDR peptide;
(6) The therapeutic agent according to any one of the above-described (1) to (5), wherein the antibody is a fusion antibody conjugated with a radioisotope, a protein or an agent;
(7) The therapeutic agent according to any one of the above-described (1) to (6), wherein the tumor resistant to an anti-CD20 antibody is a tumor in which the expression level of CD 10 on the tumor cells is increased by administration of an anti-tumor agent comprising an anti-CD20 antibody as an active ingredient;
(8) The therapeutic agent according to any one of the above-described (1) to (7), wherein the tumor resistant to an anti-CD20 antibody is a tumor in which the expression level of CD20 on the tumor cell is decreased or depleted by administration of the anti-CD20 antibody; and
(9) Use of an antibody which specifically binds to CD 10 and has cytotoxic activity or the antibody fragment thereof for the manufacture of a therapeutic agent for treating a tumor resistant to an anti-CD20 antibody.

Effect of the Invention

The present invention relates to an agent for treating tumor resistant to an anti-CD20 antibody, comprising an antibody which specifically binds to CD10 and has cytotoxic activity or the antibody fragment thereof as an active ingredient.

Brief Description of the Drawings

[Fig. 1] Fig. 1 shows the result in which the expression level of antigen of RTX/Raji cells and Raji which is a parent cell line thereof on cell surface was analyzed by a flow cytometer. The upper histgrams show the result for Raji cells while the lower histgrams show the result for RTX/Raji cells. The ordinate shows cell numbers while the abscissa shows the fluorescence intensity. The histograms surrounded by closed area (■) show the result for a negative control antibody staining. The histograms surrounded by opened area (□) show the results for staining by anti-CD10 antibody NL-1, anti-CD19 antibody J4.119, anti-CD20 antibody B1 and anti-CD22 antibody SJ.10.1H11 in the order of from left to right.
[Fig. 2] Fig. 2 shows the ADCC activity of the anti-CD 10 human chimeric antibody, Ms705/CD10 (upper graph) and the anti-CD20 antibody, rituximab (lower graph) in the RTX/Raji cells and Raji cells. Black circles show the result for RTX/Raji cells while white circles show the result of Raji cells. The ordinate shows the cytotoxic activity and the abscissa shows the antibody concentration.
[Fig. 3] Fig. 3 shows the CDC activity of the anti-CD10 human chimeric antibody, Ms705/CD10 (upper graph) and the anti-CD20 antibody, rituximab (lower graph) in the RTX/Raji cells and Raji cells. Solid line with black circles shows the result for RTX/Raji cells while solid line with white circles shows the result of Raji cells. The ordinate shows the cell survival rate and the abscissa shows the antibody concentration.
[Fig. 4] Fig. 4 shows the anti-tumor activity when the anti-CD10 human chimeric antibody, Ms705/CD10 (upper graph) and the anti-CD20 antibody, rituximab (lower graph) were administered to mice to which RTX/Raji cells and Raji cells were transplanted. The ordinate shows T/C and the abscissa shows the elapsed day(s) from the date when administration of the antibody was started. Solid line with black circles shows the result in the mice to which RTX/Raji cells were transplanted and solid line with white circles shows the result in the mice to which Raji cells were transplanted.

[Fig. 5] Fig. 5 shows the anti-tumor activity of the anti-CD10 humanized antibody, HV2LV9 MS705 in non-Hodgkin's lymphoma cell line Raji cell transplanted mice to which the anti-CD20 antibody, rituximab was administered and non-Hodgkin's lymphoma cell line Raji cells were transplanted. The ordinate shows the tumor volume (V/V0) and the abscissa shows the elapsed day(s) from the initial day of administration of rituximab. The day when administration of rituximab was started was named day 0 and rituximab was administered four times until day 14 (shown by an arrow). After day 14, there are a group to which the CD10 humanized antibody HV2LV9 MS705 was administered four times (solid line with white circles) and a group to which rituximab was administered four times (solid line with black circles).

[Fig. 6] Fig. 6 shows the anti-tumor activity of the anti-CD10 humanized antibody, HV2LV9 MS705 and the anti-CD20 antibody, rituximab in non-Hodgkin's lymphoma cell line Namalwa cell-transplanted mice. The ordinate shows the tumor volume (V/V0) and the abscissa shows the elapsed day(s) from the initial day of administration of the antibody. There are shown a group to which a physiological saline solution was administered (dashed line with white circles), a group to which the CD10 humanized antibody HV2LV9 MS705 was administered (solid line with white circles) and a group to which rituximab was administered (solid line with black circles) are shown.

[Fig. 7] Fig. 7 is a drawing which shows construction of a plasmid pCRHV0.

[Fig. 8] Fig. 8 is a drawing which shows the construction of a plasmid pCRHV2.

[Fig. 9] Fig. 9 is a drawing which shows the construction of a plasmid pCRLV0.

[Fig. 10] Fig. 10 is a drawing which shows the construction of a plasmid pCRLV9.

[Fig. 11] Fig. 11 is a drawing which shows the construction of anti-CD10 human chimeric antibody expression vector pKANTEX 3061 chimeric HLV0.

[Fig. 12] Fig. 12 is a drawing which shows the construction of anti-CD10 humanized antibody expression vector pKANTEX 3061 HV0LV0.

[Fig. 13] Fig. 13 is a drawing which shows the construction of anti-CD10 humanized antibody expression vector pKANTEX 3061 HV0LV9.

[Fig. 14] Fig. 14 is a drawing which shows the construction of anti-CD10 humanized antibody expression vector pKANTEX 3061 HV2LV0.

[Fig. 15] Fig. 15 is a drawing which shows the ADCC activity of purified anti-CD10 humanized antibody HV0LV9 MS705 antibody and anti-CD10 humanized antibody HV2LV9 MS705 to Daudi cells or Raji cells which are human non-Hodgkin's lymphoma cell line. The abscissa shows the antibody concentration and the ordinate shows the ADCC activity (%) in each antibody concentration. "A" shows the result when Daudi cell was used as a target cell while "B" shows the results when Raji cell was used as a target cell. In the graph, closed square (■) shows MS705/CD10, opened circle (○) shows anti-CD10 humanized antibody, HV0LV9 MS705 and opened triangle (Δ) shows anti-CD10 humanized antibody, HV2LV9 MS705.

[Fig. 16] Fig. 16 is a drawing which shows the CDC activity of purified anti-CD10 humanized antibody, HV0LV9 MS705 and anti-CD10 humanized antibody, HV2LV9 MS705 to Daudi cell which is a human non-Hodgkin's lymphoma cell line. The abscissa shows the antibody concentration and the ordinate shows the CDC activity (%) in each antibody concentration.

[Fig. 17] Fig. 17 is a graph in which the abscissa shows the day(s) after the transplantation and the ordinate shows the body weight. In the graph, × shows the control group, closed square (■) shows the group to which 1 mg/kg of MS705/CD10 was administered, opened circle (○) shows the group to which 0.1 mg/kg of anti-CD10 humanized antibody, HV0LV9 MS705 was administered and opened triangle (Δ) shows the group to which 1 mg/kg of anti-CD10 humanized antibody HV2LV9 MS705 was administered. Bar shows a standard deviation.

[Fig. 18] Fig. 18 is a graph in which the abscissa shows the day(s) after the transplantation and the ordinate shows the survival rate. In the graph, × shows the control group, closed square (■) shows the group to which 1 mg/kg of Ms705/CD10 was administered, opened circle (○) shows the group to which 0.1 mg/kg of anti-CD10 humanized antibody, HV0LV9 MS705 was administered and opened triangle (Δ) shows the group to which 1 mg/kg of anti-CD10 humanized antibody, HV2LV9 MS705 was administered. Bar shows a standard deviation.

Best Mode for Carrying Out the Invention

The agent for treating a tumor resistant to an anti-CD20 antibody, comprising an antibody which specifically binds to CD10 and has cytotoxic activity or the antibody fragment thereof as an active ingredient used in the present invention may be any antibody, so long as it can specifically bind to CD10 and injure or remove a tumor cell. It is preferably an antibody which can bind to an extracellular region of CD10 and injures a tumor cell expressing CD10 by cytotoxic activity, and more preferably an antibody an antibody which specifically binds to an epitope present at positions 52 to 750 in the amino acid sequence represented by SEQ ID NO:1, or an antibody which specifically binds to an epitope recognized by a monoclonal antibody produced by FERM BP-10099. The cytotoxic activity in the present invention includes at least one activity of antibody-dependent cell-mediated cytotoxicity (hereinafter referred to as ADCC activity) and complement-dependent cytotoxicity (hereinafter referred to as CDC activity).

The antibody used in the present invention includes a gene recombinant antibody. The gene recombinant antibody includes a human chimeric antibody, a humanized antibody, a human antibody and antibody fragments thereof.

The human chimeric antibody is an antibody comprising a heavy chain variable region (hereinafter the heavy chain may be referred to as H chain, the variable region may be referred to as V region, and H chain V region may be referred to as VH) of an antibody derived from a non-human animal and a light chain V region (hereinafter the light chain being referred to as L chain, and the L chain V region may be referred to as VL) of an antibody derived from a non-human animal, and an H chain constant region (hereinafter the constant region being referred to as C region, and the H chain C region may be referred to as CH) of a human antibody and an L chain C region (hereinafter referred to as CL) of a human antibody. Any non-human animal can be used, so long as a hybridoma can be produced therefrom. Examples include mouse, rat, hamster, guinea pig, rabbit, dog, goat, sheep, pig, cattle and monkey.

The human chimeric antibody used in the present invention can be produced by a know method, such as a method described in EP 127095. The human chimeric antibody can be produced by obtaining cDNAs encoding VH and VL from a hybridoma capable of producing an anti-CD10 antibody, inserting each of the cDNAs into an expression vector for animal cell having DNAs encoding CH and CL of a human antibody to thereby construct a human chimeric antibody expression vector, and introducing it into an animal cell for expression.

As the CH of human chimeric antibody, any CH can be used, so long as it belongs to human immunoglobulin (hIg), and those belonging to the hIgG class are preferred, and any one of the subclasses belonging to the hIgG class, such as $\gamma1$, $\gamma2$, $\gamma3$ and $\gamma4$, can be used. As the CL of human chimeric antibody, any CL can be used, so long as it belongs to the hIg class, and those belonging to the $\kappa$ class or $\lambda$ class can also be used.

Transformant Ms705/CD10 producing an anti-CD10 human chimeric antibody, Ms705/CD10 has been deposited to International Patent Organism Depositary, National Institute of Advanced Industrial Science and Technology (Tsukuba Central 6, 1-1, Higashi 1-chome, Tsukuba-shi, Ibaraki-ken, Japan) as FERM BP-08478 on September 11, 2003.

A humanized antibody is an antibody in which CDRs of VH and VL of a human antibody are each replaced by CDR sequences of a non-human animal antibody, and is also called a human CDR-grafted antibody.

The humanized antibody used in the present invention can be produced by constructing cDNAs encoding V regions in which CDR sequences of VH and VL in any human antibody are each replaced by CDR sequences of VH and VL in an anti-CD10 antibody of a non-human animal, inserting them respectively into an expression vector for animal cell comprising genes encoding CH of human antibody CL of human antibody to thereby construct a humanized antibody expression vector, and then introducing the expression vector into an animal cell to express the humanized antibody.

As the CH of the humanized antibody, any CH can be used, so long as it belongs to the hIg, and those of the hIgG class are preferred and any one of the subclasses belonging to the hIgG class, such as $\gamma1$, $\gamma2$, $\gamma3$ and $\gamma4$, can be used. As the CL of the humanized antibody, any CL can be used, so long as it belongs to the hIg class, and those belonging to the $\kappa$ class or $\lambda$ class can be used.

The method for selecting amino acid sequences of FRs of VH and VL of a human antibody may be any amino acid sequences, so long as they are derived from a human antibody. For example, amino acid sequences of FRs of VH and VL of human antibodies registered in database such as Protein Data Bank, common amino acid sequences of each subgroups of FRs of VH and VL of human antibodies described in *Sequences of Proteins of Immunological Interest*, US Dept. Health and Human Services (1991), and the like are included.

Specific examples of the humanized antibody which specifically reacts with CD 10 used in the present invention include:

a humanized antibody which specifically reacts with CD 10, in which the heavy chain (H chain) variable region (V region) of the antibody comprises the amino acid sequence represented by SEQ ID NO:8 or an amino acid sequence in which at least one amino acid residue selected from Glu at position 1, Arg at position 16, Leu at position 18, Gly at position 42, Tyr at position 80, Asn at position 84 and Val at position 86 in the amino acid sequence represented by SEQ ID NO:8 is substituted with other amino acid residue;

a humanized antibody which specifically reacts with CD10, in which the light chain (L chain) variable region (V region) of the antibody comprises the amino acid sequence represented by SEQ ID NO:10 or an amino acid sequence in which at least one amino acid residue selected from Asp at position 9, Ser at position 10, Leu at position 11, Asn at position 22, Gly at position 42, Gln at position 34, Pro at position 44, Leu at position 47, Leu at position 48, Asp at position 61, Asp at position 71, Phe at position 72, Thr at position 73, Leu at position 79, Gln at position 80 and Val at position 86 in the amino acid sequence represented by SEQ ID NO:10 is substituted with other amino acid residue; or

a humanized antibody which specifically reacts with CD10, in which the heavy chain (H chain) variable region (V region) of the antibody comprises the amino acid sequence represented by SEQ ID NO:8 or an amino acid sequence in which at least one amino acid residue selected from Glu at position 1, Arg at position 16, Leu at position 18, Gly at position 42, Tyr at position 80, Asn at position 84 and Arg at position 98 in the amino acid sequence represented by SEQ ID NO:8 is substituted with other amino acid residue, and the light chain (L chain) variable region (V region) of the antibody comprises the amino acid sequence represented by SEQ ID NO:10 or an amino acid sequence in which at least one amino acid residue selected from Asp at position 9, Ser at position 10, Leu at position 11, Asn at position 22, Gly at position 42, Gln at position 43, Pro at position 44, Leu at position 47, Leu at position 48, Asp at position 61, Asp at position 71, Phe at position 72, Thr at position 73, Leu at position 79, Gln at position 80 and Val at position 86 in the amino acid sequence represented by SEQ ID NO:10 is substituted with other amino acid residue; and
preferred examples include:

a humanized antibody which specifically reacts with CD 10, in which the heavy chain (H chain) variable region (V region) of the antibody comprises any of the amino acid sequences represented by SEQ ID NO:8, 35 or 37;
a humanized antibody which specifically reacts with CD10, in which the light chain (L chain) variable region (V region) of the antibody comprises any of the amino acid sequences represented by SEQ ID NO:10, 12 or 33, or the antibody fragment thereof;
a humanized antibody which specifically reacts with CD10, in which the heavy chain (H chain) variable region (V region) of the antibody comprises any of the amino acid sequences represented by SEQ ID NO:8, 35 or 37, and the light chain (L chain) variable region (V region) of the antibody comprises any of the amino acid sequences represented by SEQ ID NO:10, 12 or 33;
and the like.

Transformant HV0LV9MS705 capable of producing humanized antibody HV0LV9MS705 has been deposited to International Patent Organism Depositary, National Institute of Advanced Industrial Science and Technology [Tsukuba Central 6, 1-1, Higashi 1-chome, Tsukuba-shi, Ibaraki, Japan] as FERM BP-10099 on August 17, 2004.
The antibody used in the present invention includes an antibody which specifically reacts with CD10, in which one or more amino acids is/are deleted, added, substituted or inserted in these amino acid sequences.
In the present invention, one or more amino acid residue deletion, substitution, insertion or addition in the amino acid sequence means that one or plurality of amino acid residues are deleted, substituted, inserted and/or added to at one or plural arbitrary positions in the amino acid sequence. The deletion, substitution, insertion or addition may be caused in the same amino acid sequence simultaneously. Also, the amino acid residue, deleted, substituted, inserted or added can be natural or non-natural. Examples of the natural amino acid residue include L-alanine, L-asparagine, L-aspartic acid, L-glutamine, L-glutamic acid, glycine, L-histidine, L-isoleucine, L-leucine, L-lysine, L-methionine, L-phenylalanine, L-proline, L-serine, L-threonine, L-tryptophan, L-tyrosine, L-valine, L-cysteine, and the like.
Thereinafter, examples of amino acid residues which are capable of substituting one another are shown. The amino acid residues in the same group can be substituted with each other.

Group A: leucine, isoleucine, norleucine, valine, norvaline, alanine, 2-aminobutanoic acid, methionine, O-methylserine, t-butylglycine, t-butylalanine, cyclohexylalanine;
Group B: aspartic acid, glutamic acid, isoaspartic acid, isoglutamic acid, 2-aminoadipic acid, 2-aminosuberic acid;
Group C: asparagine, glutamine;
Group D: lysine, arginine, ornithine, 2,4-diaminobutanoic acid, 2,3-diaminopropionic acid; Group E: proline, 3-hydroxyproline, 4-hydroxyproline;
Group F: serine, threonine, homoserine;
Group G: phenylalanine, tyrosine.

The antibody fragment of the present invention includes Fab, Fab', $F(ab')_2$, scFv, diabody, dsFv, a peptide comprising CDR, and the like.
An Fab is an antibody fragment having a molecular weight of about 50,000 and having antigen binding activity, in which about a half of the N-terminal side of H chain and the entire L chain, among fragments obtained by treating IgG with a protease, papain (by cleaving an amino acid residue at position 224 of the H chain), are bound together through a disulfide bond (S-S bond).
The Fab used in the present invention can be obtained by treating the antibody which specifically binds to CD10 and has cytotoxic activity, with a protease, papain. Also, the Fab can be produced by inserting DNA encoding Fab of the antibody into an expression vector for prokaryote or an expression vector for eukaryote, and introducing the vector into a prokaryote or eukaryote to express the Fab.
An $F(ab')_2$ is an antibody fragment having antigen binding activity and having a molecular weight of about 100,000 which is slightly larger than the Fab bound via an S-S bond in the hinge region, among fragments obtained by treating IgG with a protease, pepsin (by cleaving an amino acid residue at position 234 of the H chain).
The $F(ab')_2$ used in the present invention can be obtained by treating the antibody which specifically binds to CD10 and has cytotoxic activity, with a protease, pepsin. Also, it can be prepared by binding Fab' described below via a thioether bond or an S-S bond.

An Fab' is an antibody fragment having a molecular weight of about 50,000 and having antigen binding activity, which is obtained by cleaving an S-S bond at the hinge region of the above $F(ab')_2$.

The Fab' used in the present invention can be obtained by treating the $F(ab')_2$ of the antibody which specifically binds to CD 10 and has cytotoxic activity, with a reducing agent, dithiothreitol. Also, the Fab' can be produced by inserting DNA encoding the Fab' of the antibody which specifically binds to CD 10 and has cytotoxic activity into an expression vector for prokaryote or an expression vector for eukaryote, and introducing the vector into a prokaryote or eukaryote to express the Fab'.

An scFv is a VH-P-VL or VL-P-VH polypeptide in which one chain VH and one chain VL are linked using an appropriate peptide linker (P) of 12 or more amino acid residues and is an antibody fragment having antigen binding activity.

The scFv used in the present invention can be produced by obtaining cDNAs encoding VH and VL of the antibody which specifically binds to CD 10 and has cytotoxic activity, constructing DNA encoding scFv, inserting the DNA into an expression vector for prokaryote or an expression vector for eukaryote, and then introducing the expression vector into a prokaryote or eukaryote to express the scFv.

A diabody is an antibody fragment in which scFv having the same or different antigen binding activity forms a dimer, and has divalent antigen binding activity to the same antigen or two specific antigen binding activities to different antigens.

The diabody used in the present invention can be produced by obtaining cDNAs encoding VH and VL of the antibody which specifically binds to CD10 and has cytotoxic activity, constructing DNA encoding scFv having a polypeptide linker of 3 to 10 residues, inserting the DNA into an expression vector for prokaryote or an expression vector for eukaryote, and then introducing the expression vector into a prokaryote or eukaryote to express the diabody.

A dsFv is obtained by binding polypeptides in which one amino acid residue of each of VH and VL is substituted with a cysteine residue via an S-S bond between the cysteine residues. The amino acid residue to be substituted with a cysteine residue can be selected based on a three-dimensional structure estimation of the antibody in accordance with the method shown by Reiter et al. [Protein Engineering, 7, 697 (1994)].

The dsFv used in the present invention can be produced by obtaining cDNAs encoding VH and VL of the antibody which specifically binds to CD10 and has cytotoxic activity in the present invention, constructing DNA encoding dsFv, inserting the DNA into an expression vector for prokaryote or an expression vector for eukaryote, and then introducing the expression vector into a prokaryote or eukaryote to express the dsFv.

A peptide comprising CDR is constituted by including one region or more of CDRs of VH or VL.

Plural peptide comprising CDRs can be bound directly or via an appropriate peptide linker.

The peptide comprising CDR used in the present invention can be produced by constructing cDNAs encoding CDRs of VH and VL of the antibody which specifically binds to CD 10 and has cytotoxic activity, inserting the cDNAs into an expression vector for prokaryote or an expression vector for eukaryote, and then introducing the expression vector into a prokaryote or eukaryote to express the peptide. The peptide comprising CDR can also be produced by a chemical synthesis method such as an Fmoc method (fluorenylmethoxycarbonyl method), a tBoc method (t-butyloxycarbonyl method), or the like.

The antibody of the present invention includes antibody derivatives in which a radioisotope, a protein or an agent is bound to the antibody or the antibody fragment thereof described above.

The antibody derivatives of the present invention can be produced by chemically conjugating an agent to the N-terminal side or C-terminal side of an H chain or an L chain of the antibody which specifically binds to CD 10 and has cytotoxic activity or the antibody fragment thereof, to an appropriate substituent group or side chain of the antibody or to a sugar chain in the antibody [Antibody Engineering Handbook, edited by Osamu Kanemitsu, published by Chijin Shokan (1994)].

Also, it can be produced by genetic engineering techniques in which a DNA encoding an antibody which specifically binds to CD10 and has cytotoxic activity or the antibody fragment thereof is ligated with a DNA encoding an agent to be conjugated, such as a protein, the ligated DNA is inserted into an expression vector, and the expression vector is introduced into a host cell.

The agent includes a chemotherapeutic agent, a therapeutic antibody, an immunostimulator such as cytokine, a radioisotpoe, an immunoadjuvant and the like.

Furthermore, the agent to be bound to an antibody or to the antibody fragment thereof may be in a form of a prodrug. The prodrug in the present invention is a agent which is subjected to a chemical modification by an enzyme of the patient himself/herself existing in the tumor environment and is converted to a substance having an activity of damaging the tumor cells.

The chemotherapeutic agent includes any chemotherapeutic agents such as an alkylating agent, a nitrosourea agent, a metabolism antagonist, an anticancer antibiotic substance, an alkaloid derived from a plant, a topoisomerase inhibitor, an agent for hormonotherapy, a hormone antagonist, an aromatase inhibitor, a P glycoprotein inhibitor, a platinum complex derivative, an M-phase inhibitor and a kinase inhibitor. Examples of the chemotherapeutic agent include amifostine (Ethyol), cisplatin, dacarbazine (DTIC), dactinomycin, mecloretamin (nitrogen mustard), streptozocin, cyclophosphamide, iphosphamide, carmustine (BCNU), lomustine (CCNU), doxorubicin (adriamycin), doxorubicin lipo (Doxyl), epirubicin, gemcitabine (Gemsal), daunorubicin, daunorubicin lipo (Daunozome), procarbazine, mitomycin, cytarabine, etoposide, methotrexate, 5-fluorouracil, fluorouracil, vinblastine, vincristine, bleomycin, daunomycin, peplomycin, estramustine, paclitaxel (Taxol), docetaxel (Taxotea), aldesleukin, asparaginase, busulfan, carboplatin, oxaliplatin, nedaplatin, cladribine, camptothecin, CPT-11, 1 0-hydroxy-7-ethylcamptothecin (SN38), floxuridine, fludarabine, hydroxyurea, iphosphamide, idarubicin, mesna, irinotecan, nogitecan, mitoxantrone, topotecan, leuprolide, megestrol, melfalan, mercaptopurine, hydroxycarbamide, plicamycin, mitotane, pegasparagase, pentostatin, pipobroman, streptozocin, tamoxifen, goserelin, leuprorelin, flutamide, teniposide, testolactone, thioguanine, thiotepa, uracil mustard, vinorelbine, chlorambucil, hydrocortisone, prednisolone, methylprednisolone, vindesine, nimustine, semustine, capecitabine, Tomudex, azacytidine, UFT, oxaliplatin, gefitinib (Iressa), imatinib (STI 571), elrotinib, Flt3 inhibitor, VEGFR inhibitor, FGFR inhibitor, radicicol, 17-allylamino-17-demethoxygeldanamycin, rapamycin, amsacrine, all-trans-retinoic acid, thalidomide, anastrozole, fadrozole, letrozole, exemestane, gold thiomalate, D-penicillamine, bucillamine, azathioprine, mizoribine, cyclosporine, rapamycin, hydrocortisone, bexarotene (Targretin), tamoxifen, dexamethasone, progestin substances, estrogen substances, anastrozole (Arimidex), Leuplin, aspirin, indomethacin, celecoxib, azathioprine, penicillamine, gold thiomalate, chlorpheniramine maleate, chlorpheniramine, clemastine, tretinoin, bexarotene, arsenic, voltezomib, allopurinol, gemtuzumab, ibritumomab tiuxetan, 131 tositumomab, Targretin, ONTAK, ozogamine, clarithromycin, leucovorin, ifosfamide, ketoconazole, aminoglutethimide, suramin and methotrexate.

The method for conjugating the chemotherapeutic agent with the antibody include a method in which the chemotherapeutic agent and an amino group of the antibody are conjugated via glutaraldehyde, a method in which an amino group of the chemotherapeutic agent and a carboxyl group of the antibody are bound via a water-soluble carbodiimide, and the like.

Examples of the therapeutic antibody include an antibody against an antigen expressed in tumor cells which directly injure cancer cells and an antibody against an antigen participating in formation of morbid state of tumor such as growth or metastasis of tumor cells and, moreover, an antibody which regulates immune in the living body to which the antibody is administered and an antibody which inhibits the angiogenesis in the living body to which the antibody is administered.

The antigen expressed in tumor cells which directly injure cancer cells include CD19, CD20, CD21, CD22, CD23, CD24, CD37, CD53, CD72, CD73, CD74, CDw75, CDw76, CD77, CDw78, CD79a, CD79b, CD80 (B7.1), CD81, CD82, CD83, CDw84, CD85, CD86 (B7.2), HLA-Class II and the like.

The antigen for the antibody which regulates immune in the living body to which the antibody is administered is preferably CD4, CD40, CD40 ligand, B7 family molecule (CD80, CD86, CD274, B7-DC, B7-H2, B7-H3, B7-H4), ligand of B7 family molecule (CD28, CTLA-4, ICOS, PD-1, BTLA), OX-40, OX-40 ligand, CD137, TNF receptor family molecule (DR4, DR5, TNFR1, TNFR2), TRAIL family molecule, receptor family of TRAIL family molecule (TRAIL-R1, TRAIL-R2, TRAIL-R3, TRAIL-R4), RANK, RANK ligand, CD25, folic acid receptor 4, cytokine (IL-1α, IL-1β, IL-4, IL-5, IL-6, IL-10, IL-13, TGFβ, TNFα, *etc.*), receptors of these cytokines, chemokine (SLC, ELC, I-309, TARC, MDC, CTACK, *etc.*) and receptors of these chemokines.

The antigen for the antibody which inhibits the angiogenesis in the living body to which the antibody is administered includes VEGF, Angiopoietin, FGF, EGF, PDGF, IGF, EPO, TGFβ, IGF, IL-8, Ephilin, SDF-1 and the like.

The immunostimulator may be any cytokine, so long as it enhances cells such as NK cells, macrophages, neutrophils and the like. Examples include interferon α, interferon β, interferon γ, interleukin-2, interleukin-12, interleukin-15, interleukin-18, interleukin-23, granulocyte-colony stimulating factor (G-CSF), granulocyte macrophage-colony stimulating factor (GM-CSF), macrophage-colony stimulating factor (M-CSF) and the like. Also, natural products known as immunostimulators are included, and examples of an agent enhancing immunogen include β(1→3)glucan (lentinan, schizophyllan), α-galactosylceramide (KRN7000), fungus powder(picibanil, BCG) and fungus extract (krestin). The radioisotope includes $^{131}$I, $^{125}$I, $^{90}$Y, $^{64}$Cu, $^{199}$Tc, $^{77}$Lu, $^{211}$At and the like. The radioisotope can directly be conjugated with the antibody by a Chloramine-T method. Also, a substance chelating the radioisotope can be conjugated with the antibody. The chelating agent includes methylbenzyldiethylene-triaminepentaacetic acid (MX-DTPA) and the like.

In the present invention, the antibody used in the present invention can be administered in combination with one or more of other agents. Also, irradiation can be used in combination. The other agent includes the above-described chemotherapeutic agent, therapeutic antibody, immunostimulator such as cytokine, and the like.

The radiation irradiation include photon (electromagnetic) irradiation such as X-ray or γ-ray, particle irradiation such as electron beam, proton beam or heavy particle bema, and the like.

In the method for combined administration, the agent may be simultaneously administered with the antibody used in the present invention, or the agent may be administered before or after the administration of the antibody used in the present invention.

The therapeutic agent of the present invention has therapeutic effect on tumor resistant to an anti-CD20 antibody. The tumor resistant to an anti-CD20 antibody is a tumor in which the therapeutic effect of the anti-CD20 antibody is decreased or deleted by administration of an anti-CD20 antibody in comparison with the tumor which has no resistance to the anti-CD20 antibody. Preferred examples include a tumor in which the expression amount of CD 10 on the tumor cells is increased by administration of an anti-tumor agent comprising an anti-CD20 antibody as an active ingredient, and a tumor in which the expression amount of CD20 on the tumor cells is decreased or deleted by administration of an anti-tumor agent comprising an anti-CD20 antibody as an active ingredient.

The anti-CD20 antibody in the present invention may be any antibody, so long as it has a property capable of specifically binding to CD20. Examples include a human and mouse chimeric anti-human CD20 antibody, rituximab; a complete humanized antibody HuMAX-CD20 [Blood, 106, 448 (2005)]; a humanized anti-CD20 antibody hA20 [Proc Am Soc Clin Oncol, 22, 2393 (2003)]; a mouse antibody bound with methylbenzyldiethylene-triaminepentaacetic acid (MX-DTPA) via a covalent bond, ibritumomab tiuxetan [Blood, 106, 1131 (2005)]; a mouse antibody labeled with radioactive iodine, $^{131}$I-tositumomab [J Clin Oncol, 23, 712 (2005)], and the like.

In the present invention, the tumor in the tumor resistant to an anti-CD20 antibody is not particularly limited, but a tumor derived from B cell is preferable. Examples include B-cell neoplasms classified by WHO classification [*Tumours of haematopoietic and lymphoid tissues*, IARC-Press, Lyon (2001)], and specific examples include precursor B-cell lymphoblastic leukemia/lymphoma, B-cell chronic lymphocytic leukemia, B-cell prolymphocytic leukemia, lymphoplasmacytic lymphoma, Mantle cell lymphoma, follicular lymphoma, cutaneous follicle center lymphoma, marginal zone B-cell lymphoma, nodal marginal zone lymphoma, splenic marginal zone B-cell lymphoma, hairy cell leukemia, diffuse large B-cell lymphoma, Burkitt's lymphoma, plasmacytoma, plasma cell myeloma, and the like. Also, it may be a hematopoietic tumor. The hematopoietic tumor includes leukemia, lymphoma and myeloma. Examples include acute lymphatic leukemia, chronic lymphocyte leukemia, acute myelocytic leukemia, chronic myelocytic leukemia, non-Hodgkin's lymphoma, Hodgkin's lymphoma and multiple myeloma. Furthermore, in the chronic myelocytic leukemia and chronic lymphocyte leukemia, conditions at any disease stage are included, and any of chronic phase, accelerating phase and blastic phase are included.

The antibody of the present invention can be administered as a therapeutic agent alone, but generally, it is preferred to provide it as a pharmaceutical preparation produced by an appropriate method well known in the technical field of pharmaceutics, by mixing it with one or more pharmaceutically acceptable carriers.

It is preferred to select a route of administration which is most effective in treatment. Examples include oral administration and parenteral administration, such as buccal, tracheal, rectal, subcutaneous, intramuscular or intravenous administration, and among them, intravenous administration is preferred.

The dosage form includes sprays, capsules, tablets, granules, syrups, emulsions, suppositories, injections, ointments, tapes and the like.

The pharmaceutical preparation suitable for oral administration includes emulsions, syrups, capsules, tablets, powders, granules and the like.

Liquid preparations such as emulsions and syrups can be produced using, as additives, water; sugars such as sucrose, sorbitol and fructose; glycols such as polyethylene glycol and propylene glycol; oils such as sesame oil, olive oil and soybean oil; antiseptics such as p-hydroxybenzoic acid esters; flavors such as strawberry flavor and peppermint; and the like.

Capsules, tablets, powders, granules and the like can be produced using, as additives, excipients such as lactose, glucose, sucrose and mannitol; disintegrating agents such as starch and sodium alginate; lubricants such as magnesium stearate and talc; binders such as polyvinyl alcohol, hydroxypropylcellulose and gelatin; surfactants such as fatty acid ester; plasticizers such as glycerin; and the like.

The pharmaceutical preparation suitable for parenteral administration includes injections, suppositories, sprays and the like.

Injections can be prepared using a carrier such as a salt solution, a glucose solution or a mixture of both. Also, powdered injections can be prepared by freeze-drying the glycoprotein in the usual way and adding sodium chloride thereto.

Suppositories can be prepared using a carrier such as cacao butter, hydrogenated fat or carboxylic acid.

Sprays can be prepared using the antibody as such or in combination with a carrier which does not stimulate the buccal or airway mucosae of the patient and can facilitate absorption of the antibody or antibody fragment thereof by dispersing it as fine particles.

The carrier includes lactose, glycerol and the like. Depending on the properties of the antibody of the present invention and the carrier used, it is possible to produce pharmaceutical preparations such as aerosols and dry powders. In addition, the components exemplified as additives for oral preparations can also be added to the parenteral preparations.

Although the dose or the frequency of administration varies depending on intended therapeutic effect, administration method, treating period, age, body weight and the like, it is usually 0.01 mg/kg to 20 mg/kg per day per adult.

A process for producing the humanized antibody which specifically binds to CD10 and has cytotoxic activity or the antibody fragment thereof used in the present invention, a method for evaluating the activity and a method for using it are specifically described below.

1. Preparation of humanized antibody

(1) Construction of a vector for expression of humanized antibody

A vector for expression of humanized antibody is an expression vector for animal cell into which DNAs encoding CH and CL of a human antibody are inserted, which can be constructed by cloning each of DNAs encoding CH and CL of a human antibody into an expression vector for animal cell.

The C regions of a human antibody may be any of CH or CL in human antibody. Examples include CH belonging to IgG1 subclass of a human antibody, CL belonging to κ class of a human antibody, and the like. As DNAs encoding CH and CL of a human antibody, a chromosomal DNA comprising an exon and an intron can be used, and a cDNA can also be used. As the expression vector for animal cell, any vector can be used, so long as a gene encoding the C region of a human antibody can be inserted thereinto and expressed therein. Examples include pAGE107 [*Cytotechnol*., 3, 133 (1990)], pAGE103 [J. Biochem., 101, 1307 (1987)], pHSG274 [Gene, 27, 223 (1984)], pKCR [Proc. Natl. Acad. Sci. USA, 78, 1527 (1981), pSG1bd2-4 [Cytotechnol., 4, 173 (1990)], pSE1UKSed1-3 [Cytotechnol., 13, 79 (1993)] and the like. The promoter and enhancer used for the expression vector for animal cell include SV40 early promoter [J. Biochem., 101, 1307 (1987)], Moloney mouse leukemia virus LTR [Biochem. Biophys. Res. Commun., 149, 960 (1987)], immunoglobulin H chain promoter [Cell, 41, 479 (1985)] and enhancer [Cell, 33, 717 (1983)], and the like.

The vector for expression of humanized antibody may be either of a type where antibody H chain and L chain exist on separate vectors or where they are on the same vector (tandem type). In respect of easiness of construction of a humanized antibody expression vector, easiness of introduction into animal cells, and balance between the expression levels of the H and L chains of an antibody in animal cells, a tandem type of the vector for expression of humanized antibody is more preferred [J. Immunol. Methods, 167, 271 (1994)]. The tandem type of the vector for expression of humanized antibody includes pKANTEX93 (WO97/10354), pEE18 [Hybridoma, 17, 559 (1998)] and the like.

The constructed vector for expression of humanized antibody can be used for expression of humanized antibody in animal cells.

(2) Construction of cDNA encoding V region of humanized antibody

cDNAs encoding VH and VL of a humanized antibody can be constructed in the following manner. First, amino acid sequences of FRs of VH and VL of a human antibody for grafting CDRs of VH and VL of a desired non-human animal-derived antibody are selected. The amino acid sequences of FRs of VH and VL of a human antibody may be any of those derived from human antibodies. Suitable sequences include the amino acid sequences of FRs of VHs and VLs of human antibodies registered at databases such as Protein Data Bank, and the amino acid sequences common to subgroups of FRs of VHs and VLs of human antibodies [Sequences of Proteins of Immunological Interest, US Dept. Health and Human Services (1991)]. In order to prepare a humanized antibody having a sufficient activity, it is preferred to select amino acid sequences having as high a homology as possible (at least 60% or more) with the amino acid sequences of FRs of VH and VL of the desired non-human animal-derived antibody.

Next, the amino acid sequences of CDRs of VH and VL of the desired on-human animal-derived antibody are grafted to the selected amino acid sequences of FRs of VH and VL of a human antibody to design amino acid sequences of VH and VL of a humanized antibody. The designed amino acid sequences are converted into nucleotide sequences taking into consideration the frequency of codon usage found in the nucleotide sequences of antibody genes [Sequences of Proteins of Immunological Interest, US Dept. Health and Human Services (1991)], and the desired nucleotide sequence of the non-human animal antibody, and nucleotide sequences encoding the amino acid sequences of VH and VL of the humanized antibody are designed. Several synthetic DNAs constituting approximately 100 to 200 nucleotides are synthesized based on the designed DNA sequences, and PCR is carried out using the synthetic DNAs. It is preferred to design 4 to 6 synthetic DNA for each of VH and VL in view of the reaction efficiency of PCR and the lengths of DNAs that can be synthesized. Cloning into the vector for expression of the humanized antibody of the present invention constructed in the above 1 (1) can be easily carried out by introducing recognition sequences for appropriate restriction enzymes to the 5'-terminals of synthetic DNAs present on both ends. After the PCR, the amplification products are cloned into a plasmid vector such as pBluescript SK(-) (manufactured by Stratagene) and the nucleotide sequences are determined by the method described in the above 1 (2) to obtain a plasmid nucleotide sequences encoding the amino acid sequences of VH and VL of the desired humanized antibody.

(3) Modification of amino acid sequence of V region of humanized antibody

It is known that a humanized antibody prepared merely by grafting CDRs of VH and VL of the desired non-human animal-derived antibody to FRs of VH and VL of a human antibody has a lower antigen binding activity compared with the original non-human animal-derived antibody [*BIO/TECHNOLOGY*, 9, 266 (1991)]. This is probably because in VH and VL of the original non-human animal-derived antibody, not only CDRs but also some of the amino acid residues in FRs are involved directly or indirectly in the antigen binding activity, and such amino acid residues are replaced by amino acid residues derived from FRs of VH and VL of the human antibody by CDR grafting. In order to solve this problem, it has been conducted in a humanized antibody that, among the amino acid sequence of FRs in VH and VL of human antibody, an amino acid residue which directly relates to binding to the antigen, or amino acid residue which indirectly relates to binding to an antigen by interacting with an amino acid residue in CDRs or by maintaining the three-dimensional structure of an antibody, is identified and that the amino acid residues are modified to amino acid residues found in the original antibody of non-human animal to thereby increase the lowered antigen binding activity [BIO/TECHNOLOGY, 9, 266 (1991)]. In the preparation of a humanized antibody, it is most important to efficiently identify the amino acid residues relating to the antigen binding activity in FR. For the efficient identification, construction and analyses of the tertiary structures of antibodies have been carried out by X ray crystallography [J. Mol. Biol., 112, 535 (1977)], computer modeling *[Protein Engineering,* 7, 1501 (1994)], or the like. Although these studies on the three-dimensional structures of antibodies have provided much information useful for the preparation of humanized antibodies, no method for preparing a humanized antibody which is adaptable to any type of antibody has been established yet. That is, at present, it is still necessary to make trial-and-error approaches, for example, modifications are prepared for each antibody and the correlation to each antigen binding activity is examined.

Modification of the amino acid residues in FRs of VH and VL of a human antibody can be achieved by PCR using synthetic DNAs for modification. The nucleotide sequence of the PCR amplification product is determined by the method described in the above 1 (2) to confirm that the desired modification has been achieved.

(4) Construction of a humanized antibody expression vector

A humanized antibody expression vector can be constructed by cloning the cDNAs encoding VH and VL of the humanized antibody constructed in the above 1 (2) and (3) into sites upstream of the DNAs encoding CH and CL of a human antibody in the vector for expression of the humanized antibody described in the above 1 (1). For example, a humanized antibody expression vector can be constructed by introducing recognition sequences for appropriate restriction enzymes to the 5'-terminals of synthetic DNAs present on both ends among the synthetic DNAs used for constructing VH and VL of the humanized antibody in the above 1 (2) and (3), and cloning them into sites upstream of the DNAs encoding CH and CL of a human antibody in the vector for humanized antibody expression described in the above 1 (1) so as to express them in an appropriate form.

(5) Transient expression of humanized antibodies

In order to efficiently evaluate the antigen binding activity of various humanized antibodies prepared, the humanized antibodies can be expressed transiently using the humanized antibody expression vector as described in the above 1 (4) or the modified expression vector thereof. Any cell can be used as a host cell into which expression vector is introduced, so long as the host cell can express a humanized antibody. Generally, COS-7 cell (ATCC CRL1651) is used in view of its high expression level [Methods in Nucleic Acids Res., CRC Press, p. 283 (1991)]. Examples of the method for introducing the expression vector into COS-7 cell include a DEAE-dextran method [Methods in Nucleic Acids Res., CRC Press, p. 283 (1991)], a lipofection method [Proc. Natl. Acad. Sci. USA, 84, 7413 (1987)], and the like.

After introduction of the expression vector, the expression level and antigen binding activity of the humanized antibody in the culture supernatant can be determined by the enzyme immunoassay [ELISA; Antibodies-A Laboratory Manual, Cold Spring Harbor Laboratory, Chapter 14 (1988), Monoclonal Antibodies: Principles and Practice, Academic Press Limited (1996)] and the like.

(6) Stable expression of humanized antibody

A transformant capable of stably expressing a humanized antibody can be obtained by introducing the humanized antibody expression vector described in the above 1 (4) into an appropriate host cell. The method for introducing an expression vector into a host cell includes electroporation [Cytotechnology, 3, 133 (1990)] and the like.

The host cell to which the humanized antibody expression vector is introduced may be any cell, so long as it is a host cell capable of expressing a humanized antibody. Examples include mouse SP2/0-Ag14 cell (ATCC CRL1581), mouse P3X63-Ag8.653 cell (ATCC CRL1580), dihydrofolate reductase gene (dhfr)-defected CHO cell [Pro. Natl. Acad.

Sci. U.S.A., 77, 4216 (1980)], rat YB2/3HL.P2.G11.16Ag.20 cell (YB2/0 cell; ATCC CRL1662) and the like. In order to express a humanized antibody having high ADCC activity, preferred examples include a cell resistant to a lectin which recognizes a sugar chain in which 1-position of fucose is bound to 6-position of *N*-acetylglucosamine in the reducing end through α-bond in the complex *N*-glycoside-linked sugar chain, such as a host cell in which genome is modified so that an enzyme relating to the synthesis of an intracellular sugar nucleotide, GDP-fucose is inactivated, and a cell in which genome is modified so that an enzyme relating to the modification of a sugar chain wherein 1-position of fucose is bound to 6-position of *N*-acetylglucosamine in the reducing end through α-bond in the complex N-glycoside-linked sugar chain is inactivated. Specific preferred examples include a host cell in which a gene encoding α1,6-fucosyltransferase in the host cell is knocked out (WO02/31140, WO03/85107).

After the introduction of the expression vector, the transformant capable of stably expressing the humanized antibody can be selected by culturing in a medium for animal cell culture containing an agent such as G418 sulfate (G418; manufactured by SIGMA) (J. Immunol. Methods, 167, 271 (1994). Examples of the media for animal cell culture include RPMI1640 medium (manufactured by Nissui Pharmaceutical Co., Ltd.), GIT medium (manufactured by Nihon Pharmaceutical Co., Ltd.), EX-CELL 302 medium (manufactured by JRH), IMDM medium (manufactured by GIBCO BRL), Hybridoma-SFM medium (manufactured by GIBCO BRL), and media prepared by adding various additives such as fetal bovine serum (FBS) to these media. By culturing the obtained transformant in the medium, the humanized antibody can be formed and accumulated in the culture supernatant. The amount of the humanized antibody expressed and antigen binding activity of the humanized antibody in the culture supernatant can be measured by ELISA or the like. The level of the humanized antibody expressed by the transformant can be increased by utilizing a dhfr gene amplification system or the like [J. Immnol. Methods, 167, 271 (1994)].

The humanized antibody can be purified from the culture supernatant of the transformant using a protein A column [Antibodies: A Laboratory Manual, Cold Spring Harbor Laboratory, Chapter 8 (1988); Monoclonal Antibodies: Principles and Practice, Academic Press Limited (1996)]. In addition, purification methods generally employed for the purification of proteins can also be used. For example, the purification can be carried out by combinations of gel filtration, ion exchange chromatography, ultrafiltration and the like. The molecular weight of the H chain, L chain or whole antibody molecule of the purified humanized antibody can be measured by polyacrylamide gel electrophoresis [SDS-PAGE; Nature, 227, 680 (1970)], Western blotting [Antibodies: A Laboratory Manual, Cold Spring Harbor Laboratory, Chapter 12 (1988); Monoclonal Antibodies: Principles and Practice, Academic Press Limited (1996)], or the like.

2. Preparation of antibody fragment

The antibody fragment can be prepared from the humanized antibody mentioned in the above 1 by genetic engineering techniques or protein chemical techniques. The antibody fragment includes Fab, $F(ab')_2$, Fab', scFv, diabody, dsFv, a peptide comprising CDR, and the like.

(1) Preparation of Fab

Fab can be prepared by treating IgG with protease, papain. After the treatment with papain, it is possible to collect as a uniform Fab by passing through a protein A column to separate from IgG molecule and Fc fragment provided that the original antibody is an IgG subclass having a binding property to protein A [Monoclonal Antibodies: Principles and Practice, third edition (1995)]. In the case of an antibody of an IgG subclass having no binding property to protein A, Fab can be collected by ion-exchange chromatography at a fraction eluted by a low salt concentration [*Monoclonal Antibodies: Principles and Practice*, third edition (1995)]. Fab can also be prepared by genetic engineering techniques using *E. coli*. For example, DNA encoding the V region of the antibody mentioned in the above 1 (2) and (3) is cloned into a vector for expression of Fab whereupon Fab expression vector can be prepared. With regard to vector for expression of Fab, any vector may be used so long as DNA for Fab can be inserted and expressed. Example include pIT 106 [Science, 240, 1041 (1988)] and the like. Fab expression vector is introduced into an appropriate *E. coli* whereby Fab can be formed and accumulated in an inclusion body or a periplasmic space. From the inclusion body, active Fab can be obtained by a refolding method generally used for proteins and, when expressed in periplasmic space, active Fab leaks out in a culture supernatant. After the refolding or from the culture supernatant, a uniform Fab can be purified using a column to which antigen is bound [Antibody Engineering, A Practical Guide, W. H. Freeman and Company (1992)].

(2) Preparation of $F(ab')_2$

$F(ab')_2$ can be prepared by treating IgG with protease, pepsin. After the treatment with pepsin, it can be recovered as a uniform $F(ab')_2$ by the same purifying operation as in the case of Fab [Monoclonal Antibodies: Principles and Practice, third edition, Academic Press (1995)]. It can also be prepared by a method in which Fab' mentioned in the above 2 (3) is treated with a maleimide such as o-PDM or bismaleimide to form a thioether bond or by a method in which it is treated with DTNB to form an S-S bond [Antibody Engineering, A Practical Approach, IRL Press (1996)].

(3) Preparation of Fab'

Fab' can be prepared by genetic engineering techniques using *E. coli*. For example, DNA encoding the V region of the antibody mentioned in the above 1 (2) and (3) is cloned into a vector for expression of Fab' whereupon Fab' expression vector can be prepared. With regard to a vector for expression of Fab', any vector may be used so long as DNA for Fab' can be inserted and expressed. An example thereof is pAK 19 [Bio/Technology, 10, 163 (1992)]. The Fab' expression vector is introduced into an appropriate *E. coli* to form and accumulate Fab' in an inclusion body or periplasmic space. From the inclusion body, active Fab' can be obtained by a refolding method which is generally used in proteins and, when the Fab' is expressed in periplasmic space, it can be recovered extracellularly by disrupting the cell with a treatment such as partial digestion by lysozyme, osmotic shock and sonication. After the refolding or from the disrupted cell solution, a uniform Fab' can be purified using a protein G column or the like [Antibody Engineering, A Practical Approach, IRL Press (1996)].

(4) Preparation of scFv

scFv can be prepared using phage or *E. coli* by genetic engineering techniques. For example, DNAs encoding VH and VL of the antibody mentioned in the above 1 (2) and (3) are ligated via a DNA encoding a polypeptide linker comprising the amino acid sequence of 12 or more residues to thereby prepare a DNA encoding scFv. The prepared DNA is cloned into a vector for expression of scFv whereupon an scFv expression vector can be prepared. With regard to the vector for expression of scFv, any vector may be used so long as the DNA of scFv can be inserted and expressed. Examples include pCANTAB5E (manufactured by Pharmacia), pHFA [Hum. Antibodies & Hybridomas, 5, 48 (1994)], and the like. When scFv expression vector is introduced into an appropriate *E. coli* and a helper phage is infected, a phage which expresses scFv on the phage surface in a fused form with the surface protein of the phage can be obtained. Also, scFv can be formed and accumulated in periplasmic space or an inclusion body *of E. coli* into which scFv expression vector is introduced. From the inclusion body, active scFv can be obtained by a refolding method generally used for proteins and, when scFv is expressed in periplasmic space, it can be recovered extracellularly by disrupting the cell with a treatment such as partial digestion by lysozyme, osmotic shock and sonication. After the refolding or from the disrupted cell solution, a uniform scFv can be purified using cation-exchange chromatography or the like *[*Antibody Engineering, A Practical Approach, IRL Press (1996)].

(5) Preparation of diabody

Diabody can be prepared by changing the size of the polypeptide linker in the above preparation of scFv to about 3 to 10 residues. When VH and VL of one kind of antibody are used, a divalent diabody can be prepared, and when VH and VL of two kinds of antibodies are used, a diabody having bispecificity can be prepared [FEBS Letters, 453, 164 (1999), Int. J. Cancer, 77, 763 (1998)].

(6) Preparation of dsFv

dsFv can be prepared using *E. coli* by genetic engineering techniques. Firstly, mutation is introduced into an appropriate position of DNAs encoding VH and VL of the antibody mentioned in the above 1 (2) and (3) to prepare DNAs in which an encoded amino acid residue is replaced with cysteine. Each DNA prepared is cloned into a vector for expression of dsFv whereby an expression vector of VH and VL can be prepared. With regard to a vector for expression of dsFv, any vector may be used so long as the DNA for dsFv can be inserted and expressed. Examples include pULI 9 *[*Protein Engineering, 7, 697 (1994)] and the like. The expression vector of VH and VL is introduced into an appropriate *E. coli* and dsFv is formed and accumulated in an inclusion body or periplasmic space. VH and VL are obtained from the inclusion body or periplasmic space, mixed and subjected to a refolding method generally used for proteins to thereby obtain active dsFv. After the refolding, it can be further purified by ion-exchange chromatography, a gel filtration, and the like *[*Protein Engineering, 7, 697 (1994)].

(7) Preparation of peptide comprising CDR

A peptide comprising CDR can be prepared by a chemical synthesis method such as an Fmoc method or a tBoc method. Furthermore, DNA encoding a peptide comprising CDR is prepared and the resulting DNA is cloned into an appropriate vector for expression whereby a peptide comprising CDR expression vector can be prepared. With regard to a vector for expression, any vector may be used so long as the DNA which encodes peptide comprising CDR can be inserted and expressed. Examples include pLEX (manufactured by Invitrogen), pAX4a+ (manufactured by Invitrogen) and the like. The expression vector is introduced into an appropriate *E. coli* and formed and accumulated in an inclusion body or periplasmic space. From the inclusion body or the periplasmic space, peptide comprising CDR is obtained and can be purified by ion-exchange chromatography and a gel filtration [Protein Engineering, 7, 697 (1994)].

3. Evaluation of activity of the antibody of the present invention

The binding property between the purified antibody of the present invention and the antigen and the binding activity to a CD10-expressing cell can be measured by ELISA, immunofluorescent method [Cancer Immunol. Immunother., 36, 373 (1993)], surface plasmon resonance using BIAcore™, and the like. The cytotoxic activity for antigen-positive cultured cells can be evaluated by measuring complement-dependent cytotoxicity (hereinafter referred to as CDC activity), antibody-dependent cell-mediated cytotoxicity (hereinafter referred to as ADCC activity) and the like [Cancer Immunol. Immunother., 36, 373 (1993)]. The change of the amount of produced cytokine can be measured by ELISA, immunofluorescent method or the like.

4. Method for using humanized antibody or antibody fragment

Since a humanized antibody is derived from amino acid sequences of a human antibody, it has high effect in the human body and has low immunogenicity in comparison with an antibody derived from a non-human animal and a chimeric antibody, therefore it is expected that the effect is sustained for a long term.

Also, the ADCC activity of the antibody or the antibody fragment thereof can be enhanced by modifying a complex *N*-glycoside-linked sugar chain bound to the Fc region of the antibody to a sugar chain in which fucose is not bound to *N*-acetylglucosamine in the reducing end in the sugar chain.

The present invention is explained below based on Examples; however, the present invention is not limited thereto.

Examples

Example 1

*In vitro* analysis of expression level of cell surface antigen in cells resistant to an anti-CD20 antibody

(1) Establishment of non-Hodgkin's lymphoma cell line RTX/Raji cells being exposed to rituximab for a long period *in vitro*

Raji cells (JCRB 9012) which is a non-Hodgkin's lymphoma cell line was cultured for 59 days by subculturing every 3 to 4 days in a medium containing rituximab (manufactured by Chugai Pharmaceutical). With regard to the medium, one which was prepared in such a manner that rituximab was added to an RPMI-1640 (manufactured by Invitrogen) to which 10 V/V % of heat inactivated calf serum (manufactured by Invitrogen), 1.56 V/V % of guinea pig serum (manufactured by ARK Resource) and 1 V/V % of penicillin-streptomycin solution (manufactured by Invitrogen) were added, was used. Concentrations of rituximab added to the medium were 0.08 μg/ml for 1 to 7 subculture(s), 10 μg/ml for 8 to 9 subcultures and 1.0 μg/ml for 10 to 16 subcultures. Cells prepared as such were called RTX/Raji cells.

In the case of passage maintenance of the RTX/Raji cells, RPMI-1640 medium to which 10 V/V % of thermally inactivated calf serum, 0.8V/V % of guinea pig serum (Sigma) and 1V/V % of penicillin-streptomycin solution were added was used as a medium.

(2) Investigations for comparison of expression level of cell surface antigen in the RTX/Raji cells and Raji cells which were parent cell line thereof

Expressed amounts of CD 10, CD 19, CD20 and CD22 on the cell surfaces of RTX/Raji cells and Raji cells were analyzed by flow cytometry.

About $2 \times 10^7$ Raji cells or Raji/RTX cells were prepared and suspended in an FACS buffer [a phosphate buffered saline without calcium and magnesium (PBS, manufactured by Invitrogen) to which 0.1 w/v% sodium azide, 1 w/v% bovine serum albumin (manufactured by Seikagaku Corporation) and 2 mmol/L of ethylenediaminetetraacetic acid (EDTA, manufactured by Nacalai Tesque) were added]. The supernatant liquid was removed, cell pellets were loosened and 10 μL of FcR blocking reagent (manufactured by Miltenyi Biotec) was added thereto. After the mixture was allowed to stand on ice for about 30 minutes, an FACS buffer was added thereto and washed by centrifugal separation (about $5000 \times g$ for 1 minutes at 4°C) was repeated three times. An FACS buffer was added so as to give a cell density of $2 \times 10^7$ cells/ml for suspension and each 50 μL thereof was aliquated into a tube. The FACS buffer (50 μL) was added

to each tube to suspend and the primary antibody solution was added thereto in an amount of 2.5 μL per tube. With regard to the primary antibody solution, each of mouse anti-CD10 antibody NL-1 [Proc. Natl. Acad. Sci. USA, 79, 4386 (1982)], anti-CD20 antibody B1 (manufactured by Beckman Coulter), FITC-labeled anti-CD19 antibody J4.119 (manufactured by Beckman Coulter) and FITC-labeled anti-CD22 antibody SJ.10.1H11 (manufactured by Beckman Coulter) was used. With regard to an iso-type control antibody, each of FITC-labeled isotypic control IgG1 (manufactured by Beckman Coulter), isotypic control IgG2a (manufactured by BD Bioscience) and FITC-labeled isotypic control IgG2a (manufactured by BD Bioscience) was used. The above was allowed to stand under shielding from light for about 30 minutes on ice, washed with an FACS buffer by centrifugal separation (about 5000 × g for 1 minute at 4˚C) was carried out three times. To the tube in which reaction with the mouse anti-CD10 antibody NL-1 and the anti-CD20 antibody B1 was carried out was further added 100 μL of an FITC-labeled $F(ab)_2$ antimouse IgG (manufactured by ICN/Cappel) as the secondary antibody solution, the mixture was allowed to stand under shielding from light for about 30 minutes on ice and washed with an FACS buffer by centrifugal separation (about 5000 × g for 1 minute at 4˚C) was carried out for three minutes. After removal of the supernatant, 500 μL of the residue was collected from the tube and suspended in an isoflow (manufactured by Beckman Coulter). It was filtered by a Nylon mesh and transferred to a test tube to prepare a sample to be measured. Expression of CD antigen on the cell surface was measured using a flow cytometer Epics Elite (manufactured by Beckman Coulter). The result is shown in Fig. 1.

The expression level of CD10 was promoted in the RTX/Raji cells in which the anti-CD20 antibody had been exposed, and the enhanced CD10 was higher in the RTX/Raji cells than that of Raji cells. The expression levels of CD19 and CD22, were lower in the RTX/Raji cells than the Raji cells. Furthermore, expression of CD20 disappeared in the RTX/Raji cells.

Example 2

Cytotoxic activity of anti-CD10 antibody against RTX/Raji cells

(1) Comparative investigation of ADCC activities in RTX/Raji cells and in Raji cells

ADCC activities of the anti-CD 10 human chimeric antibody Ms705/CD10 prepared in Reference Example 1 and the anti-CD20 human chimeric antibody rituximab were measured by a $^{51}Cr$ release method using RTX/Raji cells and Raji cells as target cells and human PBMC as an effector cell.

Raji cells and Raji/RTX cells were suspended in an RPMI-1640 (hereinafter referred to as an assay medium) to which 10V/V % of heat inactivated calf serum and 1 V/V % of penicillin-streptomycin solution were added to make $2 \times 10^6$ cells/mL. To 1 mL of the cell suspension was added 50 μL of radioisotope of sodium chromate ($Na_2{}^{51}CrO_4$, 37 MBq/mL; hereinafter, just referred to as $^{51}Cr$, manufactured by Perkin Elmer). That was cultured for about 1.5 hours in an incubator under conditions of 37˚C and 5V/V % of carbon dioxide gas (95V/V % of air). After washing by centrifugal separation (about 300 × g for 3 minutes at room temperature) in the assay medium was carried out twice, it was suspended in about 10 mL of an assay medium, allowed to stand for about 30 minutes in ice and $^{51}Cr$ which was naturally released, was removed by centrifugal separation (about 300 × g for 3 minutes at room temperature). Cells were suspended on a medium, was carried out so as to give a cell density of $3 \times 10^5$ cells/mL to prepare a target cell suspension. Healthy human peripheral blood treated with heparin was transferred to a Lymphoprep Tube (manufactured by Nycomed), diluted about 2-fold with a physiological saline and centrifuged at room temperature (about 800 × g for 20 minutes). A monocyte fraction was diluted 2-fold or more with PBS, centrifuged (about 300 × g for 5 minutes at room temperature) and washed by centrifugal separation (about 300 × g for 3 minutes at room temperature) in the assay medium, twice. Cells were suspended in an assay medium to give a cell density of $7.5 \times 10^6$ cells/mL whereupon an effector cell suspension was prepared. Any of target cell suspension (50 μL), 50 μL of effector cell suspension and 50 μL of each antibody solution diluted by an assay medium was added to a 96-well U-shped bottom plate for cell culturing. The plate was centrifuged at room temperature (about 300 × g for 10 seconds) and allowed to stand in an incubator for about 3.5 hours under conditions of 37˚C and 5V/V % of carbon dioxide gas (95V/V % of air). Then, the supernatant was collected by centrifugal separation (about 800 × g for 5 minutes at room temperature), 30 μL thereof was transferred to a Luma Plate (manufactured by Perkin Elmer) and dried by being allowed to stand at about 60˚C for 2 hours or more and radiation activity was measured by Top Count NXT (manufactured by Perkin Elmer). The result is shown in Fig. 2.

In RTX/Raji cells in which the anti-CD20 antibody had been exposed, the ADCC activity of the anti-CD 10 antibody was enhanced (upper drawing of Fig. 2) and the ADCC activity by the anti-CD20 antibody disappeared (lower drawing of Fig. 2). With regard to the ADCC activity of the anti-CD10 antibody, it was higher in the RTX/Raji cells than in Raji cells (upper drawing of Fig. 2).

(2) Comparative investigation of CDC activities in the RTX/Raji cells and in the Raji cells

CDC activities of anti-CD 10 human chimeric antibody Ms705/CD10 and anti-CD20 human chimeric antibody rituximab were measured by a WST-1 assay method using RTX/Raji cells and Raji cell as target cells.

Raji cells and Raji/RTX cells suspended in an assay medium in cell density of $1 \times 10^5$ cells/mL were added to a 96-well flat bottom plate at 50 μL/well. Then each antibody diluted with an assay medium was added thereto in an amount of 25 μL/well. Human complement serum (manufactured by Sigma) diluted 4-fold by a medium was further added thereto in an amount of 25 μL/well. After culturing was carried out in an incubator for 72 hours at 37°C in the presence of 5V/V % of $CO_2$, 10 μL/well of a WST-1 reagent (manufactured by Roche Diagnostics) was added thereto, followed by culturing for 3 hours or more. Absorbance at 450 nm (as a control wavelength: 650 nm) was measured using a microplate reader SpectraMax 250 (manufactured by Wako Pure Chemical). The result is shown in Fig. 3.

The CDC activity of the anti-CD 10 antibody was much more increased in the RTX/Raji cells in which the anti-CD20 antibody had been exposed than in the Raji cells (upper drawing of Fig. 3). The CDC activity of the anti-CD20 antibody in RTX/Raji cells disappeared, although it was detected in Raji cells (lower drawing of Fig. 3).

(3) Comparative investigation for *in vivo* anti-tumor effects in RTX/Raji cells and Raji cells

Anti-CD10 antibody, Ms705/CD10 or anti-CD20 antibody, rituximab was administered to mice to which RTX/Raji cells or Raji cells were transplanted and *in vivo* therapeutic effects were investigated by examining changes in tumor volume.

The RTX/Raji cells or Raji cells ($1 \times 10^7$ cells/mouse) were transplanted into male SCID mice to which 20 μL/mouse of an anti-Asialo GM1 antibody (manufactured by Wako Pure Chemical) had been intraperitoneally administered on the seventh day before the administration and on the day before the administration of the antibody. Long diameter and short diameter of tumors were measured and the volume of tumor mass was calculated as Formula 1. When mean value of the tumor volume became about 150 $mm^3$, the mice were divided into three groups each comprising five mice whereby the tumor volumes were not unevenly distributed for each group.

Formula 1:

$$\text{Tumor volume } (mm^3) = \text{long diameter (mm)} \times \text{short diameter (mm)} \times \text{short diameter (mm)} \times 0.5$$

The day when the mice were grouped into three was defined as "day 0". Drug administration was started from day 0. For the group treated with the anti-CD10 antibody, 100 μg/mouse of the anti- CD10 human chimeric antibody, Ms705/CD10 was administered intravenously twice a week, for eight times in total. For the group treated with the anti-CD20 antibody, 100 μg/mouse of the anti-CD20 anti body, rituximab was administered intravenously twice a week, for eight times in total. For the control group, 200 μL/mouse of a physiological saline solution was administered intravenously twice a week for eight times in total. During day 0 and day 28, the volume of the tumors was measured twice a week.

The ratio of V to V0 (V/V0) in which V0 is tumor volume on day 0 and V is tumor volume for each day measured was calculated for each mouse. Mean value of V/V0 was calculated for each group. The value (T/C) in which the mean value of V/V0 of the group treated with Ms705/CD 10 or the mean value of V/V0 of the group treated with rituximab was divided by the mean value of V/V0 of the control group was calculated.

Changes in T/C of the group treated with the anti-CD10 antibody and the group treated with the anti-CD20 antibody are shown in Fig. 4. T/C of the group treated with the anti-CD10 antibody in the mice to which RTX/Raji cells were transplanted was lower than that in the mice to which Raji cells were transplanted. From these results, it is now apparent that the therapeutic effect of the anti-CD10 antibody is enhanced in tumor cells in which the anti-CD20 antibody has been exposed. On the other hand, T/C of the group treated with the anti-CD20 antibody in the mice to which the RTX/Raji cells were transplanted showed higher than that in the mice to which Raji cells were transplanted. From these results, it is now apparent that the therapeutic effect of the anti-CD20 antibody is reduced in tumor cells in which the anti-CD20 antibody has been exposed.

Example 3

*In vivo* therapeutic effect of anti-CD 10 antibody in tumor-bearing mice to which anti-CD20 antibody had been administered

Mice bearing non-Hodgkin's lymphoma cell line were administered with the anti-CD20 antibody, rituximab, then the mice were administered with either anti-CD 10 humanized antibody HV2LV9 MS705 prepared in Reference Example 2 or retuximab. The *in vivo* therapeutic effect of HV2LV9 MS705 and rituximab were compared.

To the right frank of the male SCID mice, $1 \times 10^7$ Raji cells per one area were transplanted. On the seventh day before the transplantation and on the day before the transplantation, 20 μL of anti-Asialo GM1 antibody (manufactured by Wako Pure Chemical) was intraperitoneally administered per mouse. After 20 days from the transplantation, administration of rituximab was started to 20 mice in which the tumor volume reached 107.0 to 322.4 $mm^3$ (mean ± SD: 206.9 ± 66.6 $mm^3$). The day when administration of rituximab was started was defined as day 0. Rituximab (100 μg/mouse) was administered to the tail vein on days 0, 3, 7 and 10 four times in total. On the day of the administration, long diameter and short diameter of tumor were measured to calculate the tumor volume. On day 14, the individuals in which the tumor volume of day 14 was smaller than tumor volume of day 0 or the individuals in which the tumor volume decreased for two consecutive times or more were excluded. Furthermore, the individuals in which the tumor volume on day 14 was out of range of the mean value ± standard deviation of the 20 mice to which rituximab had been administered were excluded. Eleven mice which did not meet the above exclusion criteria among the twenty were used for the experiment.

Eleven mice were divided into two groups based on the tumor volume on day 14 so as not to make imbalance in tumor volumes. Then, one group was intravenously administered with 100 μg/mouse of rituximab on days 14, 17, 21 and 24 four times in total (n=5). Another group was intravenously administered with 100 μg/mouse of an anti-CD10 humanized antibody, HV2LV9 MS705 on days 14, 17, 21 and 24 four times in total (n = 6). Tumor volumes of the groups treated with the anti-CD20 antibody and with the anti-CD10 antibody were measured on the days of antibody administration.

The ratio of V to V0 (V/V0) was measured for each mouse in which V0 is tumor volume of day 0 and V is tumor volume on the measured day. With regard to the difference of V/V0 recognized between the groups, its statistic significant difference was calculated using a statistic analysis software SAS (Release 8.2, manufactured by SAS Institute). When P value was 0.05 or less, the difference was judged to be significant.

V/V0 of the group treated with the anti-CD 10 antibody was significantly smaller than that of the group treated with the anti-CD20 antibody (Fig. 5). From that result, it is now apparent that sequential treatment with anti-CD20 antibody followed by the anti-CD 10 antibody gives higher therapeutic effect than the continuous treatment of the anti-CD20 antibody.

Example 4

*In vivo* therapeutic effect of anti-CD10 antibody in non-Hodgkin's lymphoma resistant to anti-CD20 antibody

Therapeutic effect of anti-CD10 antibody and anti-CD20 antibody were investigated in mice bearing non-Hodgkin's lymphoma cell line Namalwa cells (National Institutes of Health, U. S. A.).

Namalwa cells were transplanted to right frank of male SCID mice in an amount of $5 \times 10^6$ cells per area. On the day before the transplantation, an anti-Asialo GM1 antibody (Wako Pure Chemical) was intraperitoneally administered in an amount of 20 μL per mouse. When tumor volume reached 200.1 to 399.7 $mm^3$ (mean value ± standard deviation was 289.7 ± 63.2 $mm^3$), the mice were divided into three groups each comprising five mice so that tumor volumes were not unbalanced among the groups. For the group treated with the anti-CD 10 antibody, 100 μg/mouse of the anti-CD10 humanized antibody, HV2LV9MS705 was administered into tail vein twice a week for six times in total. For the group treated with the anti-CD20 antibody, 100 μg/mouse of the anti-CD20 antibody rituximab (manufactured by Chugai Pharmaceutical) was administered into tail vein twice a week for six times in total. For the control group, 200 μL/mouse of a physiological saline solution was administered into tail vein twice a week for six times in total. From day 0 to day 28, the volume of the tumor was measured twice a week.

Ratio of V to V0 (V/V0) was calculated for each mouse in which V0 is tumor volume of day 0 and V is tumor volume on the measured day. Mean value of V/V0 was calculated for each group. With regard to the difference of V/V0 recognized between the groups, its statistic significant difference was calculated using a statistic analysis software SAS. When P value was 0.05 or less, the difference was judged to be significant. The result is shown in Fig. 6. V/V0 of the group treated with the anti-CD20 antibody and that of the control group increased nearly in the same rate. The result shows that no therapeutic effect by administration of the anti-CD20 antibody was recognized or, in other words, the Namalwa cells became resistant to rituximab. On the other hand, V/V0 of the group treated with the anti-CD10 antibody was significantly small as compared with that of the group treated with rituximab and that of the control group. The result shows that administration of the anti-CD 10 antibody was effective to the tumor which was resistant to the anti-CD20 antibody.

Example 5

*In vivo* effect of anti-CD20 antibody on expression level of CD10

An anti-CD20 antibody rituximab was administered to mice bearing non-Hodgkin's lymphoma cell line Ramos cells (ATCC CRL-1596) were transplanted and expression level of CD 10 of tumor xenograft of Ramos cells was measured by a flow cytometric method.

Ramos cells were transplanted to the right frank of the male SCID mice in an amount of $1 \times 10^7$ cells per area. Three days before the transplantation, an anti-Asialo GM1 antibody (Wako Pure Chemical) was intraperitoneally administered in an amount of 20 μL per mouse. When tumor volume became within a range of 97.5 to 187.6 $mm^3$ (mean value ± standard deviation: 128.6 ± 29.0 $mm^3$), the mice was divided into two groups each comprising five mice so that the tumor volumes were not unbalanced between the groups. The day when the grouping was carried out was defined as day 0. To the group treated with the anti-CD20 antibody, 100 μg/mouse of anti-CD20 antibody rituximab (manufactured by Chugai Pharmaceutical) was administered into tail vein twice a week for eight times in total. To the control group, 200 μL/mouse of a physiological saline solution was administered into tail vein twice a week for eight times in total. On day 29, the mice were sacrificed and tumor lumps were excised.

The excised tumor xenograft was finely cut into small pieces each being about 1 mm square by scissors on ice and in a small amount of DMEM. About 2 mL of minced tumor was placed in a 15-mL round bottom tube, nearly the same amount of a collagenase/DNase solution [a solution which was prepared in such a manner that 641 mg of Collagenase Type 1A (manufactured by Wako Pure Chemical) and 81 mg of DNase (manufactured by Sigma) were weighed, made into 200 mL using an RPMI-1640 and aseptically filtered through a 0.2-μm filter] was added thereto and the mixture was incubated for 30 minutes at 37°C with shaking. To the tumor xenograft after the incubation was added 10 mL of ice-cooled RPMI-1640, followed by stirring for suspension. The suspension of the tumor xenograft was passed through a mesh of 70 μm pore size whereby the filtrate was obtained as a cell suspension. The cell suspension was washed twice with 15 mL of ice-cooled RPMI-1640. The cells were suspended in PBS and cell density was counted.

The cell suspension was placed into 1.5-mL tubes in which each tube contained $5 \times 10^5$ cells and 0.75 mL of PBS containing 1% of bovine serum albumin and 0.05 w/v% sodium azide, was added to each tube. After it was centrifuged at 5,000 rpm for 1 minute, the supernatant liquid was discarded. The cells were suspended in 40 μL of PBS containing 0.1 mg of FITC-labeled anti-CD10 human chimeric antibody Ms705/CD10 (FERM BP-8478), 0.4 w/v% bovine serum albumin, 0.01 w/v% sodium azide and 50 mg/mL of human γ-globulin. As an antibody for control staining, human IgG conjugated with a fluorescent pigment (FITC) was used. After incubation in a refrigerator for 30 minutes, the cells were centrifuged using 0.75 mL of PBS containing 1% of bovine serum albumin and 0.05 w/v% sodium azide for washing. The cells were suspended in an isoflow containing 10 μg/mL of propidium iodide. Expression level of CD10 was analyzed for 5,000 cells which were not stained with propidium iodide using a flow cytometer in which the mean fluorescence intensity derived from FITC was used as an index. With regard to the difference in the mean fluorescence intensity recognized between the groups, its statistic significant difference was calculated using SAS which is the statistic analysis software. When P value was 0.05 or less, the difference was judged to be significant.

The expression level of CD 10 of Ramos cell line excised from mice to which rituximab was administered was significantly higher than that of the control group. From the result, it is now apparent that the treatment of anti-CD20 antibody increases the expression level of CD10.

Reference Example 1

Cloning of variable region gene of mouse anti-CD10 antibody NL-1

1. Isolation and analysis of cDNA encoding V region of anti-CD10 antibody NL-1

(a) Preparation of mRNA from anti-CD10 antibody NL-1 producing hybridoma

mRNA derived from NL-1 was prepared from $5 \times 10^7$ anti-CD10 antibody NL-1 producing hybridoma cell NL-1 [Proc. Natl. Acad. Sci., USA, 79, 4386 (1982)] using Fast Track mRNA Isolation Kit (manufactured by Invitrogen) (a preparation kit for mRNA) according to the instructions attached thereto.

(b) Preparation of H chain and L chain cDNA libraries of anti-CD 10 antibody NL-1

cDNA having *Eco*RI-*Not*I adaptor sequences at both ends was synthesized using Time Saver cDNA Synthesis Kit (manufactured by Amersham-Pharmacia) according to the instructions attached thereto from 5 μg of mRNA of NL-1 prepared in the above (a). All of the cDNAs synthesized were dissolved in 20 μL of sterilized water, subjected to agarose gel electrophoresis and cDNA fragment of about 1.5 kb corresponding to H chain of the IgG class antibody and cDNA fragment of about 1 kb corresponding to L chain of the kapper class antibody were extracted using QIAquick Gel Extraction Kit (manufactured by Qiagen).

Then, 0.1 μg of each cDNA fragment and 1 μg of λ ZIPII vector attached to the kit were ligated using λ ZAPII Predigested *Eco*RI/CIAP-Treated Vector Kit (manufactured by Stratagene) according to the instructions attached thereto. Each 2.5 μL of the reaction solutions was subjected to packaging to λ phase using Gigapack III Gold Packaging Extracts (manufactured by Stratagene) according to the instructions attached thereto whereupon $5 \times 10^4$ and $4 \times 10^4$ phage clones as NL-1 H chain cDNA library and L chain cDNA library, respectively. Then, each phage was fixed on a Nylon membrane filter Hybond $H^+$ (manufactured by Amersham Pharmacia) according to a conventional method [Molecular Cloning: A Laboratory Manual, Cold Spring Harbor Lab. Press, New York (1989)].

(c) Cloning of cDNA of H chain and L chain of anti-CD10 antibody NL-1

The Nylon membrane filter of NL-1 H chain cDNA library and L chain cDNA library prepared in the above item (b) was subjected to ECL Director Nucleic Acid Labeling and Detection Systems (manufactured by Amersham Pharmacia) according to the instructions attached thereto in which cDNA of C region of mouse antibody was used as a probe. As to H chain and L chain, fragments of mouse Cγ2a cDNA [Nature, 283, 786 (1980)] and fragments of mouse Cκ cDNA [Cell, 22, 197 (1980)] were used, respectively. Each 10 clones of H chain and L chain were obtained as phage clones strongly bound to probe. Then each phage clone was converted to plasmid by an *in vivo* excision method according to the instructions for use of λ ZAPII Predigested *Eco*RI/CIAP-Treated Vector Kit (manufactured by Stratagene). Nucleotide sequence of cDNA contained in each of the plasmids prepared as such was allowed to react using BigDye Terminator Cycle Sequencing FS Ready Reaction Kit (manufactured by Applied Biosystems) according to the instructions attached thereto and the nucleotide sequence was analyzed by an autoanalyzer for nucleotide sequences ABI PRISM 377 of the same company whereupon it was confirmed that plasmid containing full-length of functional H chain cDNA in which ATG sequence deduced to be initiation codon is present at 5' terminal of cDNA and plasmid containing L chain DNA were prepared.

(d) Analysis of amino acid sequence of V region of anti-CD10 antibody NL-1

The total nucleotide sequence of VH of NL-1 contained in the plasmid pNL-1H containing H chain cDNA is represented by SEQ ID NO:29, the total amino acid sequence of VH of NL-1 presumed from the sequence is represented by SEQ ID NO:30, the total nucleotide sequence of VL of NL-1 contained in the plasmid pNL-1L is represented by SEQ ID NO:31 and the total amino acid sequence of VL of NL-1 deduced from the sequence is represented by SEQ ID NO:32.

The VH and VL amino acid sequences of NL-1 represented by SEQ ID NOs:30 and 32 were compared with the sequence data of known antibodies [Sequences of Proteins of Immunological Interest, U. S. Dept. Health an Human Services (1991)] and, furthermore, N-terminal amino acid sequences of VH and VL of the purified anti-CD10 antibody NL-1 were compared with the result analyzed by Protein Sequencer PPSQ-10 (manufactured by Shimadzu). As a result, each isolated cDNA was a full-length cDNA encoding H chain and L chain of anti-CD10 antibody NL-1 containing a secretion signal sequence.

2. Stable expression in FUT8 gene double-knockout cells

Cells stably producing anti-CD10 chimeric antibody were prepared by a method mentioned in WO 03/85107 using FUT8 gene double-knockout cells mentioned in WO 03/85107 and CHO/DG44 cells which are parent cell lines thereof as host cells. Incidentally, the anti-CD10 chimeric antibody expression vector was prepared from cDNA encoding VH and VL of NL-1 prepared in item 1 of this Reference Example (SEQ ID NOs:29 and 31) by a method mentioned in Example 1 of WO 03/018635. In Fig. 3, an outlined drawing of the prepared anti-CD10 chimeric antibody expression vector is shown.

As a result, a transformant which is capable of growing in an IMDM-dFBS (10) medium containing G418 in a final concentration of 500 μg/mL and which produces an anti-CD 10 chimeric antibody is obtained. The transformant prepared from CHO/DG44 cells which are parent cell line was designated DG44/CD10 while the transformant strain prepared from the FUT8 gene double-knockout cells was designated Ms705/CD10.

3. Measurement of human IgG antibody concentration in the culture supernatant (ELISA)

Goat anti-human IgG (H&L) antibody (manufactured by American Qualex) was diluted with a phosphate buffered saline (hereinafter referred to as PBS) (manufactured by Invitrogen) to make 1 μg/mL, dispensed to a 96-well plate for ELISA (manufactured by Gleiner) in 50 μL/well and adsorbed by being allowed to stand at 4°C for one night. After washing with PBS, 100 μL/well of PBS containing 1% of BSA (hereinafter referred to as 1% BSA-PBS) (manufactured by Wako Pure Chemical) was added thereto and reaction was carried out at room temperature for 1 hour to block the remaining active groups. The 1% BSA-PBS was discarded and the supernatant liquid of the culture of the transformant or variously diluted solution of the antibody purified from the culture supernatant was added in an amount of 50 μL/well and was allowed to react at room temperature for 1 hour. After the reaction, each well was washed with PBS containing 0.05% of Tween 20 (hereinafter referred to as Tween-PBS) (manufactured by Wako Pure Chemical), then a peroxidase-labeled goat anti-human IgG (H&L) antibody solution (manufactured by American Qualex) diluted 2,000-fold with 1% BSA-PBS was added as a secondary antibody solution in an amount of 50 μL/well and reaction was carried out at room temperature for 1 hour. After the reaction, it was washed with Tween-PBS and colored by addition of 50 μL/well of an ABTS substrate solution [a solution prepared in such a manner that 0.55 g of ammonium 2,2'-azino-bis(3-ethylbenzothiazoline-6-sulfonate) (manufactured by Wako Pure Chemical) was dissolved in 1L of 0.1M citrate buffer (pH 4.2) and, immediately before use, 1 μL/mL of hydrogen peroxide (manufactured by Wako Pure Chemical) was added thereto] and absorbance at 415 nm (hereinafter referred to as OD415) was measured.

4. Purification of anti-CD 10 human chimeric antibody

The transformant cells DG44/CD10 and Ms705/CD10 prepared in item 2 of this Reference Example were used and anti-CD10 human chimeric antibodies produced by each of them were purified as follows.

Each transformant was suspended in an IMDM-dFBS (10) containing 500 μg/mL of G418 and 30 mL of the suspension was seeded in a 182-$cm^2$ flask (manufactured by Gliner) and cultured for several days at 37°C in a 5% $CO_2$ incubator. When the cell density became confluent, the culture supernatant was removed, the cells were washed with 25 mL of PBS and Excell 301 medium (manufactured by JRH Biosciences) was poured therein. After culturing in a 5% $CO_2$ incubator at 37°C for seven days, the cell suspension was recovered and the supernatant was recovered by centrifugal separation for 5 minutes under conditions of 3,000 rpm and 4°C and filtered and sterilized using Millex GV filter of 0.22 μm pore size (manufactured by Millipore). From the culture supernatant prepared by the above method, an anti-CD 10 human chimeric antibody was purified using Mab Select (manufactured by Amersham Biosciences) column according to the instructions attached thereto. With regard to the purified anti-CD10 human chimeric antibody, the antibody prepared from DG44/CD10 was designated DG44/CD10 antibody while that prepared from Ms705/CD10 was designated Ms705/CD10 antibody. Incidentally, the transformant Ms705/CD10 which produces the human chimeric antibody, Ms705/CD10 was deposited as FERM BP-8478 on September 11, 2003 with the International Patent Organism Depositary (Tsukuba Central 6, 1-1 Higashi-1-chome, Tsukuba-shi, Ibaraki-ken, Japan), the National Institute of Advanced Industrial Science and Technology.

Reference Example 2

Preparation of humanized antibody which specifically binds to CD10

1. Design of cDNA encoding VH and VL of humanized antibody which specifically binds to CD10

(1) Design of amino acid sequence of VH of humanized antibody which specifically binds to CD10

Firstly, an amino acid sequence of VH of humanized antibody which specifically binds to CD10 (hereinafter referred to as anti-CD10 CDR-grafted antibody) was designed as follows.

Amino acid sequence of FR of VH of human antibody for graft of amino acid sequences of CDR1, 2 and 3 of VH represented by SEQ ID NOs:2, 3 and 4 was selected. Human antibody having a high homology with NL-1 was searched in the known amino acid sequence database of protein using BLASTP method [Nucleic Acid Res., 25, 3389 (1997)]. When the homology score and the actual amino acid sequence homology were compared, Ig Heavy Chain mentioned in SWISSPROT database accession No. AAA69734 [Proc. Natl. Acad. Sci. USA, 1691, 6146 (1990)] showed 87.4% and was a human antibody having the highest homology and, therefore, the amino acid sequence of FR of VH of this antibody was selected. However, the amino acid which was the first one at N-terminal of the clone AAA69734 on the database was glutamine Gln which was low in an appearing frequency in the sequence of human antibody. Therefore, with regard to the first amino acid, it was substituted with glutamic acid Glu which is found in high frequency even in any human antibody sequence [*Sequence of Proteins of Immunological Interest,* U. S. Dept. Health and Human Services (1991)].

The amino acid sequences of CDR1, 2 and 3 ofVH of the anti-CD10 mouse antibody NL-1 represented by SEQ ID NOs:2, 3 and 4 were grafted into the appropriate positions of the amino acid sequence of FR of the human antibody determined as above whereupon the amino acid sequence HV0 of VH of anti-CD10 CDR-grafted antibody represented by SEQ ID NO:8 was designed. The nucleotide sequence of cDNA encoding the amino acid sequence is represented by SEQ ID NO:9.

(2) Design of amino acid of VL of anti-CD10 humanized antibody

Then the amino acid sequence of VL of the anti-CD10 humanized antibody was designed as follows. Amino acid sequences of FR of VL of the human antibody for grafting of the amino acid sequences of CDR1, 2 and 3 of VL of the anti-CD10 mouse antibody NL-1 represented by SEQ ID NOs:5, 6 and 7 were selected. Kabat, *et al*. classified the VL of various known human antibody into four subgroups (HSG I to IV) depending upon the homology of amino acid sequences thereof and further reported of the common sequences for each subgroup [Sequences of Proteins of Immunological Interest, U. S. Dept. Health and Human Services (1991)]. Now, amino acid sequence of FR having the highest homology with the amino acid sequence of FR of VL of NT -1 among the amino acid sequences of FR of the common sequences of the four subgroups of VL of the human antibody was selected.

Table 1 shows the result of search of homology between the amino acid sequence of FR of the common sequence of each subgroup of VL of the human antibody and the amino acid sequence of FR of VL of NL-1. As shown in Table 1, the amino acid sequence of FR of VL of NL-1 has the highest homology with the subgroup IV.

Table 1

Homology between amino acid sequence of FR of common sequence of subgroups of VL of human antibody and amino acid sequence of FR of VL of anti-CD10 mouse antibody NL-1

| HSG I | HSG II | HSG III | HSG IV |
|---|---|---|---|
| 66.25% | 61.25% | 65.00% | 67.50% |

In view of the above result, the amino acid sequences of CDR1, 2 and 3 of VL of the anti-CD10 mouse antibody NL-1 represented by SEQ ID NOs:5, 6 and 7 were grafted to appropriate positions of the amino acid sequence of FR of the common sequence of the subgroup IV of VL of the human antibody whereby the amino acid sequence LV0 of VL of the anti-CD10 humanized antibody represented by SEQ ID NO:10 was designed. The nucleotide sequence of cDNA encoding the amino acid sequence is represented by SEQ ID NO:11.

(3) Modification of VL of anti-CD10 humanized antibody

The amino acid sequence HV0 of VH and the amino acid sequence LV0 of VL of the above-designed anti-CD10 humanized antibody were sequences in which only amino acid sequence of CDR of the anti-CD10 mouse antibody NL-1 was grafted into the amino acid sequence of FR of the selected human antibody. However, usually, in the humanized antibodies in which only grafting of the amino acid sequence of CDR of the mouse antibody is carried out, there are many cases that a binding activity to the antigen lowers. Therefore, in order to avoid the decrease of binding activity to the antigen, studies have been made to identify the amino acid residue of FR which was likely to affect the binding activity to the antigen for such an object that, among the amino acid residues of FR which were different between human antibody and mouse antibody, the amino acid residue which was likely to affect the binding activity to the antigen was transplanted together with the amino acid sequence of CDR.

The following modification of VL was carried out, because the homology of amino acid sequence of VL between anti-CD10 mouse antibody NL-1 and human antibody selected as the one to which CDR of the mouse antibody is to be grafted, is low.

Firstly, a three-dimensional structure of the antibody V region HV0LV0 comprising the amino acid sequence HV0 of VH of the above-designed anti-CD10 humanized antibody and the amino acid sequence LV0 of VL was constructed by means of a computer modeling. With regard to the preparation of the three-dimensional structure coordinate, the software AbM (manufactured by Oxford Molecular) was used and, with regard to the display of the three-dimensional structure, the software RasMol (manufactured by Glaxo) was used in accordance with the instructions for use attached to each of them. Computer model of the three-dimensional structure of V region of the anti-CD 10 mouse antibody NL-1 was similarly constructed as well.

As a result, with regard to the residues which are likely to affect the binding activity of the antibody to the antigen by changing the three-dimensional structure of antigen-binding site in the amino acid residues of FR of HV0LV0, there were selected Asn at position 22, Pro at position 44, Leu at position 47, Leu at position 48, Asp at position 61, Asp at position 71, Phe at position 72, Thr at position 73 and Val at position 86 of LV0. Amino acid sequence LV9 of VL of the humanized antibody represented by SEQ ID NO:12 in which all of those selected amino acid residues were modified to the amino acid residues shown in the mouse antibody NL-1 was designated. The nucleotide sequence of cDNA encoding the amino acid sequence is represented by SEQ ID NO:13.

(4) Modification of VH of anti-CD10 humanized antibody

Then the amino acid residue of FR which is likely to affect the binding activity to the antigen in VH was identified.

Firstly, the three-dimensional structure was displayed using the method mentioned in the above (3) and the computer model of the three-dimensional structure of the V region of the anti-CD10 mouse antibody NL-1 was also constructed.

As a result, with regard to the residue which changes the three-dimensional structure of the antigen-binding site in the amino acid residues of FR of HV0LV0 and is likely to affect the binding activity of the antibody to the antigen, there were selected Glu at position 1, Arg at position 16, Leu at position 18, Gly at position 42, Tyr at position 80, Asn at position 84 and Arg at position 98 of HV0. The amino acid sequence HV2 of VH of the humanized antibody represented by SEQ ID NO:37 in which, among the above selected amino acid residues, Gly at position 42 and Arg at position 98 were modified to the amino acid residue recognized in the mouse antibody NL-1. The nucleotide sequence of cDNA encoding the amino acid sequence is represented by SEQ ID NO:38.

2. Construction of cDNA encoding VH of anti-CD10 humanized antibody

The cDNA encoding the amino acid sequence HV0 of VH of the anti-CD10 humanized antibody designed in (1), item 1 of this Reference Example was constructed as follows using PCR. The construction step is shown in Fig. 7. In the following descriptions, with regard to a synthetic oligonucleotide and a primer for PCR, the synthetic oligonucleotide and the primer for PCR, respectively, manufactured by Fasmac were used.

Firstly, the designed amino acid sequence was transformed into a gene codon. When a some of gene codons were present for one amino acid residue, the corresponding gene codon was determined by taking the frequency of use recognized in nucleotide sequences of gene of the antibody [Sequence of Proteins of Immunological Interest, U. S. Dept. Health and Human Services (1991)] into consideration.

The nucleotide sequence encoding a secretion signal sequence and a 5' untranslated region of H chain represented by SEQ ID NO:43 was connected to the 5' terminal side of the transformed gene codon whereupon the nucleotide sequence of cDNA encoding the amino acid sequence of the complete antibody V region was designed and, further, recognition sequences for the restriction enzyme for cloning to the vector for expression of the humanized antibody were added to 5' terminal and 3' terminal. The designed nucleotide sequence was divided into four nucleotide sequences in total each comprising 120 to 150 bases from the 5' terminal side (the adjacent nucleotide sequences have about 20 bases of overlapped sequences at their terminals) and then they were made into the order of sense chain and antisense chain alternately to synthesize four synthetic oligonucleotides having the nucleotide sequences represented by SEQ ID NOs:14, 15, 16 and 17, respectively.

Each of the synthetic oligonucleotides was added to a reaction solution containing 0.2 mM of dNTPs and 1 mM of magnesium chloride so as to give a final concentration of 0.02 μM, then the total volume was made 50 μL using 1 μM of HVO-FW primer (SEQ ID NO:18), 1 μM of HV0-RV primer (SEQ ID NO:19) and 2.5 units of KOD polymerase (manufactured by Toyobo) and PCR was carried out. The reaction was carried out by 30 cycles, one cycle consisting of reaction at 94˚C, reaction at 55˚C for 30 seconds and reaction at 74˚C for 30 seconds, followed by one cycle of reaction at 74˚C for 10 minutes. After the PCR, the reaction solution was subjected to 2% agarose gel electrophoresis and about 0.47 kbp of the PCR product was recovered using QIAEXII Gel Extraction Kit (manufactured by Qiagen). The recovered PCR product was inserted into a plasmid pCR-Blunt using Zero Blunt PCR Cloning Kit (manufactured by Invitrogen) according to the instructions attached thereto. *Escherichia coli* DH5α strain (manufactured by Toyobo) was transformed using the resulting recombinant plasmid DNA solution, each plasmid was prepared from the clone of the transformant, the nucleotide sequence of PCR fragment inserted into the plasmid was allowed to react using BigDye Terminator Cycle Sequencing FS Ready Reaction Kit (manufactured by Applied Biosystems) according to the instructions attached thereto and then the nucleotide sequence was analyzed by an automated analyzer for nucleotide sequence ABI PRISM 377 of the same company whereupon it was confirmed that a plasmid pCRHV0 having the desired nucleotide sequence was obtained.

Then, cDNA encoding the amino acid sequence HV2 of the anti-CD10 humanized antibody designed in (4), item 2 of this Reference Example was constructed in the same manner as mentioned above. Process of the construction was shown in Fig. 8. With regard to the synthetic oligonucleotide however, the four synthetic oligonucleotides having the nucleotide sequences represented by SEQ ID NOs:14, 39, 40 an 41 were used. Finally, the nucleotide sequence was analyzed and it was confirmed that a plasmid pCRHV2 having the desired nucleotide sequence was obtained.

3. Construction of cDNA encoding VL of anti-CD10 humanized antibody

cDNA encoding the amino acid sequence LV0 of VL of the anti-CD10 humanized antibody designed in (2), item 1 of this Reference Example was constructed as follows. Process of the construction was shown in Fig. 9. With regard to the 5' untranslated region and the secretion signal sequence, the nucleotide sequence represented by SEQ ID NO:42 was used.

Firstly, four synthetic oligonucleotides having the nucleotide sequences represented by SEQ ID NOs:20, 21, 22 and 23 were designed in the same manner as in the above item 2 and then synthesized. Each of the synthetic oligonucleotides was added to a reaction solution containing 0.2 mM dNTPs and 1 mM magnesium chloride so as to give a concentration of 0.02 μM, then made 50 μL in total using 1 μM LVO-FW primer (SEQ ID NO:24), 1 μM LV0-RV primer (SEQ ID NO:25) and 2.5 units of KOD polymerase (manufactured by Toyobo) and PCR was carried out. The reaction was carried out by 30 cycles, one cycle consisting of reaction at 94˚C for 30 seconds, reaction at 55˚C for 30 seconds and reaction at 74˚C for 30 seconds, followed by one cycle of reaction at 74˚C for 10 minutes. The reaction solution was subjected to agarose gel electrophoresis and a PCR product of about 0.44 kbp was recovered using QIAEXII Gel Extraction Kit (manufactured by Qiagen). The recovered PCR product was inserted into a plasmid pCR-Blunt using Zero Blunt PCR Cloning Kit (manufactured by Invitrogen) according to the instructions attached thereto. *Escherichia coli* DH5α (manufactured by Toyobo) was transformed using the resulting recombinant plasmid DNA solution, each plasmid was prepared from the clone of the transformant, nucleotide sequence of the PCR fragment inserted into the plasmid was allowed to react using BigDye Terminator Cycle Sequencing FS Ready Reaction Kit (manufactured by Applied Biosystems) according to the instructions attached thereto and then the nucleotide sequence was analyzed using an automatic analyzer for nucleotide sequence ABI PRISM 377 of the same company whereby it was confirmed that the a plasmid pCRLV0 having the desired nucleotide sequence was obtained.

Then cDNA encoding LV9 designed in (3), item 1 of this Reference Example was constructed in the same manner as above. Process of the construction was shown in Fig. 10. However, with regard to the synthetic oligonucleotides, four synthetic oligonucleotides having the nucleotide sequences represented by SEQ ID NOs:20, 26, 27 and 28 were used and an LVO-FW primer (SEQ ID NO:24) and 1 μM LV0-RV primer (SEQ ID NO:25) were used. Finally, the nucleotide sequence was analyzed and it was confirmed that a plasmid pCRLV9 having the desired nucleotide sequence could be obtained.

4. Construction of anti-CD10 humanized antibody expression vector

An anti-CD10 humanized antibody expression vector pKANTEX 3061 chimeric HLVO and pKANTEX 3061 HV0LV0 were constructed as follows using the anti-CD10 chimeric antibody expression vector mentioned in item 2 of Reference Example 1, the plasmid pCRHV0 prepared in the above item 2 and the plasmid pCRLV0 prepared in the above item 3. The construction step are respectively shown in Fig. 11 and Fig. 12.

The plasmid pCRLV0 was digested with a restriction enzyme *Bsi*WI (manufactured by New England Biolabs) and a restriction enzyme *Eco*RI (manufactured by Takara Shuzo) and subjected to agarose gel electrophoresis to recover *Bsi*WI-*Eco*RI fragments of about 0.44 kb.

On the other hand, the anti-CD10 chimeric antibody expression vector was digested with a restriction enzyme *Bsi*WI (manufactured by New England Biolabs) and a restriction enzyme *Eco*RI (manufactured by Takara Shuzo) and subjected to agarose gel electrophoresis to recover *Bsi*WI-*Eco*RI fragments of about 13.2 kb.

The recovered *Bsi*WI-*Eco*RI fragments derived from the plasmid pCRLV0 and *Bsi*WI-*Eco*RI fragments derived from the anti-CD10 chimeric antibody were ligated using Ligation High (manufactured by Toyobo) according to the instructions attached thereto. *Escherichia coli* DH5α (manufactured by Toyobo) was transformed using the resulting recombinant plasmid DNA solution, each plasmid was prepared using clone of the transformant, the nucleotide sequence of the plasmid was allowed to react using BigDye Terminator Cycle Sequencing FS Ready Reaction Kit (manufactured by Applied Biosystems) according to the instructions attached thereto and then the nucleotide sequence was analyzed by an automatic analyzer for nucleotide sequence ABI PRISM 377 of the same company whereupon it was confirmed that an anti-CD10 humanized antibody expression vector pKANTEX 3061 chimeric HLVO having the desired nucleotide sequence was obtained.

Then, the plasmid pCRHV0 was digested with a restriction enzyme *Apa*I (manufactured by Takara Shuzo) and a restriction enzyme *Not*I (manufactured by Takara Shuzo) and then subjected to agarose gel electrophoresis to recover *Apa*I-*Not*I fragments of about 0.47 kb.

On the other hand, the above-prepared plasmid pKANTEX 3061 chimeric HLVO was digested with a restriction enzyme *Apa*I (manufactured by Takara Shuzo) and a restriction enzyme *Not*I (manufactured by Takara Shuzo) and then subjected to agarose gel electrophoresis to recover *Apa*I-*Not*I fragments of about 13.2 kb.

The recovered *Apa*I-*Not*I fragments derived from the plasmid pCRHV0 and *Apa*I-*Not*I fragments derived from the plasmid pKANTEX 3061 chimeric HLVO were ligated using Ligation High (manufactured by Toyobo) according to the instructions attached thereto. *Escherichia coli* DH5α (manufactured by Toyobo) was transformed using the resulting recombinant plasmid DNA solution, each plasmid was prepared using clone of the transformant, the nucleotide sequence of the plasmid was allowed to react using BigDye Terminator Cycle Sequencing FS Ready Reaction Kit (manufactured by Applied Biosystems) according to the instructions attached thereto and then the nucleotide sequence was analyzed by an automatic analyzer for nucleotide sequence ABI PRISM 377 of the same company whereupon it was confirmed that an anti-CD10 humanized antibody expression vector pKANTEX 3061 chimeric HV0LV0 having the desired nucleotide sequence could be obtained.

Then, an anti-CD10 humanized antibody expression vector pKANTEX 3061 HV0LV9 was constructed using the above-prepared anti-CD10 humanized antibody expression vector pKANTEX 3061 HV0LV0 and plasmid pCRLV9. The construction step is shown in Fig. 13.

The anti-CD10 humanized antibody expression vector pKANTEX 3061 HV0LV0 was digested with a restriction enzyme *Eco*RI (manufactured by Takara Shuzo) and a restriction enzyme *Bsi*WI (manufactured by Takara Shuzo) and subjected to agarose gel electrophoresis to recover *Eco*RI fragments of about 13.2 kb.

On the other hand, the plasmid pCRLV9 was digested with a restriction enzyme *Eco*RI (manufactured by Takara Shuzo) and a restriction enzyme *Bsi*WI (manufactured by Takara Shuzo) and then subjected to agarose gel electrophoresis to recover *Eco*RI-*Bsi*WI fragments of about 0.47 kb.

The recovered *Apa*I-*Not*I fragments derived from the anti-CD10 humanized antibody expression vector pKANTEX 3061 HV0LV0 and *Apa*I-*Not*I fragments derived from the plasmid pCRHV9 were ligated using Ligation High (manufactured by Toyobo) according to the instructions for use attached thereto. *Escherichia coli* DH5α (manufactured by Toyobo) was transformed using the resulting recombinant plasmid DNA solution, each plasmid was prepared using clone of the transformant, the nucleotide sequence of the plasmid was allowed to react using BigDye Terminator Cycle Sequencing FS Ready Reaction Kit (manufactured by Applied Biosystems) according to the instructions attached thereto and then the nucleotide sequence was analyzed by an automatic analyzer for nucleotide sequence ABI PRISM 377 of the same company whereupon it was confirmed that an anti-CD10 humanized antibody expression vector pKANTEX 3061 HV0LV9 having the desired nucleotide sequence could be obtained.

Then, an anti-CD10 humanized antibody expression vector pKANTEX 3061 HV2LV9 was constructed using the above-prepared anti-CD10 humanized antibody expression vector pKANTEX 3061 HV0LV9 and plasmid pCRHV2. The construction step is shown in Fig. 14.

On the other hand, the plasmid pCRHV2 was digested with a restriction enzyme *Not*I (manufactured by Takara Shuzo) and a restriction enzyme *Apa*I (manufactured by Takara Shuzo) to recover *Not*I-*Apa*I fragments of about 0.47 kb.

The recovered *Apa*I-*Not*I fragments derived from the anti-CD10 humanized antibody expression vector pKANTEX 3061 HV0LV9 and *Apa*I-*Not*I fragments derived from the plasmid pCRHV9 were ligated using Ligation High (manufactured by Toyobo) according to the instructions for use attached thereto. *Escherichia coli* DH5α (manufactured by Toyobo) was transformed using the resulting recombinant plasmid DNA solution, each plasmid was prepared using clone of the transformant, the nucleotide sequence of the plasmid was allowed to react using BigDye Terminator Cycle Sequencing FS Ready Reaction Kit (manufactured by Applied Biosystems) according to the instructions attached thereto and then the nucleotide sequence was analyzed by an automatic analyzer for nucleotide sequence ABI PRISM 377 of the same company whereupon it was confirmed that an anti-CD10 humanized antibody expression vector pKANTEX 3061 chimeric HV2LV9 having the desired nucleotide sequence could be obtained.

5. Stable expression of anti-CD10 humanized antibody using animal cells and purification of the antibody

The three kinds of anti-CD10 humanized antibody expression vectors pKANTEX 3061 HV0LV0, pKANTEX 3061 HV0LV9 and pKANTEX 3061 HV2LV9 prepared in item 4 of this Reference Example were to prepare each stable expression cell line of the anti-CD10 humanized antibody using FUT8 gene double -knockout CHO cells as host cells. FUT8 gene double-knockout CHO cells was produced in accordance with a method mentioned in WO 03/85107.

With regard to the transformants prepared using the FUT8 gene double-knockout CHO cells as a host hereinafter, the transformant prepared by introduction of the anti-CD 10 humanized antibody expression vector pKANTEX 3061 HV0LV0 will be designated an HV0LV0 MS705 clone while the transformant prepared by introduction of the anti-CD10 humanized antibody expression vector pKANTEX 3061 HV0LV9 is designated HV0LV9 MS705 clone. The transformant prepared by introduction of the anti-CD 10 humanized antibody expression vector pKANTEX 3061 HV2LV9 is designated HV2LV9 MS705 clone.

Each of the above transformants was suspended in an Iscove's Modified Dulbecco's Medium (manufactured by Invitrogen) to which a basic medium containing 500 μg/mL of G418 (10% bovine embryo dialyzed serum [manufactured by Invitrogen]), 50 μg/mL of gentamicin (manufactured by Nacalai Tesque) and 1 × HT supplement (manufactured by Invitrogen) were added and 30 mL thereof was seeded in a 182-$cm^2$ flask (manufactured by Gliner) and cultured for several days at 37°C in a 5% $CO_2$ incubator. When cell density became confluent, the culture supernatant was removed, the cells were washed with 25 mL of PBS and an Excell 301 medium (manufactured by JRH Biosciences) was infused therein. After culturing in a 5% $CO_2$ incubator at 37°C for seven days, the cell suspension was recovered, the culture supernatant was recovered by centrifugal separation for 5 minutes under conditions of 3,000 rpm and 4°C and filtered and sterilized using Millex GB filter of 0.22 μm pore size (manufactured by Millipore). From the culture supernatant prepared by the above method, each anti-CD10 humanized antibody was purified using Mab Select (manufactured by Amersham Biosciences) according to the instructions attached thereto. With regard to the purified anti-CD10 human chimeric antibody, the antibody prepared from DG44/CD10 was designated DG44/CD10 antibody while that prepared from Ms705/CD10 was designated Ms/705/CD10 antibody. Hereinafter, the anti-CD10 humanized antibody prepared from HV0LV0 MS705 is designated HV0LV0 MS705 antibody, the anti-CD10 humanized antibody prepared from HV0LV0 MS705 is designated HV0LV9 MS705 antibody and the anti-CD10 humanized antibody prepared from HV2LV9 MS705 is designated HV2LV9 MS705 antibody. The transformant HV0LV9 MS705 which produces the anti-CD 10 humanized antibody was deposited to International Patent Organism Depositary, National Institute of Advanced Industrial Science and Technology (Tsukuba Central 6, 1-1, Higashi 1-chome, Tsukuba-shi, Ibaraki, Japan) as FERM BP-10099 on August 17, 2004.

Reference Example 3

Measurement of biological activity of anti-CD10 humanized antibody

1. Measurement of binding activity of the anti-CD10 humanized antibody to CD10 (ELISA)

Each of the anti-CD10 humanized antibodies prepared in Reference Example 2 was measured by the following method.

As to an antigen, a membrane fraction prepared from human lung small-cell tumor cell line SBC-3 cells (JCRB 0818) which highly express CD10 was used. The membrane fraction was suspended in 20 mM HEPES (pH 7.4) buffer containing 1 mM EDTA and 250 mM sucrose and cells were crushed using a homogenizer, then centrifugal separation was carried out at a low speed of 8,000 $\times$ g so that granules in the cells and uncrushed cells were precipitated and removed and a centrifugal separation was carried out at a high speed of 80,000 $\times$ g to separate the desired cell membrane. The separated membrane fraction was dissolved in PBS containing 0.1% of Triton-X 100 whereby protein was quantified, then made 10 $\mu$g/mL, dispensed at 50 $\mu$L/well into a 96-well plate for ELISA (manufactured by Gliner) and allowed to stand at 4˚C for one night for adsorption. After washing with PBS, 1% BSA-PBS was added in an amount of 100 $\mu$L/well and the reaction was carried out at room temperature for 1 hour to block the remaining active groups were blocked. The 1% BSA-PBS was discarded, each well was washed with Tween-PBS, each of the diluted solutions of the DG44/CD10 antibody or the Ms705/CD10 antibody prepared in item 3 of Reference Example 2 was added in an amount of 50 $\mu$L/well and reaction was carried out at room temperature for 2 hours. After the reaction, each well was washed with Tween-PBS, a peroxidase-labeled goat anti-human IgG (H&L) antibody solution diluted 2,000-fold with 1% BSA-PBS was added as a secondary antibody solution in an amount of 50 $\mu$L/well and reaction was carried out at room temperature for 1 hour. After the reaction, washing was carried out with Tween-PBS, an ABTS substrate solution was added in an amount of 50 $\mu$L/well to develop color and OD415 was measured.

As a result, in any of the anti-CD 10 humanized antibodies, a binding activity to antigen was recognized dependent on the concentration of the antibody.

2. ADCC activity of anti-CD 10 humanized antibody

ADCC activity of the purified anti-CD10 humanized antibody prepared in Reference Example 2 was measured by the method mentioned below. Incidentally, Raji cells (JCRB 9012) and Daudi cells (JCRB 9071) were used as target cells and Polymorphprep (manufactured by Nycomed) was used for the preparation of an effector cell solution.

(1) Preparation of target cell solution

Raji cells (JCRB 9012) and Daudi cells (JCRB 9071) cultured by an RPMI 1640-FCS (10) medium (an RPMI 1640 medium containing 10% of FCS (manufactured by Gibco BRL) were washed with a medium for the measurement of ADCC activity [an RPMI 1640 medium containing 10% of dialyzed serum (manufactured by Gibco BRL)] by means of centrifugal operation and suspending and then made into 2 $\times$ $10^5$ cells/mL using a medium for the measurement of ADCC activity to give a target cell solution.

(2) Preparation of effector cell solution

Venous blood (50 mL) of a healthy person was collected and 0.5 mL of heparin sodium (manufactured by Shimizu Seiyaku) was added thereto followed by gentle stirring. A monocyte layer was separated therefrom using Polymorphprep (manufactured by Nycomed) according to the instructions attached thereto. It was washed with a medium for the measurement of ADCC activity by centrifugal separation three times and suspended in the same medium to give a concentration of 7 $\times$ $10^6$ cells/mL whereupon an effector solution was prepared.

(3) Measurement of ADCC activity

The target cell solution (50 μL) prepared in the above (1) was dispensed to each well of a 96-well U-shaped bottom plate (manufactured by Falcon) in an amount of $1 \times 10^4$ cells/well. Then, 50 μL ($2.5 \times 10^5$ cells/well; the ratio of the effector cells to the target cells was 35:1) of the effector cell solution prepared in (2) was added thereto. Furthermore, each anti-CD10 chimeric antibody was diluted with a medium for the measurement of ADCC activity and added thereto so as to give each final concentration of 0.001 to 1 μg/mL whereby the total volume was made 150 μL and reaction was carried out at 37˚C for 4 hours. After the reaction, the plate was centrifuged and a lactic acid dehydrogenase (LDH) activity of the supernatant was measured by obtaining absorbance data using CytoTox 96 Non-Radioactive Cytotoxicity Assay (manufactured by Promega) according to the instructions attached thereto. The absorbance data of spontaneous release of the target cells and those of mixed spontaneous release of the effector cells and the target cells were obtained by conducting the same operation as above by the use of the medium only instead of the effector solution and the antibody solution and by the use of the medium only instead of the antibody solution, respectively. The absorbance data of the target cells total release were obtained by the same operation as above in which a medium was used instead of the antibody solution and the effector cell solution and, 45 minutes before finish of the reaction, 15 μL of 9% Triton X-100 solution was added thereto. The ADCC activity was determined by the following formula.

(formula)

ADCC Activity (%) = {([Absorbance at each sample concentration] - [Absorbance of effector cells and target cell mixed spontaneous release])/([Absorbance of target cell total release] - [Absorbance of target cell spontaneous release])} × 100

Furthermore, a value in which background to which no antibody was added was deducted from the value obtained by the above formula was calculated and defined as an antibody-dependent cell-mediated cytotoxicity (ADCC activity).

The result is shown in Fig. 15. The anti-CD10 humanized antibodies HV0LV9 MS 705 and HV2LV9 MS 705 showed an ADCC activity to target cells.

(4) Measurement of CDC activity

a) Preparation of target cell solution

A Burkitt lymphomas cell line Daudi (JCRB 9071) cultured in an MI 1640-FCS (10) medium [an RPMI medium containing 10% of FCS (manufactured by Gibco BRL)] was washed with a medium for the measurement of CDC activity [an RPMI 1640 medium containing 1.4% of bovine serum albumin (manufactured by Gibco BRL)] by means of centrifugal operation and suspending and then made into $1 \times 10^6$ cells/mL using a medium for the measurement of CDC activity to prepare a target cell solution.

b) Preparation of human complement solution

A freeze-dried product of human serum SERA Complement (manufactured by Sigma) was dissolved in 1 mL of deionized water and diluted 2-fold by addition of the same amount of medium for the measurement of CDC activity to give a human complement solution.

c) Measurement of CDC activity

The target cell solution (50 μL; $5 \times 10^4$ cells/well) prepared in the above a) was dispensed to each well of a 96-well flat bottom plate (manufactured by Sumilon). Then, each of various antigen solutions was diluted with a medium for the measurement of CDC activity and added thereto so as to give a final concentration of 0.001 to 10 μg/mL. Furthermore, 50 μL of the complement solution prepared in b) was added to make the total volume 150 μL and reaction was carried out at 37˚C for 2 hours. Also were prepared a well (Blank) to which each 50 μL of the complement solution and 100 μL of the medium for the measurement of CDC activity were added and a well (Total) to which each 50 μL of the target cell solution, the complement solution and the medium for the measurement of CDC activity were added. After the reaction, 15 μL of a cell growth reagent WST-1 (manufactured by Roche) was added to each well, followed by stirring, reaction was carried out at 37˚C for 4 hours and absorbance at 450 nm was measured. CDC activity was calculated by the following formula.

(Formula)

Cytotoxic activity (%) = {1 - ([Absorbance of sample] - [Absorbance of blank])/([Absorbance of total] - [Absorbance of blank])} × 100

Fig. 15 shows activities of the anti-CD10 humanized antibody HV0LV9 MS 705, the anti-CD10 humanized antibody HV2LV9 MS 705 and the anti-CD10 human chimeric antibody Ms705/CD10 to Daudi cells. The anti-CD10 humanized antibody HV0LV9 MS 705 and the anti-CD10 humanized antibody HV2LV9 MS 705 showed the similar CDC activity of the anti-CD10 human chimeric antibody Ms705/CD10.

Reference Example 4

*In vivo* pharmaceutical effect test of the anti-CD10 chimeric antibody and humanized antibody

An *in vivo* pharmaceutical effect test of the anti-CD10 chimeric antibody Ms705/CD10 and humanized antibody HV2LV9 MS705 was carried out in a system of a mouse xenograft life-prolonged model using B cell acute lymphocytic leukemia (B-ALL cells). On the day before the transplantation, body weight of the SCID mice (Nippon Claire; female, seven weeks old) was measured and divided into four groups comprising a control group and groups to which the antibody was administered (1 mg/kg Ms705/CD10; and 0.1 mg/kg and 1 mg/kg of HV2LV9 MS705) (one group comprised seven mice).

CD10 positive B-ALL cells NALM-6 cultured *in vitro* (Aging Laboratory, Tohoku University) were suspended in PBS (Gibco BRL) in $5 \times 10^7$ cells/mL and each 100 μL thereof was transplanted into tail vein of mouse ($5 \times 10^6$ cells/mouse). From the same day, 100 μL of antibody solution diluted with PBS (Gibco BRL) or PBS only was administered to the tail vein of the antibody-administered group or of the control group, respectively. The administration was carried out twice a weak and eight times in total. The transplanted day was defined as day 0 and body weight and survival days were measured.

Changes in body weight and survival rate of each group with elapse of days are shown in Fig. 17 and Fig. 18. Only in the control group, a decrease in body weight was recognized starting from day 20 after the transplantation and, on about day 40, the survival rate became 0%. On the contrary, in all of the antibody-administered groups, no change was recognized in body weight and survival rate until day 121 which is the final day of the test. When an increase life span (ILS) % which is an index for the survival evaluation was calculated, it was 221% in the antibody-administered group and a significant survival rate of the anti-CD10 antibody to B-ALL was recognized. It was also clarified that chimeric antibody and humanized antibody had the similar anti-tumor effect.

While the invention has been described in detail and with reference to specific embodiments thereof, it will be apparent to one skill in the art that various changes and modifications can be made therein without departing from the spirit and scope thereof.

This application is based on Japanese application No. 2006-230300 filed on August 28, 2006, the entire contents of which are incorporated hereinto by reference. All references cited herein are incorporated in their entirety.

Industrial Applicability

The present invention relates to an agent for treating tumor resistant to an anti-CD20 antibody, comprising an antibody which specifically binds to CD 10 and has cytotoxic activity or the antibody fragment thereof as an active ingredient.

Sequence Listing Free Text

SEQ ID NO: 8 Descrption of Artificial Sequence: Amino Acid Sequence of Antibody Heavy Chain Vaiable Region
SEQ ID NO: 9 Descrption of Artificial Sequence: Synthetic DNA
SEQ ID NO: 10 Descrption of Artificial Sequence: Amino Acid Sequence of Antibody Light Chain Vaiable Region
SEQ ID NO: 11 Descrption of Artificial Sequence: Synthetic DNA

SEQ ID NO: 12 Descrption of Artificial Sequence: Amino Acid Sequence of Antibody Light Chain Vaiable Region
SEQ ID NO: 13 Descrption of Artificial Sequence: Synthetic DNA
SEQ ID NO: 14 Descrption of Artificial Sequence: Synthetic DNA
SEQ ID NO: 15 Descrption of Artificial Sequence: Synthetic DNA
SEQ ID NO: 16 Descrption of Artificial Sequence: Synthetic DNA
SEQ ID NO: 17 Descrption of Artificial Sequence: Synthetic DNA
SEQ ID NO: 18 Descrption of Artificial Sequence: Synthetic DNA
SEQ ID NO: 19 Descrption of Artificial Sequence: Synthetic DNA
SEQ ID NO: 20 Descrption of Artificial Sequence: Synthetic DNA
SEQ ID NO: 21 Descrption of Artificial Sequence: Synthetic DNA
SEQ ID NO: 22 Description of Artificial Sequence: Synthetic DNA
SEQ ID NO: 23 Descrption of Artificial Sequence: Synthetic DNA
SEQ ID NO: 24 Descrption of Artificial Sequence: Synthetic DNA
SEQ ID NO: 25 Descrption of Artificial Sequence: Synthetic DNA
SEQ ID NO: 26 Descrption of Artificial Sequence: Synthetic DNA
SEQ ID NO: 27 Descrption of Artificial Sequence: Synthetic DNA
SEQ ID NO: 28 Descrption of Artificial Sequence: Synthetic DNA
SEQ ID NO: 33 Descrption of Artificial Sequence: Amino Acid Sequence of Antibody Light Chain Vaiable Region
SEQ ID NO: 34 Descrption of Artificial Sequence: Synthetic DNA
SEQ ID NO: 35 Descrption of Artificial Sequence: Amino Acid Sequence of Antibody Heavy Chain Vaiable Region
SEQ ID NO: 36 Descrption of Artificial Sequence: Synthetic DNA
SEQ ID NO: 37 Descrption of Artificial Sequence: Amino Acid Sequence of Antibody Heavy Chain Vaiable Region
SEQ ID NO: 38 Descrption of Artificial Sequence: Synthetic DNA
SEQ ID NO: 39 Descrption of Artificial Sequence: Synthetic DNA
SEQ ID NO: 40 Descrption of Artificial Sequence: Synthetic DNA
SEQ ID NO: 41 Descrption of Artificial Sequence: Synthetic DNA
SEQ ID NO: 42 Descrption of Artificial Sequence: Synthetic DNA
SEQ ID NO: 43 Descrption of Artificial Sequence: Synthetic DNA

## Claims

1. A therapeutic agent for treating a tumor resistant to an anti-CD20 antibody, comprising administering an antibody which specifically binds to CD 10 and has cytotoxic activity or the antibody fragment thereof.

2. The therapeutic agent according to claim 1, wherein the antibody is a gene recombinant antibody.

3. The therapeutic agent according to claim 1, wherein the cytotoxic activity is at least one activity of antibody-dependent cell-mediated cytotoxicity and complement-dependent cytotoxicity.

4. The therapeutic agent according to claim 2, wherein the gene recombinant antibody is selected from the group consisting of a human chimeric antibody, a humanized antibody and a human antibody.

5. The therapeutic agent according to claim 1, wherein the antibody fragment is an antibody selected from Fab, Fab', $F(ab')_2$, a single chain antibody (scFv), a dimerized variable region (diabody), a disulfide stabilized variable region (dsFv) and a CDR peptide.

**6.** The therapeutic agent according to any one of claims 1 to 5, wherein the antibody is a fusion antibody conjugated with a radioisotope, a protein or an agent.

**7.** The therapeutic agent according to any one of claim 1 to 6, wherein the tumor resistant to an anti-CD20 antibody is a tumor in which the expression level of CD 10 on the tumor cells is increased by administration of an anti-tumor agent comprising an anti-CD20 antibody as an active ingredient.

**8.** The therapeutic agent according to any one of claim 1 to 7, wherein the tumor resistant to an anti-CD20 antibody is a tumor in which the expression level of CD20 on the tumor cell is decreased or depleted by administration of the anti-CD20 antibody.

**9.** Use of an antibody which specifically binds to CD10 and has cytotoxic activity or the antibody fragment thereof for the manufacture of a therapeutic agent for treating a tumor resistant to an anti-CD20 antibody.

FIG. 1

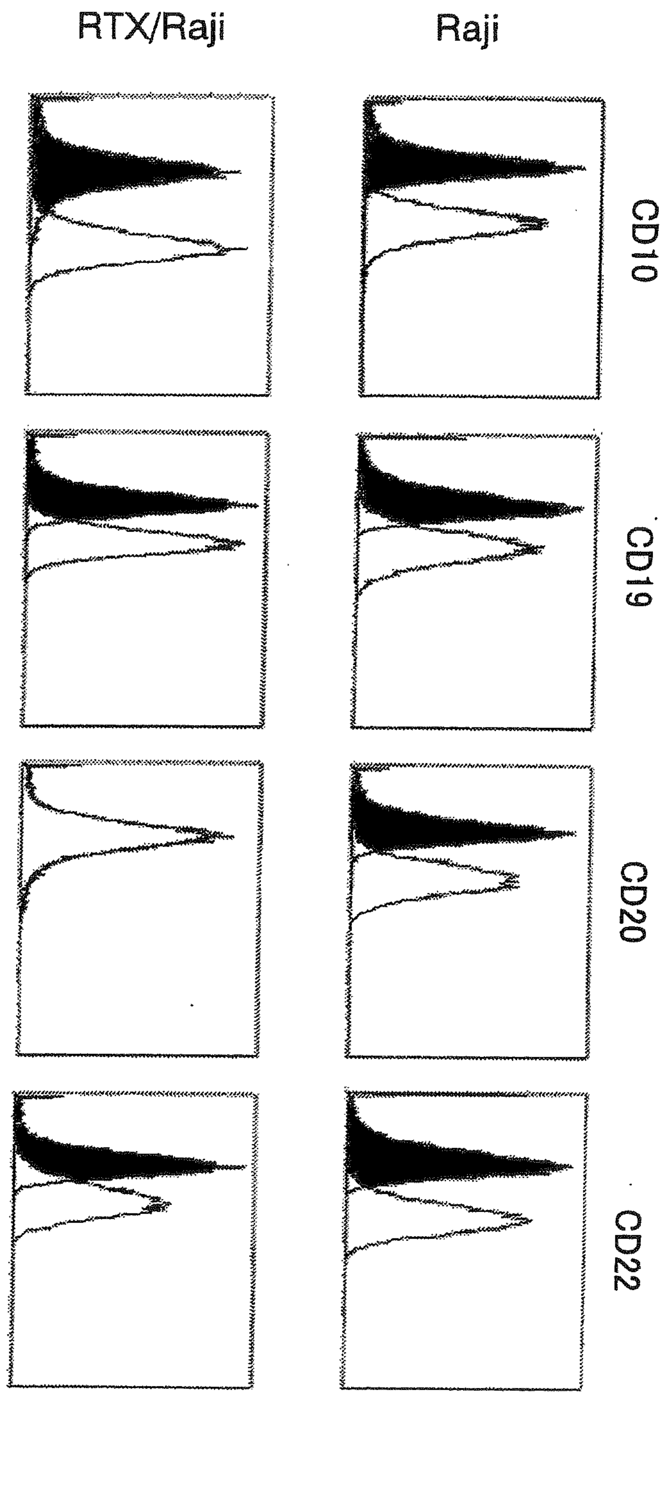

FIG. 2

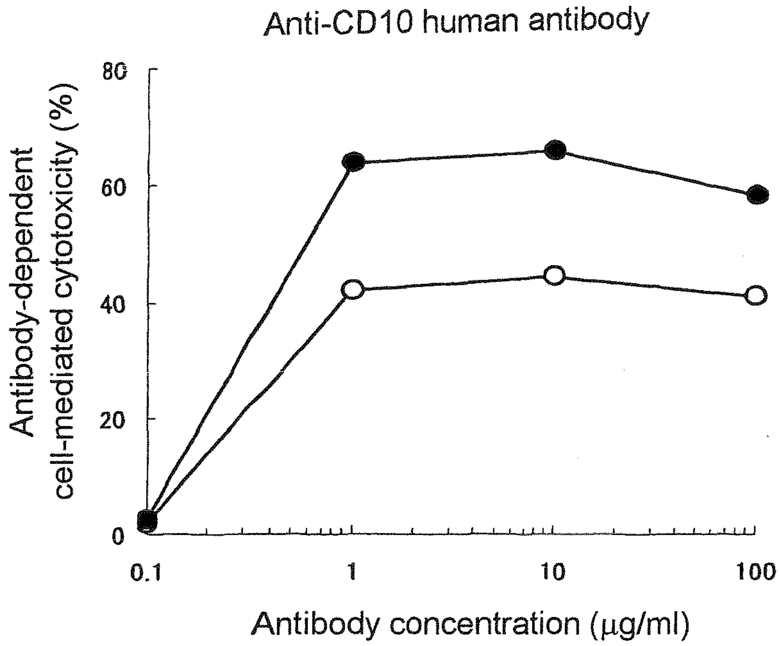
Anti-CD10 human antibody
Antibody-dependent cell-mediated cytotoxicity (%)
80
60
40
20
0
0.1
1
10
100
Antibody concentration (μg/ml)

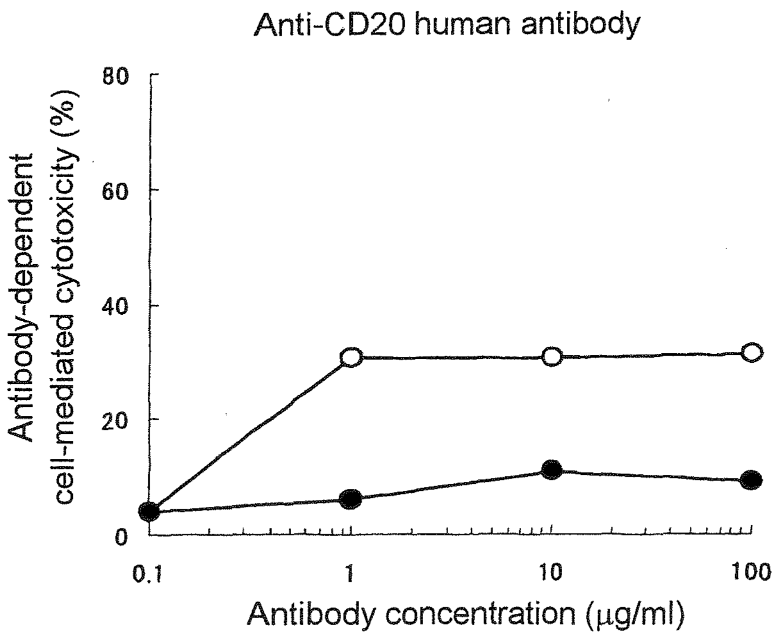
Anti-CD20 human antibody
Antibody-dependent cell-mediated cytotoxicity (%)
80
60
40
20
0
0.1
1
10
100
Antibody concentration (μg/ml)

FIG. 3

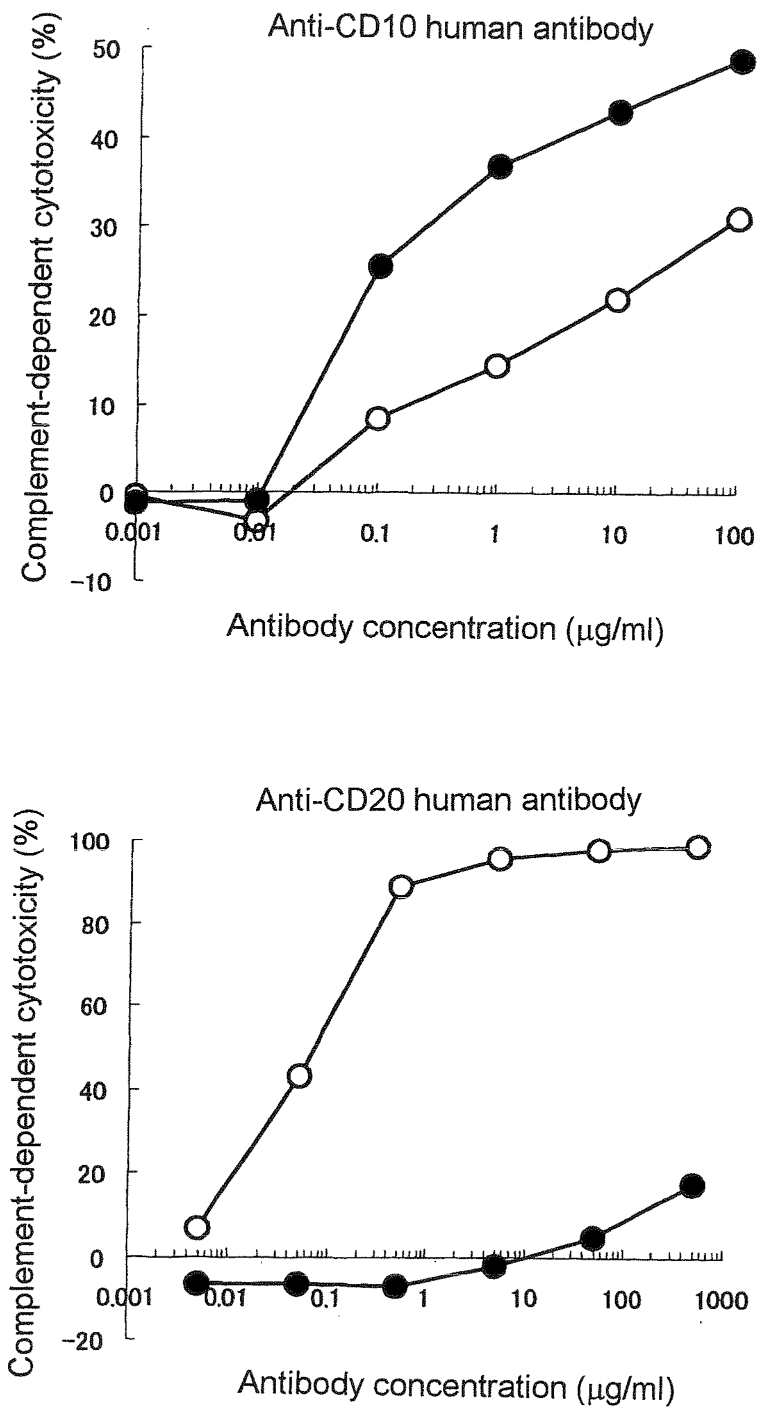
Anti-CD10 human antibody
Complement-dependent cytotoxicity (%)
50
40
30
20
10
0
-10
0.001
0.01
0.1
1
10
100
Antibody concentration (μg/ml)
Anti-CD20 human antibody
Complement-dependent cytotoxicity (%)
100
80
60
40
20
0
-20
0.001
0.01
0.1
1
10
100
1000
Antibody concentration (μg/ml)

FIG. 4

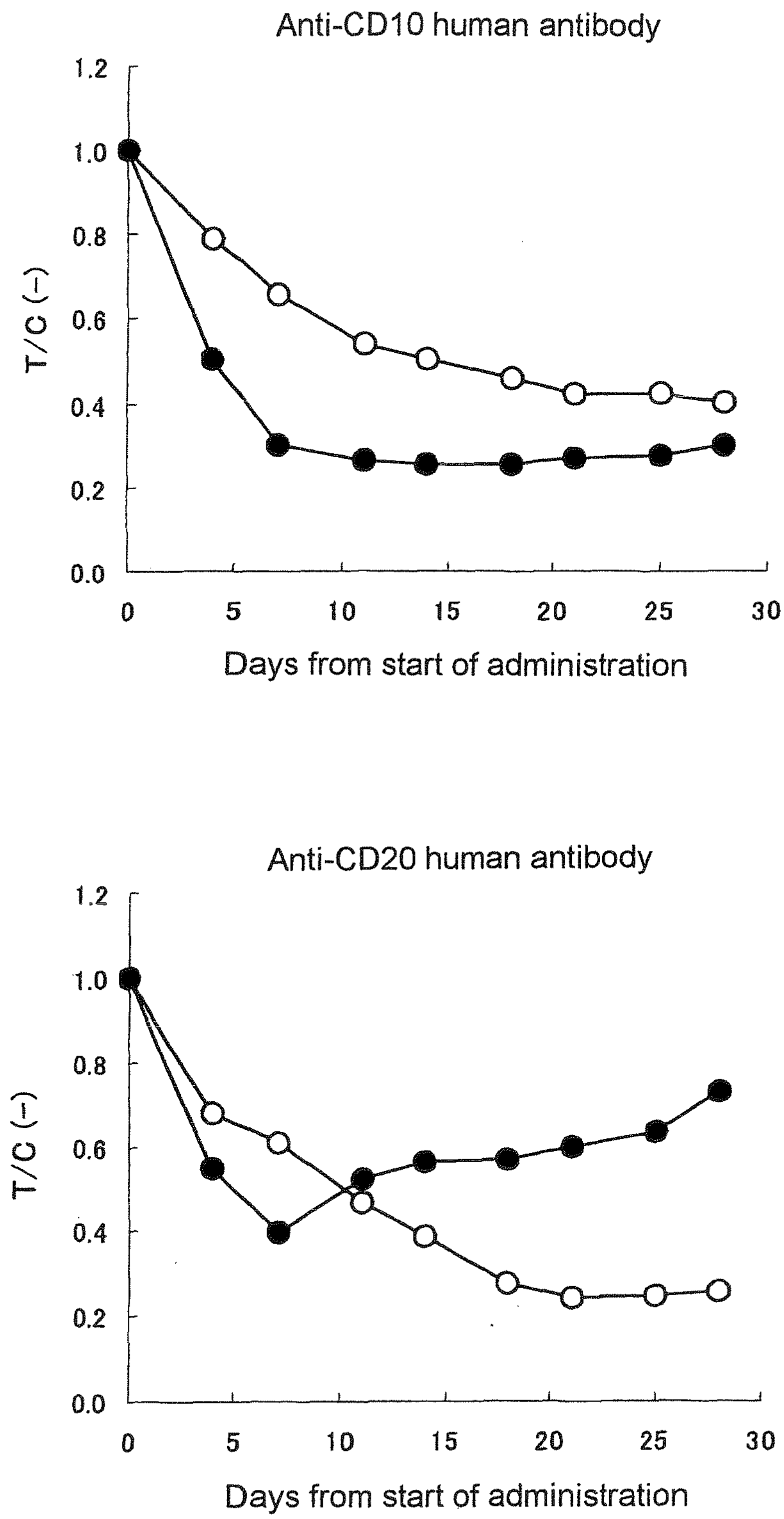
Anti-CD10 human antibody
T/C (-)
1.2
1.0
0.8
0.6
0.4
0.2
0.0
0
5
10
15
20
25
30
Days from start of administration
Anti-CD20 human antibody
T/C (-)
1.2
1.0
0.8
0.6
0.4
0.2
0.0
0
5
10
15
20
25
30
Days from start of administration

FIG. 5

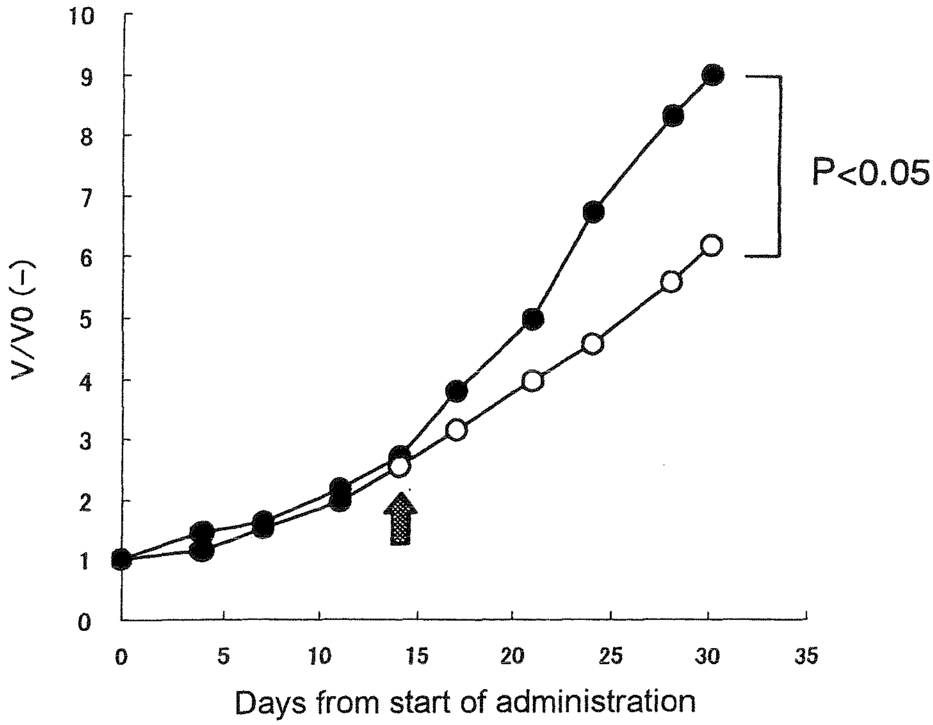
10
9
8
7
6
5
4
3
2
1
0
V/V0 (–)
P<0.05
0
5
10
15
20
25
30
35
Days from start of administration

FIG. 6

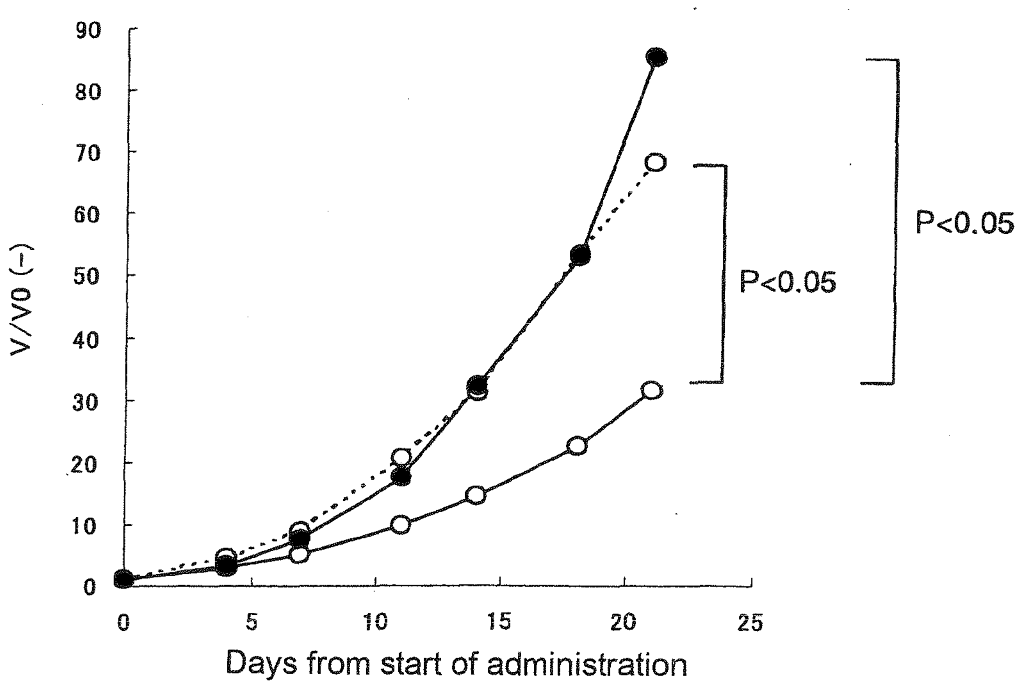
90
80
70
60
50
40
30
20
10
0
V/V0 (–)
P<0.05
P<0.05
0
5
10
15
20
25
Days from start of administration

FIG. 7

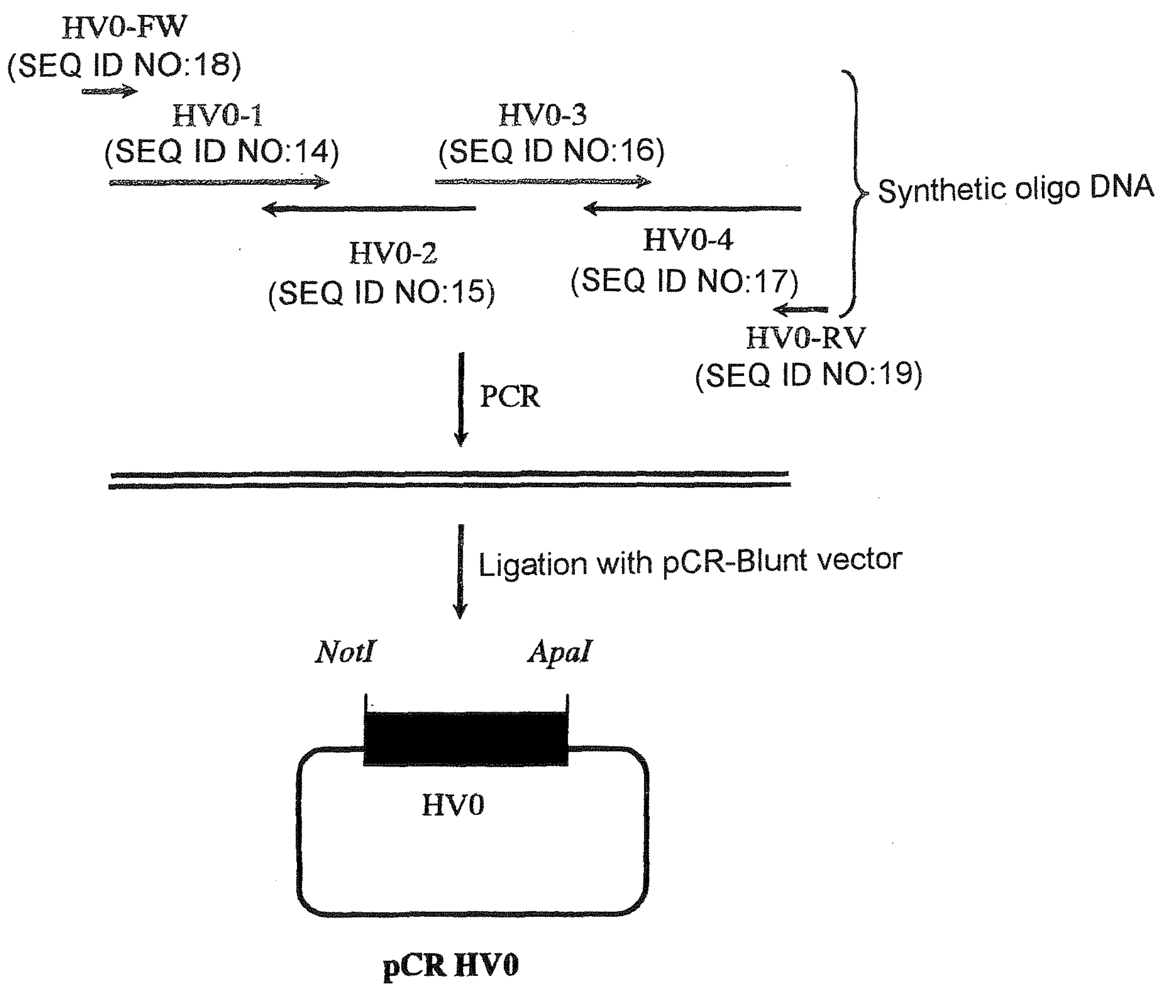
HV0-FW
(SEQ ID NO:18)
HV0-1
(SEQ ID NO:14)
HV0-3
(SEQ ID NO:16)
Synthetic oligo DNA
HV0-2
(SEQ ID NO:15)
HV0-4
(SEQ ID NO:17)
HV0-RV
(SEQ ID NO:19)
PCR
Ligation with pCR-Blunt vector
NotI
ApaI
HV0
pCR HV0

FIG. 8

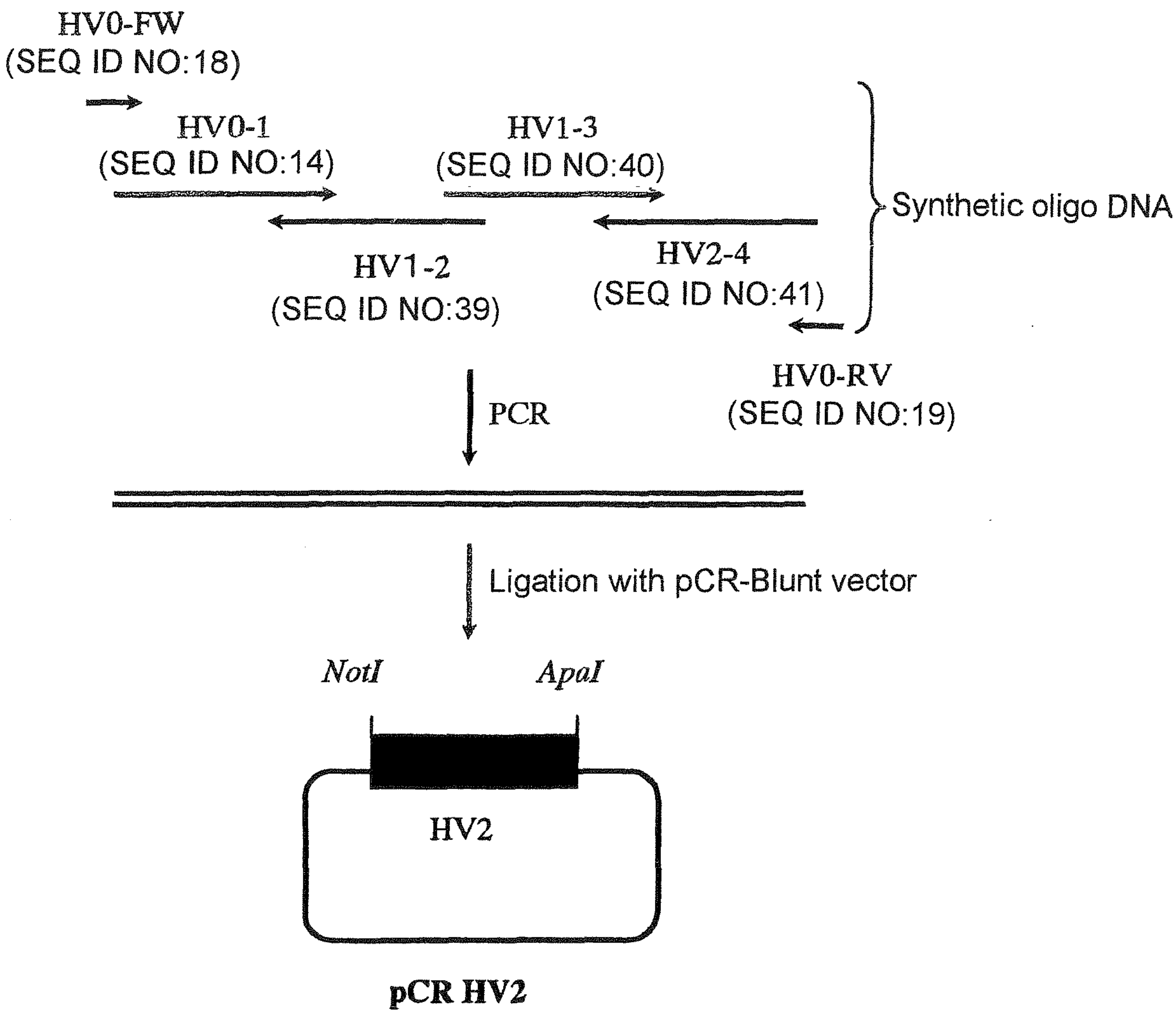
HV0-FW
(SEQ ID NO:18)
HV0-1
(SEQ ID NO:14)
HV1-3
(SEQ ID NO:40)
Synthetic oligo DNA
HV1-2
(SEQ ID NO:39)
HV2-4
(SEQ ID NO:41)
HV0-RV
(SEQ ID NO:19)
PCR
Ligation with pCR-Blunt vector
NotI
ApaI
HV2
pCR HV2

FIG. 9

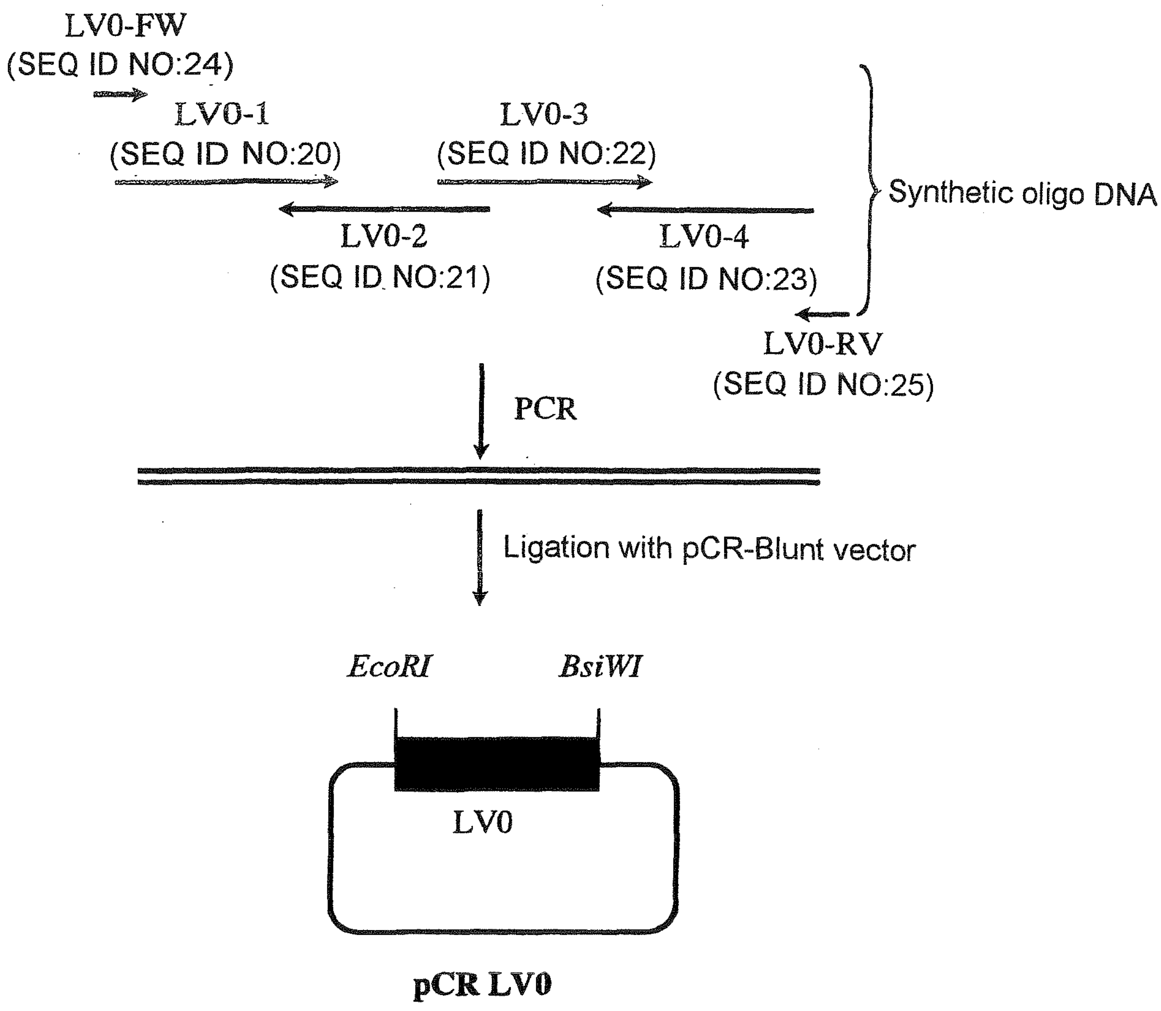
LV0-FW
(SEQ ID NO:24)
LV0-1
(SEQ ID NO:20)
LV0-3
(SEQ ID NO:22)
Synthetic oligo DNA
LV0-2
(SEQ ID NO:21)
LV0-4
(SEQ ID NO:23)
LV0-RV
(SEQ ID NO:25)
PCR
Ligation with pCR-Blunt vector
EcoRI
BsiWI
LV0
pCR LV0

FIG. 10

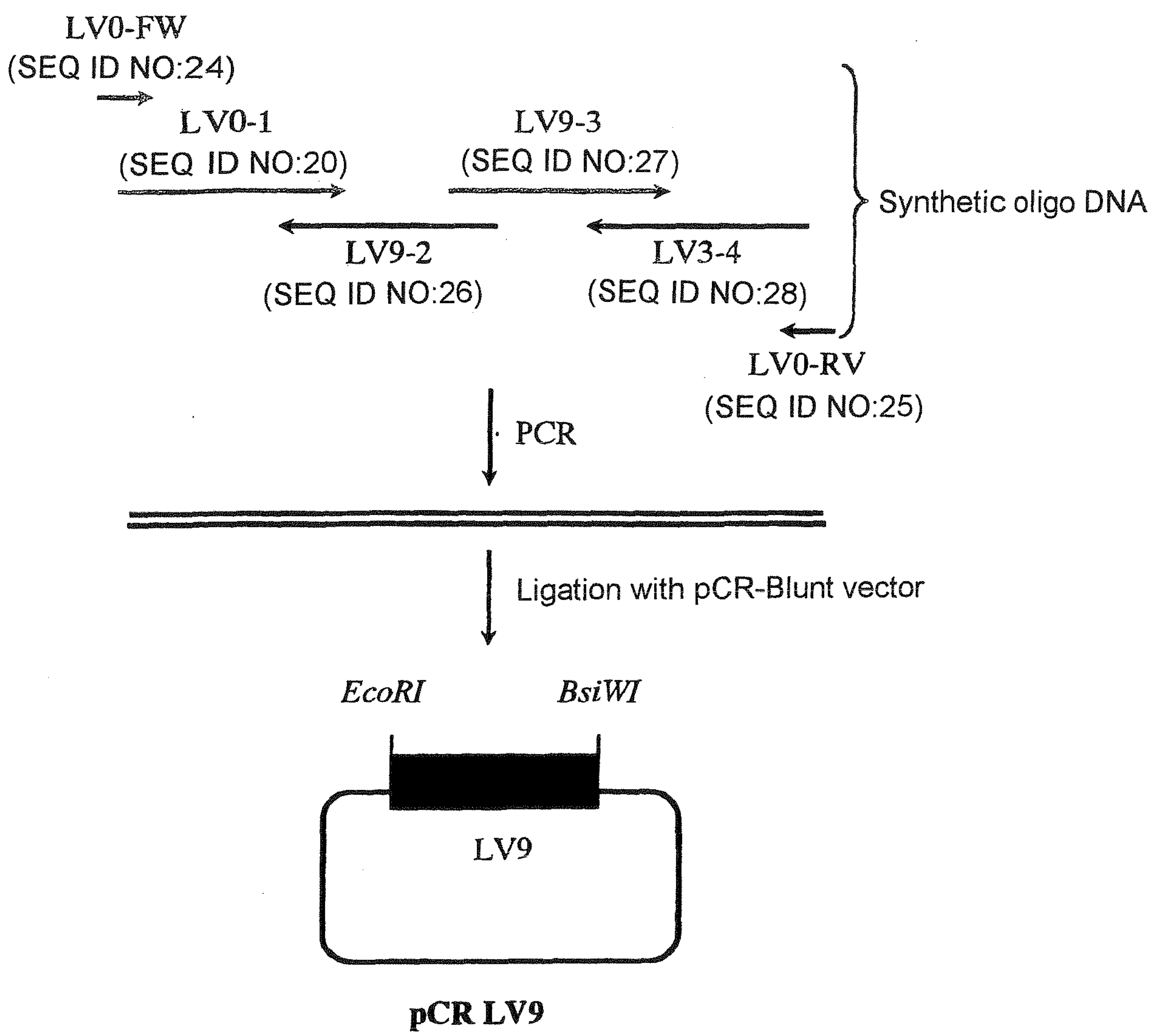
LV0-FW
(SEQ ID NO:24)
LV0-1
(SEQ ID NO:20)
LV9-3
(SEQ ID NO:27)
Synthetic oligo DNA
LV9-2
(SEQ ID NO:26)
LV3-4
(SEQ ID NO:28)
LV0-RV
(SEQ ID NO:25)
PCR
Ligation with pCR-Blunt vector
EcoRI
BsiWI
LV9
pCR LV9

FIG. 11

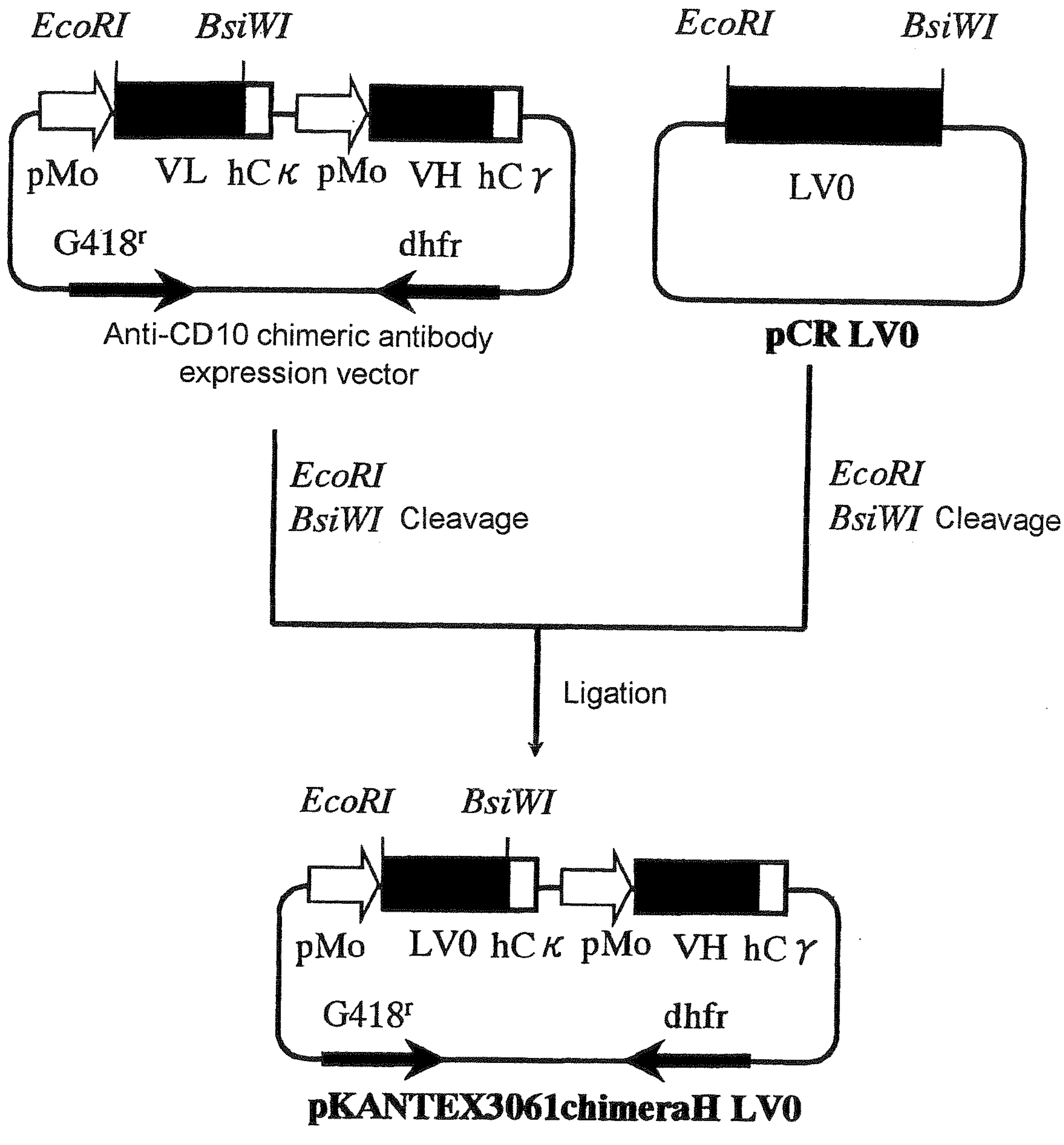
EcoRI
BsiWI
pMo
VL
hCκ
pMo
VH
hCγ
G418r
dhfr
Anti-CD10 chimeric antibody expression vector
EcoRI
BsiWI
LV0
pCR LV0
EcoRI
BsiWI Cleavage
EcoRI
BsiWI Cleavage
Ligation
EcoRI
BsiWI
pMo
LV0
hCκ
pMo
VH
hCγ
G418r
dhfr
pKANTEX3061chimeraH LV0

FIG. 12

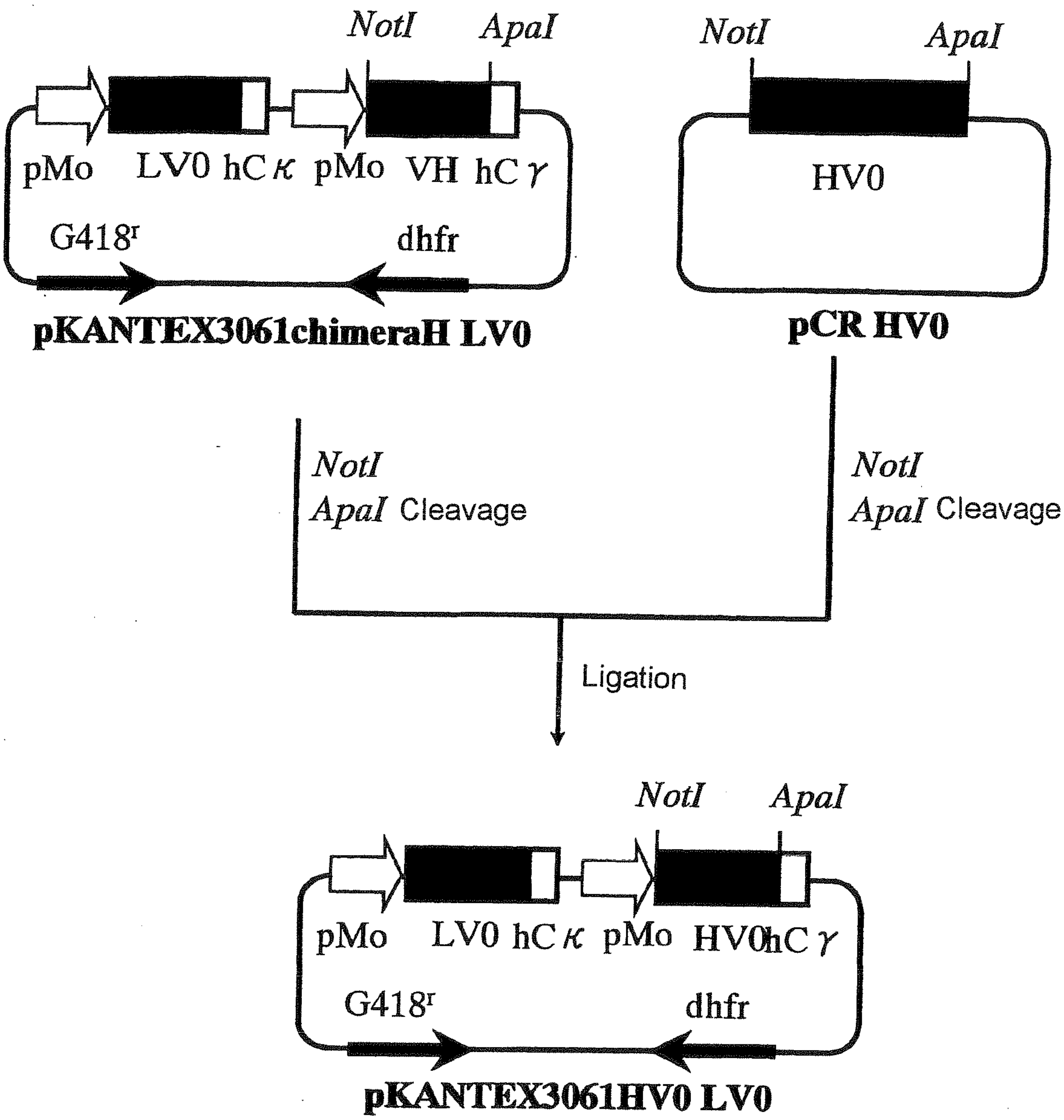
NotI
ApaI
pMo
LV0
hCκ
pMo
VH
hCγ
G418r
dhfr
pKANTEX3061chimeraH LV0
NotI
ApaI
HV0
pCR HV0
NotI
ApaI Cleavage
NotI
ApaI Cleavage
Ligation
NotI
ApaI
pMo
LV0
hCκ
pMo
HV0
hCγ
G418r
dhfr
pKANTEX3061HV0 LV0

FIG. 13

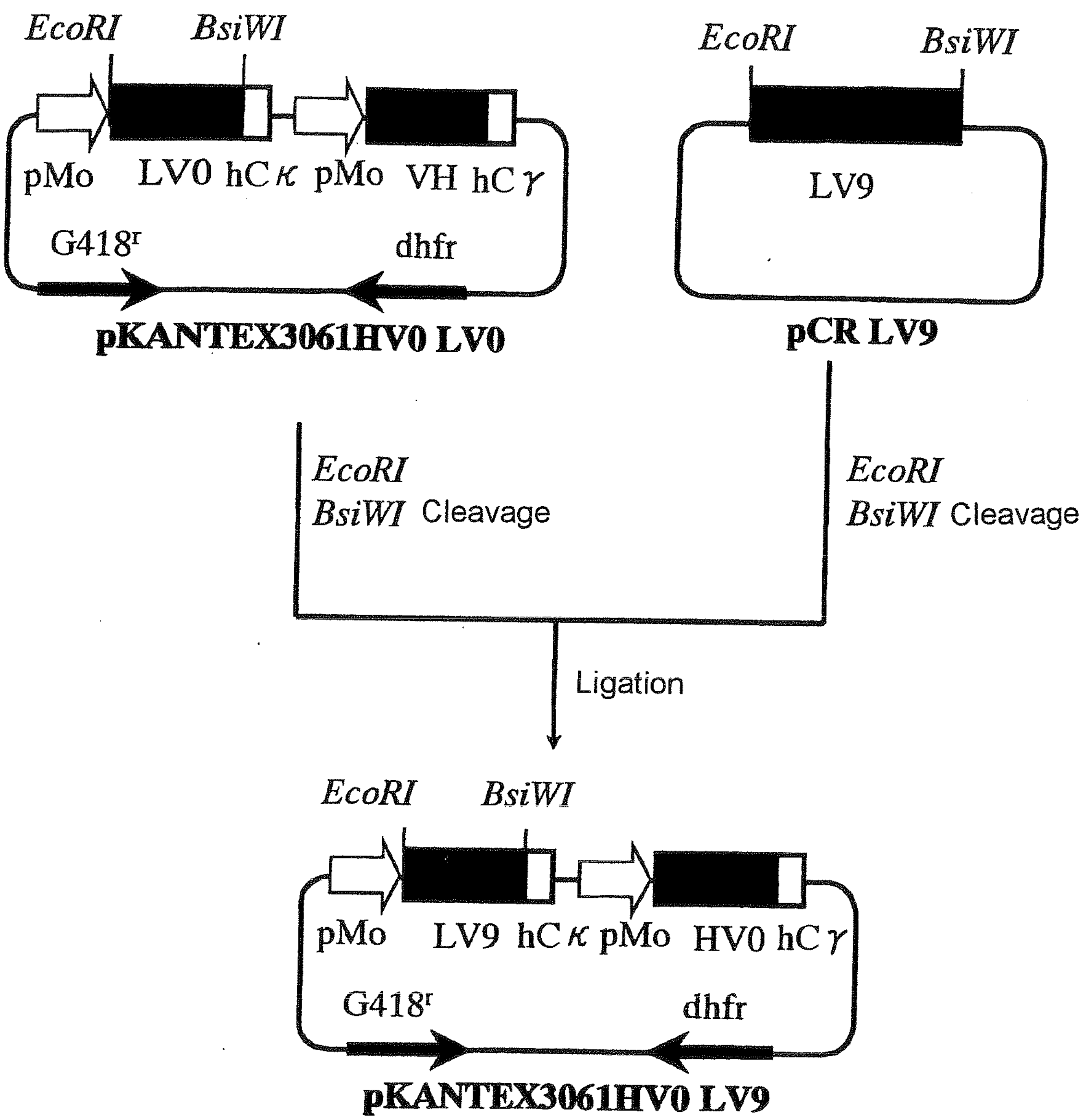
EcoRI
BsiWI
pMo
LV0
hCκ
pMo
VH
hCγ
G418r
dhfr
pKANTEX3061HV0 LV0
EcoRI
BsiWI
LV9
pCR LV9
EcoRI
BsiWI Cleavage
EcoRI
BsiWI Cleavage
Ligation
EcoRI
BsiWI
pMo
LV9
hCκ
pMo
HV0
hCγ
G418r
dhfr
pKANTEX3061HV0 LV9

FIG. 14

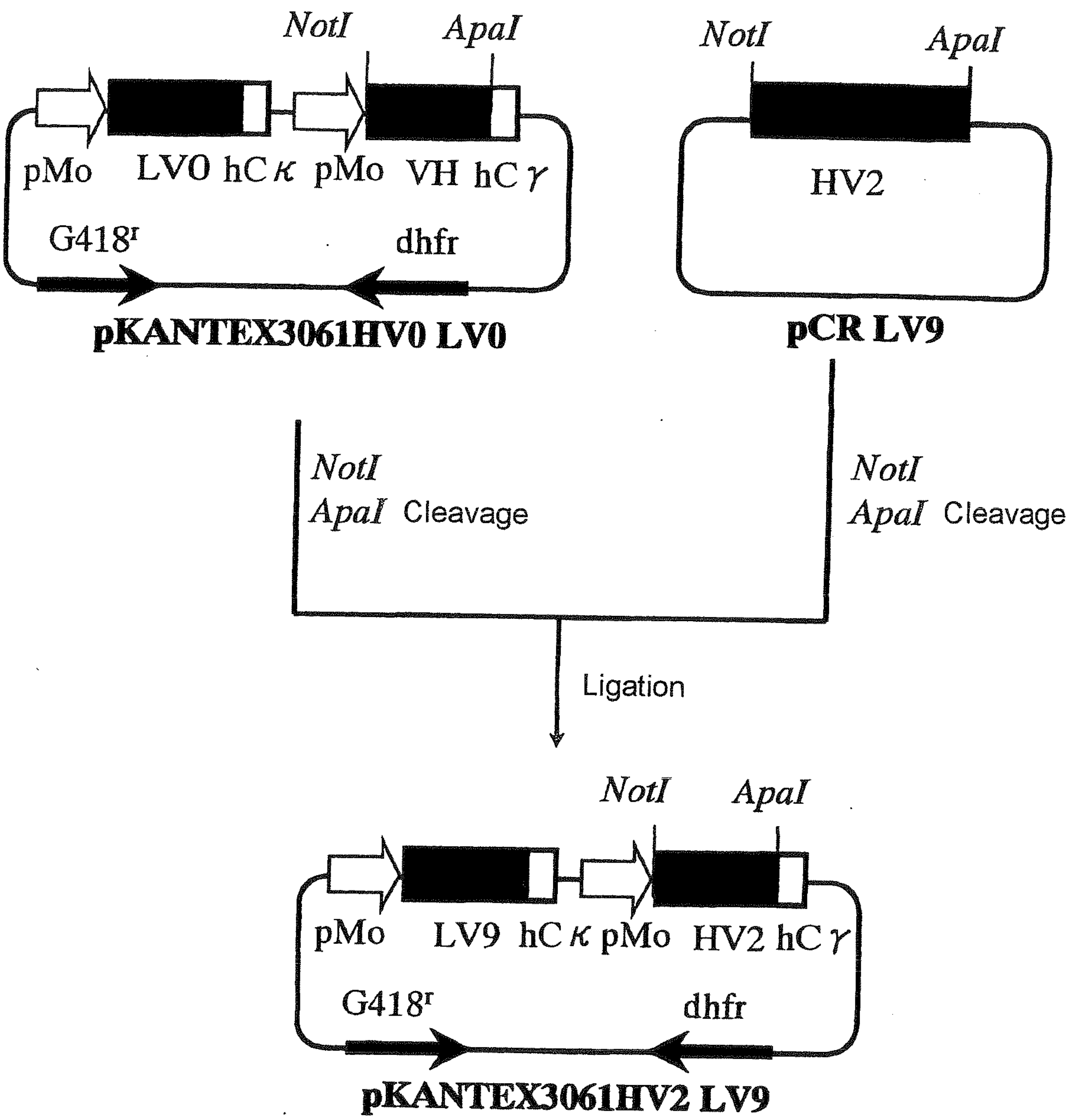
NotI
ApaI
pMo
LV0
hCκ
pMo
VH
hCγ
G418r
dhfr
pKANTEX3061HV0 LV0
NotI
ApaI
HV2
pCR LV9
NotI
ApaI Cleavage
NotI
ApaI Cleavage
Ligation
NotI
ApaI
pMo
LV9
hCκ
pMo
HV2
hCγ
G418r
dhfr
pKANTEX3061HV2 LV9

FIG. 15

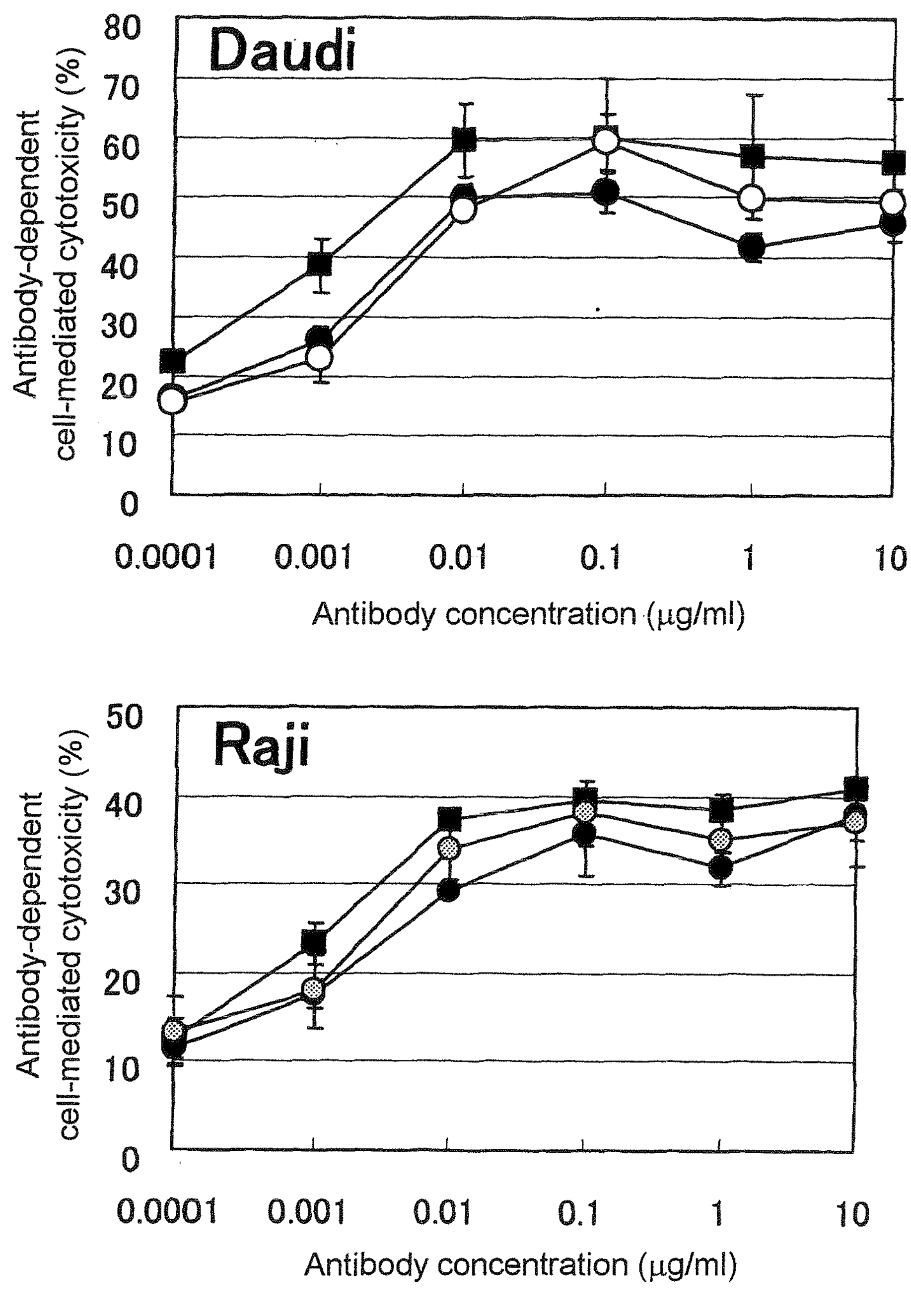
Daudi
Antibody-dependent cell-mediated cytotoxicity (%)
80
70
60
50
40
30
20
10
0
0.0001
0.001
0.01
0.1
1
10
Antibody concentration (μg/ml)
Raji
Antibody-dependent cell-mediated cytotoxicity (%)
50
40
30
20
10
0
0.0001
0.001
0.01
0.1
1
10
Antibody concentration (μg/ml)

FIG. 16

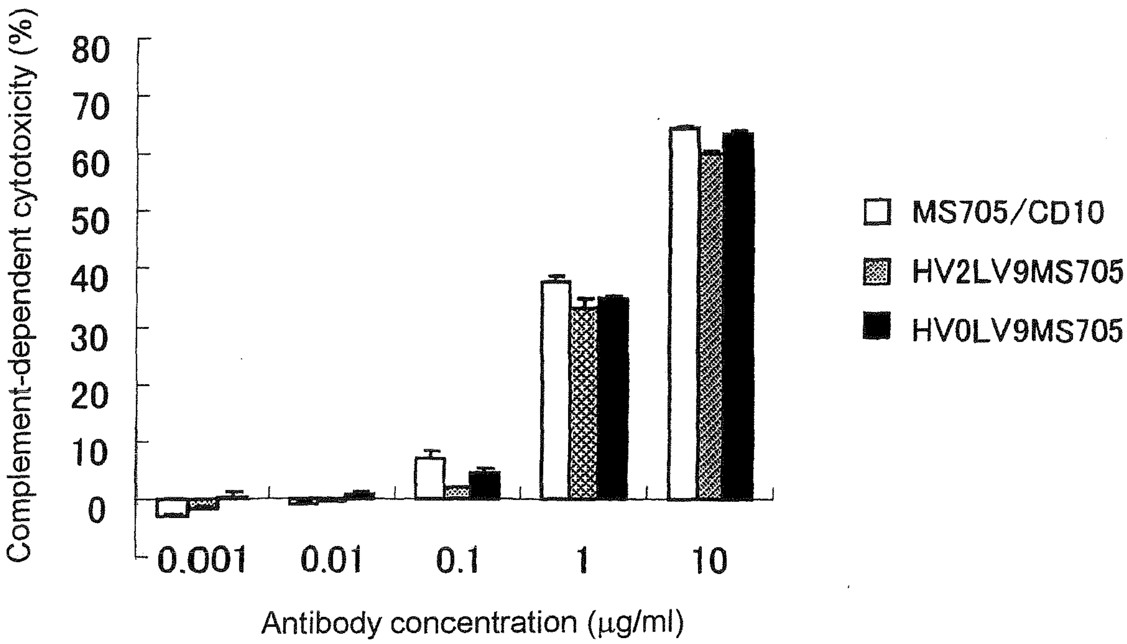
Complement-dependent cytotoxicity (%)
80
70
60
50
40
30
20
10
0
0.001
0.01
0.1
1
10
Antibody concentration (μg/ml)
MS705/CD10
HV2LV9MS705
HV0LV9MS705

FIG. 17

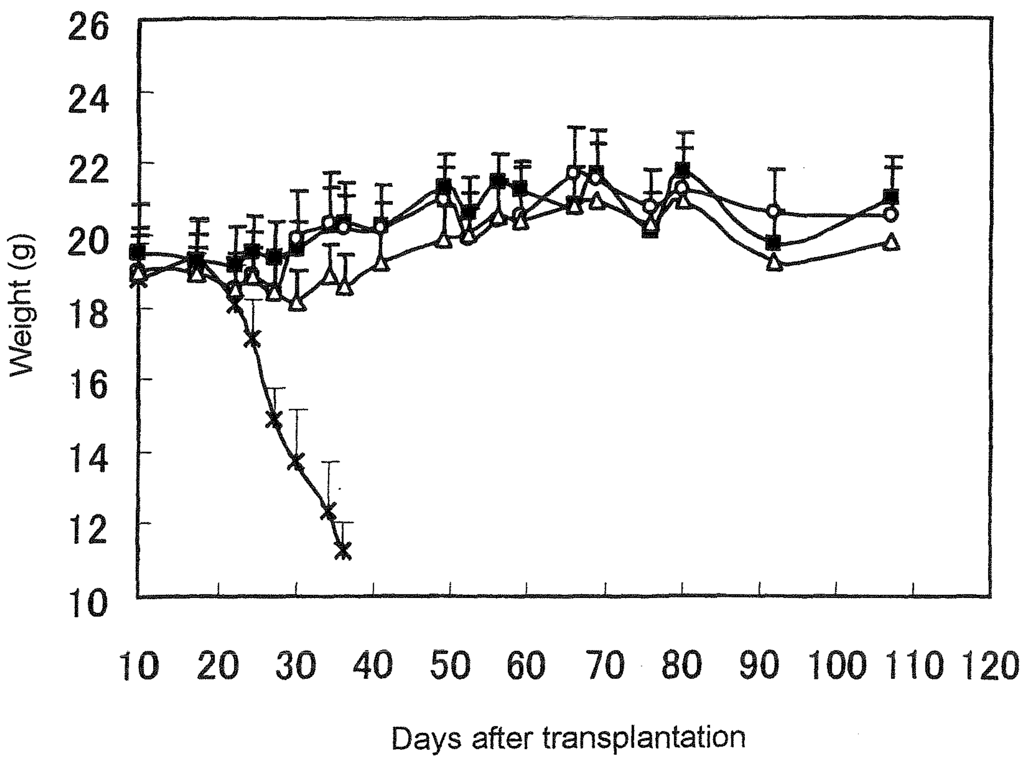
Weight (g)
26
24
22
20
18
16
14
12
10
10 20 30 40 50 60 70 80 90 100 110 120
Days after transplantation

FIG. 18

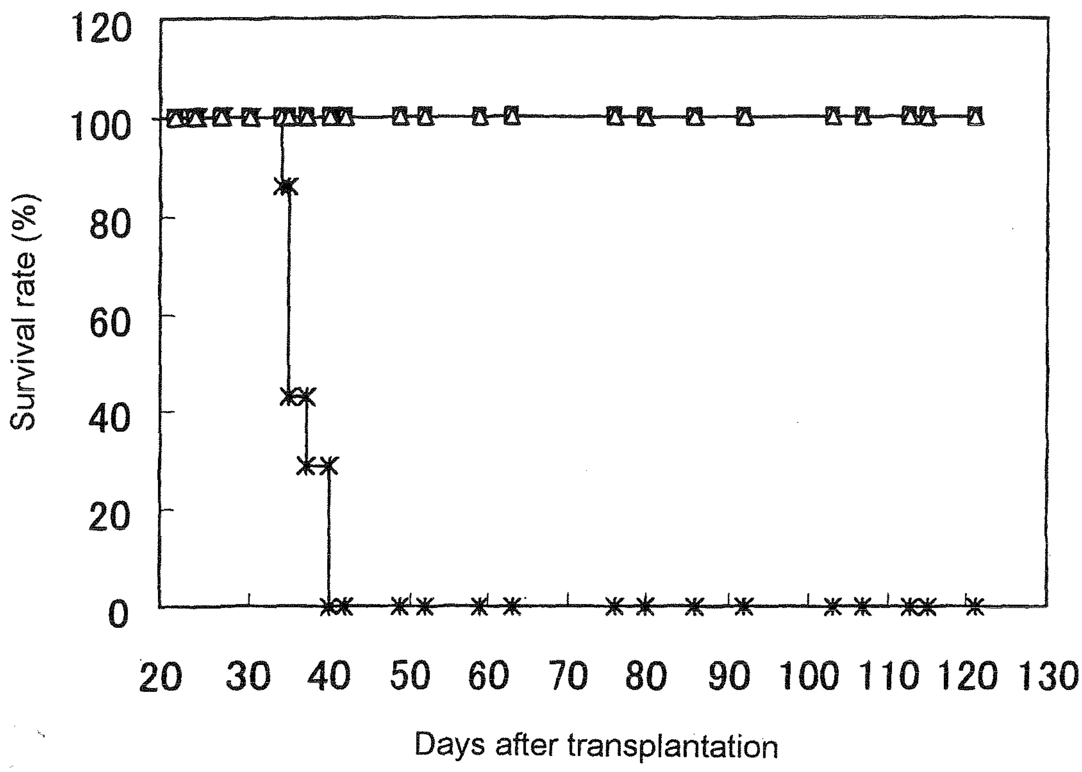
120
100
80
60
40
20
0
Survival rate (%)
20 30 40 50 60 70 80 90 100 110 120 130
Days after transplantation

**INTERNATIONAL SEARCH REPORT**

International application No.
PCT/JP2007/066685

A. CLASSIFICATION OF SUBJECT MATTER
*A61K39/395*(2006.01)i, *A61P35/00*(2006.01)i

According to International Patent Classification (IPC) or to both national classification and IPC

B. FIELDS SEARCHED

Minimum documentation searched (classification system followed by classification symbols)
A61K39/395, A61P35/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
BIOSIS/CAplus/EMBASE/MEDLINE(STN), JMEDPlus/JST7580/JSTPlus(JDream2)

C. DOCUMENTS CONSIDERED TO BE RELEVANT

| Category* | Citation of document, with indication, where appropriate, of the relevant passages | Relevant to claim No. |
|---|---|---|
| X | NISHIMURA,Y. et al., Recombinant human-mouse chimeric monoclonal antibody specific for common acute lymphocytic leukemia antigen, Cancer Research, 1987, Vol.47, No.4, p.999-1005, ABSTRACT, DISCUSSION, page 1004, left column, 'Complement-dependent Cytotoxicity' | 1-9 |
| X | HATTORI,K. et al., Bispecific antibody-mediated cytotoxicity by $CD4^+$ and $CD8^+$-activated T cells generated from leukemia patients after allogeneic bone marrow transplantation, Bone Marrow Transplantation, 1995, Vol.15, No.2, pp.193-198, Summary, page 197, right column, middle part | 1-9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 November, 2007 (14.11.07) | 27 November, 2007 (27.11.07) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2007/066685

C (Continuation). DOCUMENTS CONSIDERED TO BE RELEVANT

| Category* | Citation of document, with indication, where appropriate, of the relevant passages | Relevant to claim No. |
|---|---|---|
| X | SHIDA,Y. et al., *In vivo* and *in vitro* antitumor activity of mitomycin C conjugates at 7-N position through a linker containing thiocarbamate bond with CD10 monoclonal antibody, Biotherapy, 1992, Vol.5, No.2, p.97-105, Abstract, Results | 1-9 |
| P,X | WO 2006/121159 A1 (Kyowa Hakko Kogyo Co., Ltd.), 16 November, 2006 (16.11.06), Par. No. [0064]; examples 3, 4 (Family: none) | 1-9 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 1966042 A
- EP 184187 A
- US 4935496 A
- US 5736137 A
- EP 127095 A
- WO 9710354 A
- WO 0231140 A
- WO 0385107 A
- WO 03018635 A
- JP 2006230300 A


### Non-patent literature cited in the description


- *Expert Opin. Biol. Ther.,* 2001, vol. 1, 375
- *J. Exp. Med.,* 1988, vol. 168, 1247
- *Blood,* 1989, vol. 73, 625
- *Blood,* 1993, vol. 82, 1052
- *Anticancer Res.,* 1997, vol. 17, 3233
- *Am. J. Pathol.,* 2001, vol. 159, 1415
- *Am. J. Clin. Pathol.,* 2000, vol. 113, 374
- *Blood,* 1981, vol. 58, 648
- *Proc. Natl. Acad. Sci. U. S. A.,* 1982, vol. 79, 4386
- *Cancer Res.,* 1987, vol. 47, 999
- *J. Clin. Oncol.,* 1984, vol. 2, 881
- *Blood,* 1985, vol. 65, 1349
- *J. Natl. Cancer Inst.,* 1988, vol. 80, 932
- *Proc. Natl. Acad. Sci. U.S.A.,* 1985, vol. 82, 1242
- *J. Nucl. Med.,* 1985, vol. 26, 1011
- *J. Immunol.,* 1985, vol. 135, 1530
- *Cancer Res.,* 1986, vol. 46, 6489
- *Nature,* 1986, vol. 321, 522
- *Proc. Natl. Acad. Sci. U.S.A.,* 1989, vol. 86, 4220
- *Cancer Res.,* 1996, vol. 56, 1118
- *Immunol.,* 1995, vol. 85, 668
- *Blood,* 1994, vol. 83, 435
- *Cell Immunol.,* 2000, vol. 204, 55
- *Int J Clin Oncol.,* 2005, vol. 10
- *J Clin Oncol.,* 1998, vol. 16, 2825
- *Am Society of Hematology,* 10 December 2005
- *Br J Haematol.,* 2002, vol. 119, 412
- *Clin Cancer Res.,* 2005, vol. 11, 2327
- **Reiter et al.** *Protein Engineering,* 1994, vol. 7, 697
- Antibody Engineering Handbook. Chijin Shokan, 1994
- *Blood,* 2005, vol. 106, 448
- *Proc Am Soc Clin Oncol,* 2003, vol. 22, 2393
- *Blood,* 2005, vol. 106, 1131
- *J Clin Oncol,* 2005, vol. 23, 712
- *J. Biochem.,* 1987, vol. 101, 1307
- *Gene,* 1984, vol. 27, 223
- *Proc. Natl. Acad. Sci. USA,* 1981, vol. 78, 1527
- *Cytotechnol.,* 1990, vol. 4, 173
- *Cytotechnol.,* 1993, vol. 13, 79
- *J. Biochem,* 1987, vol. 101, 1307
- *Biochem. Biophys. Res. Commun.,* 1987, vol. 149, 960
- *Cell,* 1985, vol. 41, 479
- *Cell,* 1983, vol. 33, 717
- *J. Immunol. Methods,* 1994, vol. 167, 271
- *Hybridoma,* 1998, vol. 17, 559
- *Sequences of Proteins of Immunological Interest,* 1991
- Sequences of Proteins of Immunological Interest. US Dept. Health and Human Services, 1991
- *BIO/TECHNOLOGY,* 1991, vol. 9, 266
- *J. Mol. Biol.,* 1977, vol. 112, 535
- Methods in Nucleic Acids Res. CRC Press, 1991, vol. 283
- *Proc. Natl. Acad. Sci. USA,* 1987, vol. 84, 7413
- **ELISA.** Antibodies-A Laboratory Manual. Cold Spring Harbor Laboratory, 1988
- Monoclonal Antibodies: Principles and Practice. Academic Press Limited, 1996
- *Cytotechnology,* 1990, vol. 3, 133
- *Pro. Natl. Acad. Sci. U.S.A.,* 1980, vol. 77, 4216
- *J. Immnol. Methods,* 1994, vol. 167, 271
- Antibodies: A Laboratory Manual. Cold Spring Harbor Laboratory, 1988
- Monoclonal Antibodies: Principles and Practice. Academic Press, 1996
- *Nature,* 1970, vol. 227, 680
- Monoclonal Antibodies: Principles and Practice. Academic Press Limited (1996), 1996

- Monoclonal Antibodies: Principles and Practice. 1995
- *Science,* 1988, vol. 240, 1041
- Antibody Engineering, A Practical Guide. W. H. Freeman and Company, 1992
- Monoclonal Antibodies: Principles and Practice. Academic Press, 1995
- Antibody Engineering, A Practical Approach. IRL Press, 1996
- *Bio/Technology,* 1992, vol. 10, 163
- *Hum. Antibodies & Hybridomas,* 1994, vol. 5, 48
- *FEBS Letters,* 1999, vol. 453, 164
- *Int. J. Cancer,* 1998, vol. 77, 763
- *Protein Engineering,* 1994, vol. 7, 697
- *Cancer Immunol. Immunother.,* 1993, vol. 36, 373
- *Proc. Natl. Acad. Sci. USA,* 1992, vol. 79, 4386
- *Proc. Natl. Acad. Sci., USA,* 1982, vol. 79, 4386
- Molecular Cloning: A Laboratory Manual. Cold Spring Harbor Lab. Press, 1989
- *Nature,* 1980, vol. 283, 786
- *Cell,* 1980, vol. 22, 197
- Sequences of Proteins of Immunological Interest. U. S. Dept. Health an Human Services, 1991
- *Nucleic Acid Res,* 1997, vol. 25, 3389
- *Proc. Natl. Acad. Sci. USA,* 1990, vol. 1691, 6146
- Sequences of Proteins of Immunological Interest. U. S. Dept. Health and Human Services, 1991
- Sequence of Proteins of Immunological Interest. U. S. Dept. Health and Human Services, 1991